# EUROPÄISCHE PATENTANMELDUNG

(11) **EP 1 431 763 A1**
(43) Veröffentlichungstag der Anmeldung: **23.06.2004**
(21) Anmeldenummer: 02028540.9
(22) Anmeldetag: 20.12.2002
(51) Int. Cl.: G01N 33/68, A61K 39/395, A61K 38/17, A61K 31/70, C07K 14/47, C12N 15/11, C07K 16/18

(54) **Verfahren zum Nachweis einer Stoffwechselerkrankung**

(71) Anmelder: BioVisioN AG, 30625 Hannover (DE)
(72) Erfinder: Budde, Petra, Dr., 30655 Hannover (DE); Neitz, Susanne, Dr., 30161 Hannover (DE); Schulte, Imke, Dr., 30625 Hannover (DE); Mussauer, Heiko, Dr., 68161 Mannheim (DE); Hess, Rüdiger, Dr., 30629 Hannover (DE)
(74) Vertreter: Läufer, Martina, Dr.

(57) **Zusammenfassung**

Die Erfindung betrifft Syncollinpeptide, Syncollin-kodierende Nukleinsäuren und dazu komplementäre Antisense-Nukleinsäuren, gegen Syncollinpeptide gerichtete Antikörper oder Agonisten beziehungsweise Antagonisten dieser Stoffe, insbesondere zum Einsatz zur Diagnose, Prophylaxe und Therapie von Stoffwechselerkrankungen, insbesondere des Metabolischen Syndroms.

## Beschreibung

Die Erfindung betrifft ein Verfahren zum Auffinden von Anzeichen für das Vorliegen einer Stoffwechselerkrankung oder einer erhöhten Disposition für eine solche Erkrankung durch Bestimmung wenigstens einer Markersubstanz in einer biologischen Probe eines Organismus, zugehörige, als Marker geeignete, sowie sonst diagnostisch oder therapeutisch verwendbare Peptide, Peptidomimetika und Nukleinsäuren und die Verwendung der aufgefundenen Stoffe als Arzneimittel. Weiter umfasst die Erfindung Testkits zur Durchführung des Verfahrens, Screeningverfahren und Arzneimittel.

### Epidemiologie

Stoffwechselerkrankungen nehmen weltweit stark zu und stellen die Gesundheitssysteme vieler Länder vor immer stärker werdende medizinische und finanzielle Probleme.

Unter Stoffwechselerkrankungen versteht man allgemein Störungen des Gesamt- oder eines Teilstoffwechsels mit der daraus resultierenden Bildung quantitativ oder qualitativ abnormer Zwischenstoffwechsel. Im Rahmen der vorliegenden Erfindung umfasst der Begriff Stoffwechselerkrankungen insbesondere Erkrankungen des Energiestoffwechsels, vorzugsweise des Kohlenhydrat- und Lipidstoffwechsels sowie insbesondere das Metabolische Syndrom einschließlich seiner Krankheitsbilder Adipositas, Insulinresistenz, Glukoseintoleranz, Typ 2-Diabetes, Dyslipidämie, Hypertonie, Veränderungen des Gefäßsystems und Hyperkoagulabilität, aber auch anderer Stoffwechselerkrankungen wie beispielsweise Typ 1-Diabetes, Hyperurikämie/Gicht, Hypoglykämie, Anorexie, Bulimie oder Kachexie.

Den Hauptanteil der Kosten muss hierbei zur Behandlung von Symptomen aufgewendet werden, welche mit dem "Metabolischen Syndrom" (MTS) in enger Beziehung stehen. So sind bereits 20-40 Prozent der Bevölkerung in industrialisierten Ländern vom Metabolischen Syndrom betroffen, mit steigender Tendenz. Ein Viertel der Bevölkerung in den Industriestaaten ist bereits insulinresistent [1]. In den USA hat die Verbreitung der Adipositas in den letzen 30 Jahren um über 25% zugenommen, so dass zur Zeit bereits ca. 100 Millionen US-Bürger als übergewichtig gelten und dadurch ein stark erhöhtes Risiko besitzen, Insulinresistenz und Typ 2-Diabetes zu entwickeln [2]. Bereits 1990 betrugen in den USA die durch Adipositas verursachten Kosten über 45.000.000.000 US$ oder 6,8% der Gesamtkosten des Gesundheitswesens, mit seitdem steigender Tendenz [3]. Die Zahl der Typ 2-Diabetiker in den USA stieg als eine signifikante Folge von Adipositas und Insulinresistenz im Zeitraum von 1988 bis 1994 um 33%, so dass dort bereits 10.200.000 Personen an Diabetes und 13.400.000 an Glukoseintoleranz litten [4].

### Metabolisches Syndrom

Das Metabolische Syndrom beschreibt einen komplexen Zustand eines Organismus, bei welchem mehrere Aspekte des normalen Stoffwechsels gestört sind. Synonyme für das Metabolische Syndrom sind "Syndrom X", Insulinresistenzsyndrom, Wohlstandsyndrom, Reaven's Syndrom oder "tödliches Quartett". Das Syndrom ist durch das Zusammenspiel folgender Symptome gekennzeichnet:
- Stammbetontes Übergewicht (vermehrtes viszerales Fett)
- Insulinresistenz, Hyperinsulinämie
- Verminderte Glukosetoleranz bis hin zum manifesten Typ 2-Diabetes
- Dyslipidämie (VLDL erhöht, HDL erniedrigt)
- Arterielle Hypertonie, endotheliale Dysfunktion

Diese Symptome stellen Hauptrisikofaktoren einer kardiovaskulären Erkrankung dar, die ihrerseits die Haupttodesursache dieser Patienten ist. Als weitere Risikofaktoren, die in Zusammenhang mit dem Metabolischen Syndrom stehen, gelten unter anderem Hyperhomocysteinämie, Hyperurikosämie, Hyperleptinämie und Hyperkoagulabilität.

Im Jahr 1998 hat die World Health Organization (WHO) folgende Definition des Metabolischen Syndroms veröffentlicht [5]:
1. Diabetes mellitus Typ 2,
2. Glukoseintoleranz,
3. BMI > 30 kg m⁻²,
4. Taille-Hüft-Relation > 0,9 bei Männern beziehungsweise > 0,85 bei Frauen,
5. Triglyceride > 1,7 mmol l⁻¹
6. HDL-Cholesterin < 0,9 mmol l⁻¹ bei Männern beziehungsweise < 1,0 mmol l⁻¹ bei Frauen,
7. Blutdruck > 165/95 mmHg beziehungsweise antihypertensive Therapie und
8. Mikroalbuminurie > 20 Mikrogramm/Minute.

Die Diagnose "MTS" wird nach dieser Definition dann gestellt, wenn eine Voraussetzung für 1. oder 2. und zwei Voraussetzungen für 3. bis 8. erfüllt sind.

Der Body-Mass-Index (BMI) ist ein Maß zur Beurteilung des Körpergewichtes. Er berechnet sich nach der Formel: BMI = (Körpergewicht [in kg]) / (Körpergröße [in m])² und schätzt den Anteil der Fettmasse an der Körpermasse ab.

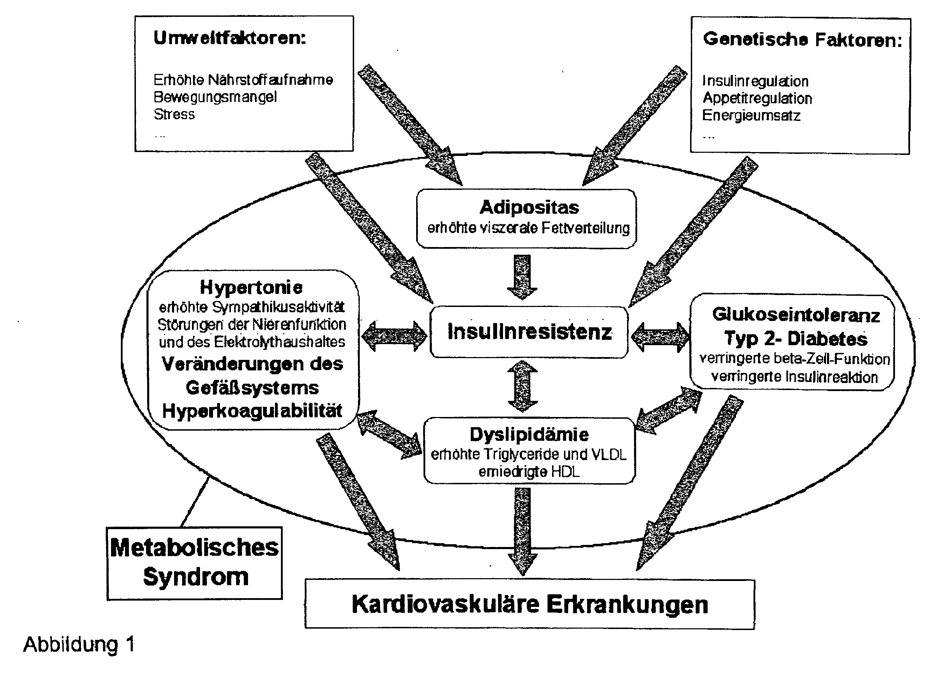

Eine schematische Übersicht über die kausalen Ursachen, Zusammenhänge und Folgen des Metabolischen Syndroms ist in Abbildung 1 dargestellt. So können genetische Faktoren und Umwelteinflüsse zu einer Adipositas und, meist als Folge dieser, schließlich zu einer muskulären Insulinresistenz führen. Dies wiederum bewirkt weitere Stoffwechselstörungen, welche sich auf andere Gewebe und Organe erstrecken können, so dass ein komplexes Netz sich gegenseitig beeinflussender metabolischer Dysfunktionen entsteht, welches unter dem Begriff "Metabolisches Syndrom" zusammengefasst wird. Eine Manifestierung des MTS führt in den meisten Fällen zu schweren Herz-Kreislauferkrankungen, so dass 78% der Todesfälle dieser Patienten kardiovaskuläre Ursachen haben [6]. Weitere Folgen des MTS können auch Mikroangiopathien, Atheriosklerose, Nierenschädigungen, Rethinopathien, Neuropathien oder das "diabetischer Fußsyndrom" sein. oder

### Schlüsselfaktoren des Metabolischen Syndroms

Es gilt inzwischen als gesichert, dass die Symptome Adipositas, Hypertonie, Insulinresistenz, Glukoseintoleranz und Dyslipidämie eine gemeinsame metabolische Ursache haben, die darauf beruht, dass insbesondere im Kohlenhydrat- und Lipidstoffwechsel oder deren Regulationsmechanismen bestimmte Anomalien bei diesen Patienten vorliegen. Diese metabolischen Störungen führen dann zur Ausbildung bestimmter Krankheiten und Symptome, die unter dem Oberbegriff "Metabolisches Syndrom" zusammen gefasst werden.

Als der entscheidende Schlüsselfaktor in der Entstehung des Metabolischen Syndroms gilt das Auftreten einer Adipositas. Als wesentliche Ursachen für die stark angestiegene Prävalenz von Adipositas vor allem in den industrialisierten Ländern werden eine Steigerung der Nahrungsaufnahme, insbesondere der Aufnahme von Kohlenhydraten und Fetten, und eine Verminderung der körperlichen Bewegung angenommen. Neben dem Lebensstil spielen aber auch genetische Faktoren für die Entstehung von Adipositas eine bedeutende Rolle. Hier zeigen Studien, dass weitere Aspekte des Energiestoffwechsels, wie zum Beispiel Gewichtsreduktion infolge einer kalorienreduzierten Diät, Ruheenergieumsatz, quantitative und qualitative Nahrungspräferenzen und das körperliche Aktivitätsniveau, genetischen Einflüssen unterliegen [7]. Solche Erbanlagen können beispielsweise auch mit einer vermehrten Nahrungsaufnahme, einem verminderten Energieumsatz oder einer bevorzugten Energiespeicherung in Form von Fett assoziiert sein. Einzelne Genmutationen wie beispielsweise des Melanocortin-4-Rezeptors (MC4R), des Proopiomelanocortins (POMC), der Endopeptidase-Prohormon-Konvertase 1 (PC1), des Leptins (LEP) oder des Leptinrezeptors (LEPR) konnten in direkten Zusammenhang mit dem Auftreten von Adipositas und Insulinresistenz / Typ 2-Diabetes gebracht werden [8]. Durch nachfolgende vergleichende Analysen und Untersuchungen konnten über 200 weitere Gene identifiziert werden, die mit einem Adipositas-Phänotyp im Verbindung stehen und als mögliche Zielmoleküle für eine Therapie des Metabolischen Syndroms dienen können. Darunter sind beispielsweise peroxisome proliferator activated receptor gamma (PPARgamma), uncoupling protein UCP1, UCP2, UCP3, fatty acid binding protein 2 (FABP2), beta2- und beta3-adrenergic receptor, melanocortin receptor MC3R, MC4R, MC5R, Neuropeptid Y (NPY), hormone sensitive lipase (HSL), lipoprotein lipase (LPL), insulin responsive substrate-1 (IRS-1), membrane glycoprotein / plasma cell differentiation factor (PC-1) und skeletal muscle glycogen synthetase [9]. In den letzten Jahren wurden auch Theorien zur Entstehung des Metabolischen Syndroms aufgestellt, welche in einer Insulin- oder Leptin-Resistenz den ursächlichen Auslöser dieses Krankheitsbildes sehen. Dort werden Hyperinsulinämie und Insulinresistenz als entscheidende Faktoren in der Ätiologie des Metabolischen Syndroms angesehen und stehen in engem Zusammenhang mit der Ausbildung des Typ 2-Diabetes. Insulinresistenz im Fett- und Muskelgewebe, fehlerhafte Funktion der pankreatischen beta-Zellen und eine Steigerung der Triglycerid- und Glukosefreisetzung der Leber können so zu erheblichen Störungen des Glukose- und Lipidstoffwechsels führen, welche das Auftreten kardiovaskulärer Erkrankungen stark begünstigen.

### bisherige Diagnose und Therapie des Metabolischen Syndroms

Die Diagnose des Metabolischen Syndroms ist bisher vor allem auf die einzelnen Erscheinungsbilder und Symptome dieses Krankheitskomplexes beschränkt. Während eine Adipositas relativ einfach zu diagnostizieren ist, lassen sich die anderen Komponenten des Metabolischen Syndroms weit schwerer feststellen. So leben in der Bundesrepublik Deutschland etwa 5 Millionen Menschen mit der Diagnose Diabetes mellitus Typ-2, wobei allerdings von einer deutlich höheren Dunkelziffer ausgegangen werden muss, deren Diagnose durch eine bislang fehlende ärztliche Untersuchung noch aussteht. Besonders problematisch wirkt sich beim MTS aus, dass durch die meist lange Zeit vorliegende Symptomlosigkeit und das dadurch fehlende Krankheitsgefühl eine rechtzeitige Diagnose und damit die Möglichkeit einer frühzeitigen Therapie verzögert wird. Wissenschaftler gehen davon aus, dass aufgrund dessen die Diagnose "Metabolisches Syndrom" in der Praxis viel zu selten gestellt wird und damit die Betroffenen nicht frühzeitig genug sensibilisiert und therapiert werden können.

Daher ist es unter anderem Aufgabe der vorliegenden Erfindung, neuartige Verfahren und die diesem Verfahren zugrundeliegenden Substanzen zur frühzeitigen und spezifischen Diagnose von Stoffwechselstörungen, insbesondere des Metabolischen Syndroms, bereitzustellen.

Die Schlüsseltherapie des Metabolischen Syndroms beruht bisher vor allem auf einer signifikanten Gewichtreduktion, wodurch die Risikofaktoren Hyperlipidämie, Hypertonie und Kontrolle des Kohlenhydratstoffwechsels deutlich reduziert werden können. So bewirkt beispielsweise eine Verringerung des Körpergewichts um 11% eine Senkung des Mortalitätsrate an kardiovaskulären Erkrankungen und Diabetes um 25% [6]. Oft wurde bisher jedes einzelne Symptom und Krankheitsbild des Metabolischen Syndroms durch eine spezifische Therapie behandelt. So werden gegen Dyslipidämien insbesondere Lipidsenker, gegen Hypertonie insbesondere blutdrucksenkende Mittel und gegen Glukoseintoleranz beziehungsweise Typ 2-Diabetes insbesondere orale Antidiabetika und Insulinpräparate eingesetzt. Im Falle einer Adipositas wird meist auf eine diätische Ernährung zurückgegriffen, um das Gewicht des Patienten zu reduzieren. Insbesondere muss hierbei darauf geachtet werden, dass die Behandlung eines Symptoms nicht zur Verschlechterung oder Manifestierung einer anderen Facette des Metabolischen Syndroms führt. Eine häufige Folgeerkrankung des MTS ist die Arteriosklerose, die insbesondere durch durchblutungssteigernde Mittel und durch Mittel zur Behandlung koronarer Herzerkrankungen oder zur Verbesserung des Gehirnstoffwechsels therapiert wird. Bisher werden also lediglich die einzelnen Symptome der unter dem Begriff Metabolisches Syndrom zusammengefassten Krankheitsbilder behandelt, wobei eine ursächliche Therapie oder Prophylaxe des Metabolischen Syndroms noch fehlt.

Es ist daher unter anderem Aufgabe der vorliegenden Erfindung, neuartige Verfahren und die diesem Verfahren zugrundeliegenden Substanzen zur Prophylaxe und Therapie von Stoffwechselstörungen, insbesondere des Metabolischen Syndroms, bereitzustellen.

Erfindungsgemäß wird diese Aufgabe dadurch gelöst, dass Syncollinpeptide, Syncollin-kodierende Nukleinsäuren und Antisense-Nukleinsäuren, gegen Syncollinpeptide gerichtete Antikörper oder Agonisten beziehungsweise Antagonisten dieser Stoffe zur Diagnose, Prophylaxe und Therapie von Stoffwechselstörungen, insbesondere des Metabolischen Syndroms, verwendet werden.

### Syncollinpeptide

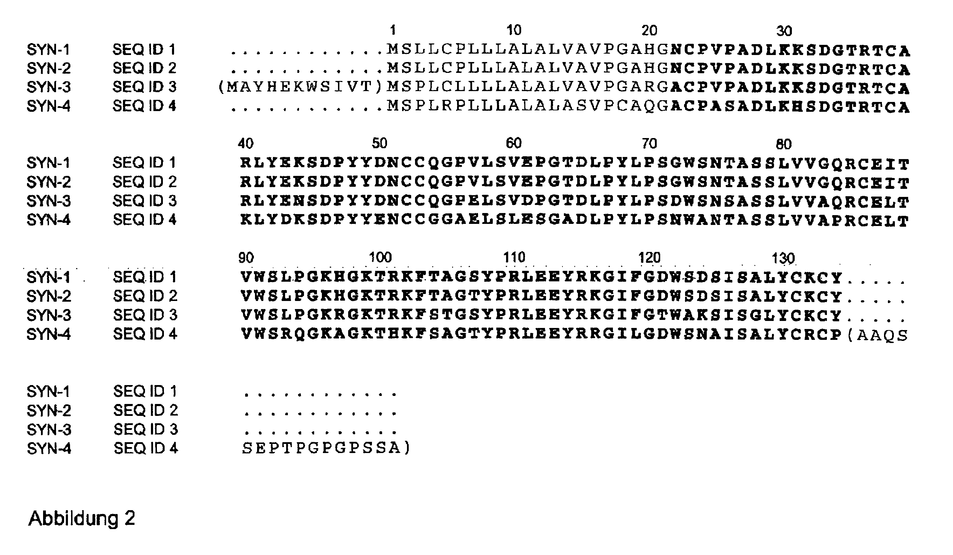

Die vorliegende Erfindung beruht insbesondere auf der Identifizierung eines neuartigen Syncollinpeptids. Die Aminosäuresequenz dieses Syncollinpeptids ist als SYN-1 in Abbildung 2 und im Sequenzprotokoll unter SEQ ID 1 dargestellt.

Weiterhin betrifft die Erfindung zu SYN-1 homologe Proteine und Peptide. Insbesondere gilt dies für die homologen Sequenzen SYN-2 (SEQ ID 2), SYN-3 (SEQ ID 3) und SYN-4 (SEQ ID 4), welche ebenfalls in Abbildung 2 und im Sequenzprotokoll dargelegt sind. Dies sind Mutanten beziehungsweise Homologe von SYN-1, teilweise aus anderen Arten stammend, welche dort eine dem SYN-1 entsprechende Rolle übernehmen.

Weiterhin umfasst die Erfindung Peptide, welche sich aus natürlich vorkommenden Syncollin-Polymorphismen und aus natürlich vorkommenden Syncollin-Mutanten ableiten, sofern sie mindestens 70% Homologie mit einer der Syncollinsequenzen entsprechend SEQ ID 1 bis SEQ ID 4 aufweisen. Homolog wird hierbei entsprechend der folgenden Definition "Bestimmung der Homologie von Sequenzen" verwendet: Zur Bestimmung der Homologie zwischen Sequenzen können Computerprogramme wie z.B. das GCG Programmpaket (Genetics Computer Group, University of Wisconsin, Madison, WI, USA) einschließlich GAP [10], BLASTP, BLASTN, FASTA [11] oder der Smith Waterman-Algorithmus zur Bestimmung der Homologien verwendet werden. Bevorzugte Parameter für den Aminosäuresequenz-Vergleich umfassen den Algorithmus von Needleman und Wunsch [12], die Vergleichsmatrix BLOSUM 62 [13], ein Lücken-Wert (Gap Penalty) von 12, ein Lückenlängen-Wert (Gap Length Penalty) von 4 und ein Homologie-Schwellenwert (Threshold of Similarity) von 0. Das GAP-Programm ist auch zur Verwendung mit den vorstehenden Parametern geeignet. Die vorstehenden Parameter sind die Fehler-Parameter (default parameters) für Aminosäuresequenz-Vergleiche, wobei Lücken an den Enden den Homologie-Wert nicht verringern. Bei sehr kurzen Sequenzen im Vergleich zur Referenz-Sequenz kann es weiterhin notwendig sein, den Erwartungswert auf bis zu 100 000 (expectation value) zu erhöhen und gegebenenfalls die Wortlänge (word size) auf bis zu 2 zu verkleinern. Weitere beispielhafte Algorithmen, Lückenöffnungs-Werte (gap opening penalties), Lückenausdehnungs-Werte (gap extension penalties) oder Vergleichsmatrizen einschließlich der im Programm-Handbuch Wisconsin-Paket (Version 9 September 1997) genannten können verwendet werden. Die Auswahl ist von dem durchzuführenden Vergleich abhängig und weiterhin davon, ob der Vergleich zwischen Sequenzpaaren, wobei GAP oder der Best Fit bevorzugt sind, oder zwischen einer Sequenz und einer umfangreichen Sequenz-Datenbank, wobei FASTA oder BLAST bevorzugt sind, durchgeführt wird. Eine mit dem oben genannten Algorithmus ermittelte Übereinstimmung von 70 % wird im Rahmen dieser Anmeldung als 70 % Homologie bezeichnet. Entsprechendes gilt für höhere und geringere Homologiegrade.

Alle Peptide, welche die obengenannten Homologiekriterien zu einem der Syncollinpeptide entsprechend SEQ ID 1 bis SEQ ID 4 erfüllen, werden erfindungsgemäß somit ebenfalls als Syncollinpeptide bezeichnet.

Syncollinpeptide können auch mit posttranslationalen Modifikationen und/oder in chemisch modifizierter Form vorliegen.

Weiterhin umfasst die Erfindung Substanzen, insbesondere Peptide, welche Syncollinaktivität besitzen. Solche syncollinanalogen Substanzen mit syncollinanaloger Aktivität fallen ebenfalls unter den Begriff Syncollinpeptide.

Weiterhin betrifft die Erfindung Syncollinpeptide und biologisch aktive Fragmente dieser Peptide als Nachweisreagenzien, Wirksubstanzen oder Zielmoleküle (Targets), insbesondere zur Behandlung, Prophylaxe oder Diagnose von Stoffwechselerkrankungen. Als biologisch aktiv wird im Sinne dieser Erfindung auch eine antigene Wirkung eines Syncollinpeptids oder eines Fragment eines Syncollinpeptids angesehen.

Weiterhin betrifft die Erfindung Fusionspeptide, welche mindestens ein Syncollinpeptid oder ein biologisch aktives Fragment eines solchen Peptids enthalten.

### Antikörper

Weiterhin betrifft die Erfindung Antikörper beziehungsweise deren Antigen-bindende Fragmente, welche mit den erfindungsgemäßen Syncollinpeptiden reagieren, vorzugsweise spezifisch an diese binden.

### Syncollin-kodierende Nukleinsäuren, Antisense-Nukleinsäuren, Vektoren und Wirtszellen

Weiterhin betrifft die Erfindung Nukleinsäuren, welche für Syncollinpeptide, insbesondere SYN-1 bis SYN-4, deren Vorläuferpeptide oder deren Fragmente, insbesondere deren biologisch aktive Fragmente, kodieren. Insbesondere betrifft die Erfindung natürlich vorkommende, Syncollin-kodierende Nukleinsäuren, insbesondere für SEQ ID 1 bis SEQ ID 4 kodierende Nukleinsäuren. Insbesondere sind hier auch nicht kodierende, beispielsweise regulatorische oder stabilisierende, Sequenzbereiche der Syncollin-kodierenden Nukleinsäuren wie beispielsweise Promotoren, Ribosomenbindungsstellen oder Terminationssequenzen der mRNA eingeschlossen. Insbesondere betrifft die Erfindung Nukleinsäuren mit den Sequenzen SEQ ID 5 bis SEQ ID 12, welche natürlich vorkommende, für die Syncollinpeptide SEQ ID 1 bis SEQ ID 4 kodierende Nukleinsäuren darstellen. SEQ ID 5 bis SEQ ID 8 stellen hierbei die cDNA-Sequenzen dar, welche den kompletten Nukleinsäuresequenzen der jeweiligen mRNA entsprechen. SEQ ID 9 bis SEQ ID 12 entsprechen den jeweiligen unmittelbar für die Syncollinpeptide SEQ ID 1 bis SEQ ID 4 kodierenden Bereiche der jeweiligen cDNA. cDNA (complementary DNA oder copy DNA) ist eine DNA, die mit Hilfe einer Reversen Transkriptase nach Vorlage einer mRNA synthetisiert wird. Diese DNA ist somit zur ursprünglichen mRNA komplementär.

Weiterhin betrifft die Erfindung Nukleinsäuren, welche zu einer der vorgenannten Nukleinsäuresequenzen, insbesondere zu einer der Nukleinsäuresequenzen SEQ ID 5 bis SEQ ID 12, homolog sind. Homolog wird hierbei entsprechend der Definition "Bestimmung der Homologie von Sequenzen" verwendet, wobei hier aufgrund der Möglichkeit stiller Mutationen in Folge des degenerierten genetischen Codes eine geringere Homologieschwelle von 60% angenommen werden muss. Alle Nukleinsäuren, welche die obengenannten Kriterien, insbesondere die Homologiekriterien, erfüllen, werden erfindungsgemäß somit als Syncollin-kodierende Nukleinsäuren bezeichnet.

Weiterhin betrifft die Erfindung Nukleinsäuren, welche aufgrund ihrer spezifischen komplementären Sequenzen als Antisense-Nukleinsäuren gegen die Syncollin-kodierenden Nukleinsäuren eingesetzt werden können. Insbesondere betrifft die Erfindung Nukleinsäuren, welche aufgrund ihrer spezifischen Bindungseigenschaften an Syncollin-kodierende Nukleinsäuren geeignet sind, als Primer oder Hybridisierungspartner zum Nachweis oder zur Quantifizierung von Syncollin-kodierenden Nukleinsäuren zu fungieren.

Weiterhin betrifft die Erfindung Nukleinsäurekonstrukte, welche die oben genannten Syncollin-kodierenden Nukleinsäuren enthalten. In einer bevorzugten Ausführungsform werden die erfindungsgemäßen Syncollin-kodierenden Nukleinsäuren mit weiteren natürlichen oder heterologen, meist regulatorischen, Sequenzen verknüpft.

Weiterhin betrifft die Erfindung Vektoren und damit transfizierte Wirtszellen, welche Syncollin-kodierende Nukleinsäuren enthalten, insbesondere Vektoren und transfizierte Wirtszellen, welche zur Herstellung und Gewinnung von Syncollinpeptiden und Syncollin-kodierende Nukleinsäuren geeignet sind.

### Erfindungsgemäßer Einsatz der Syncollinpeptide

Die vorliegende Erfindung beruht insbesondere auf der Identifizierung, dass das erfindungsgemäße Syncollinpeptid SYN-1 im Pankreasgewebe von an Stoffwechselerkrankungen leidenden Organismen im Vergleich zu gesunden Organismen deutlich erhöht ist.

Daher betrifft die Erfindung den Einsatz von Syncollinpeptiden, Syncollin-kodierenden Nukleinsäuren und Antisense-Nukleinsäuren, gegen Syncollinpeptide gerichteten Antikörper oder Agonisten beziehungsweise Antagonisten dieser Stoffe zur Diagnose, Prophylaxe und

Therapie von Stoffwechselerkrankungen, insbesondere des Metabolischen Syndroms einschließlich seiner Krankheitsbilder Adipositas, Insulinresistenz, Glukoseintoleranz, Typ 2 Diabetes, Dyslipidämie, Hypertonie, Veränderungen des Gefäßsystems und Hyperkoagulabilität, aber auch anderer Stoffwechselerkrankungen wie beispielsweise Anorexie, Bulemie oder Kachexie.

### Screeningverfahren

Insbesondere betrifft die Erfindung Verfahren zum Auffinden von Substanzen, welche die Expression, Konzentration oder Aktivität von Syncollinpeptiden oder Syncollin-kodierenden Nukleinsäuren modulieren können. Insbesondere schließt die Erfindung Verfahren ein, bei welchen eine Probe, welche mindestens ein Syncollinpeptid oder eine Syncollin-kodierende Nukleinsäure enthält, mit einer Testsubstanz in Verbindung gebracht wird und die Fähigkeit dieser Testsubstanz zur Modulation der Expression von Syncollin-kodierenden Nukleinsäuren oder der Aktivität und/oder Konzentration von Syncollinpeptiden untersucht wird.

Weiterhin betrifft die Erfindung Verfahren zum Auffinden von Substanzen, welche zur Therapie und/oder Prophylaxe von Stoffwechselerkrankungen, insbesondere des Metabolischen Syndroms und seiner Krankheitsbilder, geeignet sind. Insbesondere betrifft dies Verfahren zur Überprüfung der Eignung einer Substanz, die Expression von Syncollin-kodierenden Nukleinsäuren oder die Aktivität und/oder Konzentration von Syncollinpeptiden zu modulieren.

Weiterhin betrifft die Erfindung Verfahren zum Auffinden von Substanzen, welche die Aktivität der pankreatischen Azinuszellen modulieren können. Insbesondere betrifft die Erfindung Verfahren, bei denen durch Zusammenbringen von pankreatischen Azinuszellen, welche Syncollinpeptide oder Syncollin-kodierende Nukleinsäuren exprimieren, mit den zu testenden Substanzen die Fähigkeit dieser Substanzen überprüft werden kann, die Expression von Syncollin-kodierenden Nukleinsäuren oder die Aktivität und/oder Konzentration von Syncollinpeptiden zu modulieren.

Weiterhin betrifft die Erfindung Verfahren zur Modulierung der Aktivität der pankreatischen Azinuszellen, insbesondere durch Einsatz von Syncollinpeptiden oder Syncollinpeptidfragmenten, von gegen Syncollinpeptide gerichteten Antikörpern oder Antikörperfragmenten, von Syncollin-kodierenden Nukleinsäuren und Antisense-Nukleinsäuren oder von Agonisten beziehungsweise Antagonisten dieser Stoffe.

### Prädiktive Verfahren

Weiterhin betrifft die Erfindung Nachweisverfahren und ―substanzen zur Bestimmung der Aktivität oder der Konzentration oder der Anwesenheit oder der Abwesenheit von Syncollinpeptiden oder von Syncollin-kodierenden Nukleinsäuren in einer biologischen Probe, insbesondere zur Diagnose von Krankheiten, insbesondere von Stoffwechselerkrankungen, insbesondere des Metabolischen Syndroms. Insbesondere sind Nachweisverfahren eingeschlossen, welche das Fehlen oder Vorhandensein einer genetisch festgelegten Veränderung in Syncollinpeptiden oder in Syncollin-kodierenden Nukleinsäuren feststellen können, insbesondere in Hinblick auf einen Einsatz zur Diagnose von Stoffwechselerkrankungen. Unter Nachweis einer Stoffwechselerkrankung versteht man hier insbesondere das Auffinden von Anzeichen, insbesondere von statistisch als sicher geltenden Anzeichen, für das Vorliegen einer Stoffwechselerkrankung.

Weiterhin betrifft die Erfindung Verfahren zur Feststellung von Individuen, welche an einer Stoffwechselerkrankung, insbesondere dem Metabolischen Syndrom, leiden oder ein verändertes Risiko besitzen, an einer Stoffwechselerkrankung zu erkranken. Insbesondere betrifft die Erfindung Verfahren zur Feststellung von Individuen, welche an einer Stoffwechselerkrankung leiden oder ein verändertes Risiko besitzen, an einer Stoffwechselerkrankung zu erkranken, welche durch eine veränderte Aktivität von Syncollinpeptiden oder eine veränderte Expression von Syncollin-kodierenden Nukleinsäuren gekennzeichnet ist.

### Therapeutische Verfahren

Weiterhin betrifft die Erfindung den Einsatz von erfindungsgemäßen Syncollinpeptiden, Syncollin-kodierenden Nukleinsäuren und Antisense-Nukleinsäuren, gegen Syncollinpeptide gerichteten Antikörpern oder Agonisten beziehungsweise Antagonisten dieser Stoffe in Verfahren zur therapeutischen oder prophylaktischen Behandlung von Individuen, welche an einer Stoffwechselerkrankung, insbesondere dem Metabolischen Syndrom, leiden oder eine erhöhte Prädisposition für eine solche aufweisen. Insbesondere betrifft die Erfindung Verfahren zur Behandlung von Individuen, welche an einer Stoffwechselerkrankung leiden, welche durch eine veränderte Aktivität von Syncollinpeptiden oder eine veränderte Expression von Syncollin-kodierenden Nukleinsäuren gekennzeichnet ist. Das Verfahren betrifft insbesondere die Verabreichung von Syncollinmodulatoren, vorzugsweise in einer pharmakologisch akzeptablen Formulierung, oder den Einsatz von spezifischen Vektoren im Rahmen einer Gentherapie. Bevorzugte Syncollinmodulatoren sind insbesondere Syncollinpeptide, gegen Syncollinpeptide gerichtete Antikörper oder Antikörperfragmente, Syncollin-kodierende Nukleinsäuren oder deren Fragmente, gegen Syncollin-kodierende Nukleinsäuren gerichtete Antisense-Nukleinsäuren oder Ribozyme und Agonisten oder Antagonisten der vorgenannten Stoffe.

Solche therapeutischen oder prophylaktischen Verfahren können beispielsweise auf einer Modulation der Synthese und/oder Sekretion von Enzymen oder Hormonen (insbesondere Verdauungsenzyme wie Trypsin oder Lipase und Hormone wie Insulin, Glucagon oder Leptin), des Kohlenhydratstoffwechsels (insbesondere Glykolyse, Gluconeogenese) oder des Lipidstoffwechsels (insbesondere Lipogenese, Lipolyse) basieren.

### Detaillierte Beschreibung der Erfindung

Die vorliegende Erfindung basiert auf der Identifizierung eines Syncollinpeptids, das mit dem Auftreten von Stoffwechselerkrankungen in Mäusen, welche dem Metabolischen Syndrom des Menschens entsprechen, hochgradig korreliert. Diese hier erstmals festgestellten krankheitsspezifischen Konzentrationsänderungen von Syncollinpeptiden dienen als Grundlage für die erfindungsgemäße Verwendung der Syncollinpeptide als spezifische Marker einer Stoffwechselerkrankung und als spezifische Ansatzpunkte zur Entwicklung neuartiger therapeutischer und prophylaktischer Verfahren zur Bekämpfung und Vermeidung von Stoffwechselerkrankungen, insbesondere des Metabolischen Syndroms.

### Tiermodell

Spezielle Tiermodelle stellen wichtige Hilfsmittel zur Erforschung der Ursachen, Mechanismen und Folgen von Stoffwechselerkrankungen dar. Als Tiermodell für Stoffwechselerkrankungen im Bereich des Metabolischen Syndroms ist die sogenannte ob/ob-Maus in der Forschung weit verbreitet. Als ob/ob-Mäuse werden im Rahmen dieser Erfindung homozygote Mäuse des Mausstammes B6.V-Lep(ob) (Jackson Laboratory, Bar Harbor, ME, USA) bezeichnet. Diese Mäuse zeichnen sich durch viele Krankheitsbilder und Symptome aus, welche ebenfalls bei Menschen mit Metabolischem Syndrom auftreten. Insbesondere sind dies starke Gewichtszunahme und Adipositas bis zum dreifachen Normalgewicht, Hyperphagie, Hyperglykämie, Glukoseintoleranz, Diabetes, erhöhte Insulinspiegel im Plasma, Hypometabolismus und Hypothermie sowie eine starke Erhöhung der Zahl und Größe der Adipozyten. Bei der ob/ob-Maus liegt eine Mutation vor, die zu einem falschen Stop-Codon des Leptin-Gens führt, so dass nur ein defektes, zu kurzes "Leptin" synthetisiert wird. Das von Adipozyten produzierte Hormon Leptin, das eine Schlüsselrolle bei der Appetitregulierung und Gewichtskontrolle spielt, korreliert mit dem Auftreten einer Adipositas, einer Insulinresistenz und anderen Krankheitsbildern des Metabolischen Syndroms. Im Rahmen der WOSCOPS-Studie konnte gezeigt werden, dass bei Menschen ein erhöhter Leptin-Wert mit einem erhöhten Risiko für das Metabolische Syndrom korreliert [14].

### Syncollinpeptide

Die Erfindung beruht insbesondere auf der Identifizierung des Syncollinpeptids SYN-1. Dieses stellt ein neuartiges, bisher unbekanntes Peptid mit der Aminosäuresequenz SEQ ID 1 dar, welches hochgradig mit dem Vorliegen von Stoffwechselerkrankungen korreliert. Weiterhin betrifft die Erfindung zu SYN-1 homologe Proteine und Peptide. Insbesondere gilt dies für die homologen Sequenzen SYN-2 (SEQ ID 2), SYN-3 (SEQ ID 3) und SYN-4 (SEQ ID 4), welche ebenfalls in Abbildung 2 und im Sequenzprotokoll dargelegt sind.

Wir konnten erstmalig das Vorkommen eines Syncollinpeptids in Mäusen direkt nachweisen. Zuvor war das Vorkommen von Syncollinpeptiden lediglich in der Ratte nachgewiesen worden [15]. Zu beachten ist hierbei, dass der Orginaleintrag der Sequenz des Syncollins der Ratte (SwissProt 035775; SEQ ID 3) fälschlicherweise N-terminal um 11 Aminosäuren verlängert ist [16] (siehe auch Abbildung 2). Trotz weiterer gezielter Forschung in verschiedensten Modellorganismen, wie zum Beispiel Drosophila melanogaster [17], wurden bisher noch keine Syncollinpeptide in anderen Tierarten nachgewiesen. Es existieren lediglich indirekte Hinweise auf das Vorkommen von Syncollin in anderen Tierarten. Kawai et al. konnten 2001 in einer murinen Milz-cDNA-Datenbank eine Nukleinsäuresequenz identifizieren, welche eine 86,6%ige Homologie zur Nukleinsäuresequenz des Rattensyncollins besitzt [18] und konnten daraus die vermutete Aminosäuresequenz des Mäusesyncollins ableiten (Swiss-Prot Q9DC81, SEQ ID 1, Abbildung 2). Strausberg et al konnten 2001 eine Mutante dieser Sequenz in einer murinen Colon-cDNA-Datenbank identifizieren, deren Translation zu einem Syncollinpeptid führen würde, bei welchem im Vergleich zu Swiss-Prot Q9DC81 an Position 107 Serin gegen Threonin ausgetauscht wäre (Swiss-Prot Q8VCK7, SEQ ID 2, Abbildung 2). Neben diesen beiden murinen cDNA-Sequenzen wurde auch eine humane homologe Nukleinsäuresequenz im Rahmen des NCBI Annotation Project identifiziert, deren Translation zu einem Protein führen würde, welches eine Homologie von 72,2% zum entsprechenden Bereich des Ratten-Syncollins besitzt (NCBI XP_092067, SEQ ID 4, Abbildung 2) [19].

Allerdings konnten bisher noch keine Syncollinpeptide direkt in diesen Organismen nachgewiesen werden, sondern lediglich Nukleinsäuren, welche für solche hypothetischen Syncollinpeptide kodieren könnten. Hierbei ist besonders zu beachten, dass die Existenz einer mRNA beziehungsweise einer korrespondierenden cDNA noch keinesfalls als Nachweis für die Existenz des kodierten Peptids angesehen werden kann, da noch verschiedene prä- und posttranslationale Prozessierungs- und Modifikationsschritte nötig sein können, um das biologisch aktive Protein zu synthetisieren. Exemplarisch kann hier das Proglucagon-Gen genannt werden, dessen Expression zunächst zu einem großen Precursorprotein, dem Proglucagon, führt. Dieses Proglucagonprotein wird nun zell- und gewebespezifisch und zusätzlich in Abhängigkeit externer Zustände des Organismus, insbesondere des Ernährungszustands, in verschiedenste Peptide gespalten, welche alle unterschiedliche biologische Funktionen und Aufgaben besitzen [20]. Neben diesen exemplarisch dargestellten posttranslationalen Modifikationen können aber auch weitere Prozesse stattfinden, welche dafür sorgen, dass die von der mRNA beziehungsweise cDNA kodierende Aminosäuresequenz nicht in das entsprechende, hypothetische Protein translatiert wird. Beispiele hierfür sind unter anderen alternatives Splicing, Regulation der Proteinsynthese durch die Häufigkeit bestimmter tRNAs, Regulation der Synthese-, Turnover- und Abbauraten der mRNA, spezifische Inhibierung der mRNA, intrazellulare Lokalisation der mRNA, Kontrolle der Translation durch RNA-binding proteins (RBPs), Regulation der Proteinsynthese durch intrazellulare Substanzen (zum Beispiel cAMP, Ca⁺⁺ oder Phosphorylierungen) [20-24] oder auch posttranslationale und dadurch nicht genetisch festgelegte Modifizierungen der Proteine (zum Beispiel Bindung von freiem Cystein an ein Cystein in der Peptidsequenz, von Pyroglutaminsäure, Methyl-, Acetyl-, Farnesyl-, Biotinyl-, Stearoyl-, Palmityl-, Lipoyl -, C-Mannosyl-, Phosphor- und Sulfatgruppen, Glykosylierungen, Amidierungen, Deamidierungen, Ubiquitinierungen, Prenylierungen) [25]. Von dem Vorkommen einer bestimmten mRNA und/oder korrespondierenden cDNA darf keinesfalls direkt auf das Vorkommen bestimmter davon kodierter Peptide geschlossen werden, da vielfältige regulatorische Prozesse beteiligt sind, welche die Prozessierung dieser Peptide präund posttranslational steuern und beeinflussen können.

Bei Ratten wurde Syncollin im Pankreasgewebe und, in wesentlich geringeren Konzentrationen, in den Ohrspeicheldrüsen nachgewiesen [15]. Syncollin konnte als ein aus 113 Aminosäuren bestehendes Peptid identifiziert werden, welches aus einem Vorläufermolekül durch Abspaltung einer N-terminalen Signalsequenz von 21 Aminosäuren entsteht und sich im membranassoziierten Zustand zu Homo-Hexameren zusammenlagert [16]. Syncollin bindet kalziumabhängig an Syntaxin 2, ein integrales Membranprotein, welches an der Anbindung und Verschmelzung intrazellularer Vesikel mit der Plasmamembran beteiligt ist und interagiert spezifisch mit "Pancreatic zymogen granule membrane protein GP-2", einem membranverankerten Glycoprotein der Zymogenvesikel [15, 26]. Kürzlich gelang es, SyncollinmRNA-defizitäre Mäuse herzustellen. Diese sind vital, fertil und zeigten keine sichtbaren Veränderungen in Bezug auf Anatomie und Morphologie der Tiere. Insbesondere zeigen Pankreas, Azinuszellen und Zymogengranula keine morphologischen Anormalitäten. SyncollinmRNA-defizitäre Mäuse zeigen lediglich eine erhöhte Anfälligkeit für Pankreatitis und eine etwas verzögerte Freisetzung intrazellulärer Zymogen-Granula [27, 28]. Hierbei ist zu beachten, dass auch in diesen Versuchen kein Syncollin-Protein direkt in den Mäusen nachgewiesen wurde, sondern lediglich das Vorhandensein beziehungsweise Fehlen einer Nukleinsäure, welche homolog zur der des Ratten-Syncollins ist. In der vorliegenden Erfindung wird hingegen erstmals der direkte Nachweis des Vorhandenseins eines Syncollinpeptids in Mäusen dargelegt. Aufgrund des Vorkommens eines Peptids oder der korrespondierenden Nukleinsäure im Pankreas kann nicht auf seine Beteilung an der Regulation von Stoffwechselvorgängen beziehungsweise bei seiner Konzentrationsänderung auf eine Beteiligung am Auftreten einer Stoffwechselkrankheit geschlossen werden. So sind in Pankreasextrakten viele weitere Peptide und Proteine enthalten. Analysen der mRNA-Pools von Pankreata der von uns verwendeten Wildtypmäuse konnten über 13000 Nukleotidsequenzen nachweisen, welchen mehr als 2900 verschiedene Proteine und Peptide zugeordnet werden können. Eine Übersicht über diese Proteine und Peptide ist in der UniGene-Datenbank (NCBI, National Center for Biotechnology Information) "Mus musculus pancreas C57BL/6J adult (dbEST Library ID.1870)" zu finden. Daher kann aus dem alleinigen Nachweis eines Peptids im Pankreas keinesfalls unmittelbar auf eine Beteilung an der Regulation von Stoffwechselvorgängen oder einer Beteiligung am Entstehen von Stoffwechselerkrankungen geschlossen werden. Weiterhin können auch bestimmte Peptide und Proteine im Pankreas bestimmter Individuen in ihrer Konzentration verändert sein, ohne dass eine Stoffwechselerkrankung vorliegt, sondern stattdessen andere sich auf den Pankreas erstreckende Erkrankungen wie beispielsweise akute oder chronische Pankreatitiden, Pankreaskarzimome oder erbliche und angeborene Krankheiten wie Mukoviszidose oder Shwachman-Syndrom. Diese Krankheiten äußern sich ebenfalls in spezifischen Konzentrationsänderungen bestimmter Pankreaspeptide- und ―proteine wie beispielsweise Cystic Fibrosis Transmembrane Conductance Regulator (CFTR) bei Mukoviszidose oder Serin-Protease-Inhibitor Kazaltyp 1, Trypsinogen, Amylase und C-reaktives Protein (CRP) bei einer Pankreatitis [29]. Sowohl aus dem alleinigen Nachweis eines Peptids im Pankreas als auch aus einer Konzentrationsänderung eines Peptids im Pankreas kann somit nicht unmittelbar auf eine Beteiligung an Stoffwechselprozessen und Stoffwechselerkrankungen geschlossen werden, wie es in bisherigen Untersuchungen und Veröffentlichungen, insbesondere bei Untersuchungen an cDNA-Bibliotheken, oft erfolgt. Stattdessen muss unbedingt, wie im Rahmen dieser Erfindung dargelegt, eine eindeutige Korrelation zwischen dem Auftreten einer Stoffwechselerkrankung und dem Nachweis einer Konzentrationsänderung eines bestimmten Peptids erfolgen, wie sie insbesondere für SYN-1 in Beispiel 7 und Beispiel 8 beschrieben ist.

Somit konnten bisher weder Syncollinpeptide in Mäusen direkt nachgewiesen werden noch wurden Syncollinpeptide als Substanzen beschrieben, die mit dem Auftreten einer Stoffwechselstörung, insbesondere des Metabolischen Syndroms mit seinen Krankheitsbildern Adipositas, Insulinresistenz, Glukoseintoleranz, Typ2 Diabetes, Dyslipidämie und Hypertonie, in Verbindung stehen.

Die Erfindung betrifft weiterhin zu SYN-1 bis SYN-4 homologe Peptide. Homolog wird hierbei entsprechend der Definition "Bestimmung der Homologie von Sequenzen" verwendet. Dabei ist es möglich, dass sich die Sequenzen von den Sequenzen SEQ ID 1 bis SEQ ID 4 unterscheiden. Als Syncollinpeptide werden insbesondere auch die Peptide bezeichnet, welche eine Homologie von mindestens 70% zu einem der Syncollinpeptide SYN-1 bis SYN-4 beziehungsweise einer der Aminosäuresequenzen SEQ ID 1 bis SEQ ID 4 aufweisen. Dabei sind Punktmutationen, Deletionen, Insertionen, Inversionen, Translokationen und N-terminale und/oder C-terminale Verlängerungen zulässig, solange nicht mehr als 30% Abweichung von einer der Aminosäuresequenzen von SYN-1 bis SYN-4 beziehungsweise SEQ ID 1 bis SEQ ID 4 auftritt. Dabei müssen mindestens 85 Aminosäuren aus einer der Aminosäuresequenzen von SYN-1 bis SYN-4 beziehungsweise SEQ ID 1 bis SEQ ID 4 beibehalten bleiben. Als N- oder C-terminale Verlängerung kommen nur solche Aminosäurensequenzen in Frage, welche natürlicherweise in Syncollinpeptiden an den entsprechenden Sequenzpositionen vorkommen. Weiterhin sind Peptide eingeschlossen, welche sich aus natürlich vorkommenden Syncollin-Polymorphismen und aus natürlich vorkommenden Syncollin-Mutanten ableiten, sofern sie mindestens 70% Homologie mit einer der Aminosäuresequenzen der entsprechenden Syncollinpeptide aufweisen. So stellt beispielsweise SYN-2 eine Mutation von SYN-1 dar, bei welcher an Position 107 Serin gegen Threonin ausgetauscht ist. Insbesondere sind alle natürlich vorkommenden Allele, Mutanten und Polymorphismen der sowie gewebespezifisch exprimierte Syncollinpeptid-Varianten der Aminosäuresequenzen von SYN-1 bis SYN-4 beziehungsweise SEQ ID 1 bis SEQ ID 4 eingeschlossen. Sequenzeinträge von Syncollinpeptiden können auch in anderen, von SWISS-Prot oder NCBI ENTREZ verschiedenen, Sequenzdatenbanken, wie beispielsweise Protein Information Resource (PIR), Protein Research Foundation (PRF) oder Protein Data Bank (PDB) enthalten sein oder aus Nukleinsäuresequenzdatenbanken, welche Syncollin-kodierende Nukleinsäuresequenzen enthalten, wie beispielsweise GenBank, EMBL und DDBJ, abgeleitet sein. Dies gilt sowohl für bereits bestehende als auch in Zukunft hinzukommende Datenbankeinträge. Eingeschlossen sind sowohl Syncollinpeptide mit als auch ohne Signalsequenz, Vorläuferformen von Syncollinpeptiden, die noch nicht prozessiert sind, sowie bereits teilweise oder komplett prozessierte Syncollinpeptide, lösliche Syncollinpeptide und membranständige Syncollinpeptide, wobei die membranständigen Syncollinpeptide sowohl über transmembranäre Aminosäuresequenzen als auch über posttranslationale Modifikationen, wie zum Beispiel einen Glykosyl-Phosphatidyl-Inositol-(GPI-)Anker, mit einer Zell- oder Organellenmembran verbunden sein können. Ferner sind Variationen der Syncollinpeptid-Sequenz, die durch alternatives Splicing, durch alternative Translationsstart- und -endpunkte, durch RNA-Editing, durch alternative posttranslationale Modifikationen sowie weitere natürliche oder künstliche Mechanismen entstanden sind, eingeschlossen. Eingeschlossen sind weiterhin Syncollinpeptide, welche in posttranslationalen oder chemischen Modifikationsformen vorliegen. Ein chemisch oder posttranslational modifiziertes Peptid kann sowohl aus D- als auch aus L-Aminosäuren, sowie aus Kombinationen von D- und L-Aminosäuren bestehen und kann sowohl natürlich vorkommen, rekombinant hergestellt werden oder chemisch synthetisiert werden. Außerdem können in diesen Peptiden ungewöhnliche Aminosäuren, das heißt Aminosäuren, die nicht zu den 20 Standardaminosäuren gehören, enthalten sein. Beispiele für ungewöhnliche Aminosäuren sind unter anderem: alpha-Aminobuttersäure, beta-Aminobuttersäure, beta-Alanin, beta-Aminoisobuttersäure, Norvalin, Homoserin, Norleucin, gamma-Aminobuttersäure, Thioprolin, 4-Hydroxyprolin, alpha-Aminoadipinsäure, Diaminobuttersäure, 4-Aminobenzoesäure, Homocystein, alpha-Aminopenicillansäure, Histamin, Ornithin, Glycin-Prolin-Dipeptid, Hydroxylysin, Prolin-Hydroxyprolin-Dipeptid, Cystathionin, Citrullin, Ethionin, Seleno-Cystein, Pyrrolysin. Als posttranslationale oder chemische Modifikationen sind unter anderem Modifizierungen der Aminosäuresequenzen durch folgende Strukturen möglich: Bindung von freiem Cystein an ein Cystein in der Peptidsequenz, Bindung von Pyroglutaminsäure, Methyl-, Acetyl-, Farnesyl-, Biotinyl-, Stearoyl-, Palmityl-, Lipoyl-, C-Mannosyl-, Phosphor- und Sulfatgruppen, Glykosylierungen, Amidierungen, Deamidierungen, Ubiquitinierungen, Prenylierungen usw. Weiterhin sind als mögliche Modifikationen der Syncollinpeptide insbesondere die in der Delta Mass-Datenbank der Association of Biomolecular Resource Facilities aufgeführten Modifikationen zu berücksichtigen [25].

Weiterhin umfasst die Erfindung Substanzen, insbesondere Peptide, welche Syncollinaktivität besitzen. Unter Syncollinaktivität, Aktivität von Syncollin oder Aktivität von Syncollinpeptiden, wie sie im Rahmen der vorliegenden Erfindung synonym benutzt werden, versteht man allgemein die biologische Aktivität, welche von Syncollinpeptiden auf syncollinempfindliche Zellen oder Substrate, beispielsweise Proteine, ausgeübt wird und welche in vivo oder in vitro bestimmt werden kann. Dies kann sowohl eine direkte Aktivität, beispielsweise auf spezifische Zielmoleküle wie Targets, Bindungspartner oder Rezeptoren, oder eine indirekte Aktivität, wie sie beispielsweise durch spezifische Signalübertragungswege oder "second messenger" vermittelt sind, sein. Solche syncollinanalogen Substanzen mit syncollinanaloger Aktivität fallen ebenfalls unter den Begriff Syncollinpeptide. Eine Aktivitätsänderung einer Substanz kann insbesondere auch durch eine Konzentrationsänderung der Substanz bedingt sein.

Weiterhin umfasst die Erfindung Fragmente von Syncollinpeptiden, insbesondere biologisch aktive Fragmente von SYN-1 bis SYN-4. Als biologisch aktiv wird im Sinne dieser Erfindung auch eine antigene Wirkung eines Syncollinpeptids oder eines Fragment eines Syncollinpeptids angesehen.

Weiterhin betrifft die Erfindung Fusionspeptide, chimärische Peptide oder Proteinkomplexe, welche mindestens ein Syncollinpeptid oder ein biologisch aktives Fragment eines Syncollinpeptids enthalten. Insbesondere kann der Nicht-Syncollinpeptid-Anteil einen Bereich enthalten, der eine hohe Affinität für einen Liganden aufweist, so dass solche Fusionspeptide bevorzugt zur Aufreinigung rekombinant hergestellter Syncollinpeptide eingesetzt werden können. Beispiele hierfür sind heterologe Sequenzen von Peptiden, wie zum Beispiel Poly-Histidinsequenzen oder Hemagglutinin-Epitope (HA-tag), oder Proteinen, wie zum Beispiel Maltosebindenden Proteinen, Glutathion-S-Transferase (GST), oder Proteindomänen, wie zum Beispiel der GAL-4 DNA-Bindungsdomäne oder der GAL4-Aktivierungsdomäne. Weiterhin kann der Nicht-Syncollinpeptid-Anteil Signalsequenzen wie beispielsweise heterologe Signalsequenzen zur verbesserten Expression rekombinanter Syncollinpeptide, Markersequenzen wie beispielsweise fluoreszierende GFP-(Green Fluorescent Protein-)Sequenzen oder Transportsequenzen wie beispielsweise HIV-TAT-Sequenzen oder Antennapedia-Sequenzen enthalten, aber auch andere nichtpeptidische Substanzen wie beispielsweise pharmazeutisch aktive Substanzen wie Diphterie- oder Anthrax-Toxine, wie sie bereits zur gezielten Tumorbekämfung eingesetzt werden [30, 31]. Syncollinpeptid-Anteil und Nicht-Syncollinpeptid-Anteil können dabei kovalent oder nichtkovalent gekoppelt sein.

Weitere Ausführungsformen der Erfindung sind Verfahren zur Gewinnung von Syncollinpeptiden. Hierbei kann es sich um eine Gewinnung bereits vorhandener Syncollinpeptide beziehungsweise ihrer Vorläuferproteine aus biologischen Quellen, beispielsweise unter Verwendung von Chromatographiemethoden oder elektrophoretischen Methoden oder eine gezielte Herstellung dieser Peptide, beispielsweise mittels rekombinanter Expressionssysteme oder chemischen Synthesen, handeln. Die so gewonnenen Syncollinpeptide können unter anderem als Standards in Verfahren zur Quantifizierung der jeweiligen Syncollinpeptide oder als Antigene zur Herstellung von Syncollinpeptid-Antikörpem Verwendung finden. Zu den dem Fachmann bekannten und geeigneten Methoden zur Isolierung und Gewinnung von Syncollinpeptiden gehört unter anderem rekombinante Expressionsverfahren. Zur rekombinanten Expression von Syncollinpeptiden werden zum Beispiel Nukleinsäuresequenzen, welche für Syncollinpeptide oder deren Vorläuferproteine kodieren, in Kombination mit geeigneten regulatorischen Nukleinsäuresequenzen wie Promotoren oder Selektionsmarkern mit dem Fachmann bekannten molekularbiologischen Methoden in einen Expressionsvektor eingefügt. Ein dazu geeigneter Vektor ist zum Beispiel der Vektor pcDNA3.1 der Firma Invitrogen, Carlsbad, CA. Die so hergestellten Expressionsvektoren können dann in geeignete Zellen zum Beispiel durch Elektroporation eingefügt werden. Hierbei können insbesondere Zellsysteme wie zum Beispiel Bakterien wie Escherichia coli und Bacillus subtilis, Hefezellen wie Pichia pastoris, Hansenula polymorpha, Kluyveromyces lactis und Saccharomyces cerevisiae, Pflanzenzellen wie Solanum tuberosum- oder Nicotiana tabacum-Zellen, Insektenzellen wie zum Beispiel Spodoptera frugiperda (Sf-9) Zellen oder Säugerzellen wie COS (African Green Monkey Kidney Cells) Zellen verwendet werden.

Eine weitere Ausführungsform dieser Erfindung ist die Gewinnung von Syncollinpeptiden oder deren Vorläuferproteinen aus biologischen Proben oder aus Zellkulturmedien oder Zelllysaten von rekombinanten Expressionssystemen, zum Beispiel mit Reverse-Phase-Chromatographie, Affinitätschromatographie, Ionenaustauschchromatographie, Gelfiltration, Massenspektrometrie, Elektrophorese, Isoelektrischer Fokussierung usw. oder mit anderen Methoden wie präparativer Immunpräzipitation, Ammoniumsulfatfällung, Extraktion mit organischen Lösungsmitteln usw.

Die Herstellung der Syncollinpeptide durch chemische Synthese kann zum Beispiel nach dem Merrifield-Festphasen-Syntheseprotokoll unter Verwendung von Syntheseautomaten, die von verschiedenen Herstellern erhältlich sind, erfolgen. Werden nach den zuvor beschriebenen Verfahren die Vorläuferproteine der erfindungsgemäßen Syncollinpeptide gewonnen, so können in anschließenden Arbeitsschritten mittels biologischer, chemischer oder physikalischer Modifikationen dieser Vorläuferproteine die erfindungsgemäßen Syncollinpeptide oder deren biologisch aktive Fragmente aus diesen Vorläufern gewonnen werden. Solche Modifikationen können beispielsweise proteolytischer Verdau mit spezifischen Enzymen, gezielte chemische Spaltung der Vorläuferproteine, zum Beispiel analog dem Vorgehen bei der Edman-Sequenzierung von Proteinen, und Spaltung der Vorläuferproteine durch physikalische Prozesse, wie sie zum Beispiel bei massenspektrometrischen Sequenzierungsmethoden (zum Beispiel durch Zuführung von Kollisionsenergie mit einem Stoßgas (zum Beispiel Helium oder Stickstoff) bei ElectroSpray Ionisation Tandem Mass Spectrometry (ESI-MS-MS)) ablaufen, sein.

### Syncollin-Antikörper

Weiterhin betrifft die Erfindung Antikörper, welche mit den erfindungsgemäßen Syncollinpeptiden, deren Vorläuferpeptiden oder deren Fragmenten reagieren, vorzugsweise spezifisch an diese binden. Im Rahmen der vorliegenden Erfindung werden als Antikörper insbesondere Immunoglobuline oder immunologisch aktive Bestandteile solcher Immunoglobuline angesehen. Die Antikörper können monoklonale, oligoklonale oder polyklonale Antikörper sein. Die Antikörper können sowohl humane als auch nicht-humane, vorzugsweise von Mäusen, Ratten, Kaninchen oder Ziegen stammende, Antikörper sein. Die Antikörper können auch rekombinante Antikörper sein, beispielsweise chimärische oder humanisierte Antikörper. Als Antikörper werden im Rahmen der Erfindung auch immunologisch aktive Bestandteile der Immunoglobuline angesehen, insbesondere single-chain-Antikörper, Intrabodies, Fab-(fragment, antigen binding-), Fab2- oder scFv-(single chain Fv fragment) Fragmente. Weiterhin können die Antikörper mit anderen Substanzen kovalent oder nichtkovalent gekoppelt sein. Vorzugsweise können Markersubstanzen wie Enzyme (insbesondere Meerrettich-Peroxidase, alkalische Phosphatase, beta-Galactosidase oder Acetylcholinesterase), Coenzyme beziehungsweise prosthetische Gruppen (insbesondere Biotin/Streptavidin oder Biotin/Avidin), Fluoreszenzmarker (insbesondere Fluoresceine, Fluorescein-Isothiocyanat, Rhodamin, Phycoerythrin, Umbelliferon oder Dansylchlorid), Luminenszenzmarker (insbesondere Luminol, Luciferase, Luciferin oder Aequorin) oder radioaktive Markersubstanzen (insbesondere I-125, I-131, S-35, P-32 oder H-3) an die Antikörper gekoppelt werden. Weiterhin können auch pharmazeutisch aktive Substanzen wie beispielsweise Toxine oder Antibiotika oder Kontrastmittel an die Antikörper gekoppelt werden.

Eine weitere Ausführungsform der Erfindung ist die Herstellung und Gewinnung vorgenannter Antikörper oder Antikörperfragmente unter Verwendung von Syncollinpeptiden oder deren Fragmenten. Die Gewinnung der Antikörper geschieht in üblicher, dem Fachmann vertrauter Weise. Eine bevorzugte Ausführungsform ist die Herstellung und Gewinnung von syncollinspezifischen Antikörpern, insbesondere von syncollinspezifischen Antikörpern, welche neo-Epitope erkennen. Oligo- und polyklonale Antikörper können durch Immunisierungen von Versuchstieren wie zum Beispiel Mäusen, Ratten, Kaninchen oder Ziegen hergestellt werden. Monoklonale Antikörper können zum Beispiel durch Immunisierungen von Versuchstieren, wie zum Beispiel Mäusen oder Ratten, und anschließender Anwendung von Hybridomatechniken, wie zum Beispiel Elektrofusion, oder aber über rekombinante Versuchsansätze, wie zum Beispiel über Antikörperbanken wie die HuCAL® -Antikörperbank der Firma MorphoSys, Martinsried, Deutschland, oder andere dem Fachmann bekannte, rekombinante Herstellungsverfahren gewonnen werden.

### Syncollin-kodierende Nukleinsäuren

Weiterhin betrifft die Erfindung Nukleinsäuren, welche für Syncollinpeptide, insbesondere SYN-1 bis SYN-4, deren Vorläuferpeptide oder deren Fragmente, insbesondere deren biologisch aktiver Fragmente, kodieren. Als Nukleinsäuren werden DNA, RNA und DNA-RNA-Hybridmoleküle, sowohl natürlichen Ursprungs als auch synthetisch oder rekombinant hergestellt, angesehen. Als kodierende Nukleinsäuren gelten insbesondere genomische DNA-Sequenzen, transkribierte mRNA-Sequenzen, sowohl in unprozessierter als auch in prozessierter Form und cDNA-Sequenzen.

Insbesondere betrifft die Erfindung natürlich vorkommende, Syncollin-kodierende Nukleinsäuren, insbesondere für SEQ ID 1 bis SEQ ID 4 kodierende Nukleinsäuren. Insbesondere betrifft die Erfindung Nukleinsäuren mit den Sequenzen SEQ ID 5 bis SEQ ID 12, welche natürlich vorkommende, für die Syncollinpeptide SEQ ID 1 bis SEQ ID 4 kodierende Nukleinsäuren darstellen. SEQ ID 5 bis SEQ ID 8 stellen hierbei die cDNA-Sequenzen dar, welche den kompletten Nukleinsäuresequenzen der jeweiligen mRNA entsprechen. SEQ ID 9 bis SEQ ID 12 entsprechen den für die Syncollinpeptide SEQ ID 1 bis SEQ ID 4 kodierenden Bereiche der jeweiligen cDNA. Weiterhin sind Fragmente der Syncollin-kodierenden Nukleinsäuren eingeschlossen, insbesondere für den Einsatz als spezifische Hybridisierungssequenzen oder Primersequenzen. Weiterhin können solche Fragmente für spezielle Bereiche der Syncollinpeptide kodieren, insbesondere biologisch aktive oder immunogene Bereiche oder als spezifische Epitope wirkende Bereiche. Insbesondere sind auch nicht direkt für das jeweilige Syncollinpeptid kodierende Sequenzbereiche der entsprechenden Nukleinsäuren eingeschlossen. Dies können beispielsweise regulatorische oder stabilisierende Sequenzbereiche wie Promotoren, Ribosomenbindungsstellen oder Terminationssequenzen sein. Auch können die Nukleinsäuren noch Sequenzen enthalten, welche für Signalsequenzen der primären Translationsprodukte kodieren, die während einer eventuell stattfindenden Prozessierung abgespalten werden und im fertig prozessierten Syncollinpeptid nicht mehr vorliegen. Dies gilt beispielsweise für die Nukleotidsäuresequenzen SEQ ID 5 bis SEQ ID 8. Solche nicht direkt für das jeweilige Syncollinpeptid kodierenden Sequenzen können zu einer therapeutischen Regulation der Konzentration der Syncollinpeptide beziehungsweise der Syncollin-kodierenden Nukleinsäuren von großer Bedeutung sein, da beispielsweise durch Modifikation der Promotorsequenz der Syncollin-kodierende Nukleinsäuren die Konzentration und damit die biologische Aktivität der Syncollinpeptide moduliert werden kann. Auch in diagnostischer Hinsicht können nicht-kodierende Bereiche der Syncollin-kodierenden Nukleinsäuren genutzt werden. Beispielsweise kann die Konzentration einer Syncollin-kodierenden mRNA anhand spezifischer Nukleinsäuresequenzen (sogenannter Tags) bestimmt werden, unabhängig davon ob sie aus dem kodierenden oder nicht-kodierenden Bereich der mRNA stammen.

Weiterhin betrifft die Erfindung Varianten der vorgenannten Nukleinsäuren, welche zu einer der vorgenannten Nukleinsäuresequenzen, insbesondere zu einer der Nukleinsäuresequenzen SEQ ID 5 bis SEQ ID 12, homolog sind. Homolog wird hierbei entsprechend der Definition "Bestimmung der Homologie von Sequenzen" verwendet, wobei hier aufgrund der Möglichkeit stiller Mutationen in Folge des degenerierten genetischen Codes eine geringere Homologieschwelle von 60% angenommen werden muss. Solche Unterschiede in der Nukleotidsequenz müssen sich in Folge des degenerierten genetischen Codes nicht unbedingt auf die Aminosäuresequenz des kodierenden Syncollinpeptids auswirken. Insbesondere können Nukleotidsäuresequenzen in der Form verändert sein, dass sie bevorzugt Codons enthalten, welche für ein spezielles Expressionssystem, beispielsweise *E*. *coli*, Hefezellen, Insektenzellen, CHO-(chinese hamster ovary-)Zellen oder humane Zellen, optimiert sind. Nukleinsäurevarianten können natürlich vorkommen, wie Allele (gleicher Genlocus), Homologe (unterschiedliche Genloci) oder Orthologe (unterschiedliche Organismen) einer bestimmten Gens, oder durch künstliche Mechanismen, insbesondere Mutagenesetechniken mittels chemischen Mutagenen wie beispielsweise 5-Bromuracil, 2-Aminopurin, Dimethylsulfat, Diazomethan, N,N-Dimethylnitrosamin, Methylmethansulfonat oder Salpetrige Säure oder mittels physikalischen Mutagenen wie beispielsweise ionisierende Strahlung oder ultraviolette Strahlung, entstanden sein. Die Nukleinsäurevarianten können durch Substitutionen, Deletionen, Insertionen oder Inversionen in der Nukleinsäuresequenz entstanden sein und können sowohl kodierende als auch nicht kodierende Bereiche der Nukleinsäuresequenzen umfassen. Allelische Varianten können sowohl für funktionale als auch nicht-funktionale Peptide kodieren, wobei funktionale allelische Varianten bevorzugt eine oder wenige konservative Aminosäuresubstitutionen in einem nicht für die Funktion essentiellen Bereich des Peptids besitzen, während nicht-funktionale allelische Varianten bevorzugt nicht-konservative Aminosäuresubstitutionen, Insertionen, Deletionen oder Aminosäuresubstitutionen in einem für die Funktion essentiellen Bereich des Peptids besitzen. Alle Nukleinsäuren, welche die obengenannten Kriterien, insbesondere die Homologiekriterien, erfüllen, werden erfindungsgemäß somit als Syncollin-kodierende Nukleinsäuren bezeichnet.

Ferner eingeschlossen sind modifizierte Nukleinsäuren, insbesondere chemisch modifizierte Nukleinsäuren. Solche Nukleinsäuren können im Basen-Anteil, im Zucker-Anteil und/oder im Phosphat-Anteil modifiziert vorliegen, insbesondere um eine höhere In-vivo-Stabilität zu erzielen, wie zum Beispiel mittels Phosphorthioaten. Solche Nukleinsäuren werden bereits bei Ribozym-, Antisense- und Triplexnukleinsäure-Techniken eingesetzt.

Weiterhin betrifft die Erfindung Nukleinsäurekonstrukte, insbesondere Vektoren, welche die oben genannten, Syncollin-kodierenden Nukleinsäuren enthalten. Als Vektoren können insbesondere Plasmide, Cosmide, Phagenpartikel, künstliche Chromosomen oder virale Vektoren wie Retroviren, Adenoviren, adenoähnliche Viren oder Baculoviren eingesetzt werden. In einer bevorzugten Ausführungsform werden die erfindungsgemäßen Syncollin-kodierenden Nukleinsäuren mit weiteren natürlichen oder heterologen, meist regulatorischen Sequenzen verknüpft. Solche Sequenzen können Replikationsursprünge, Klonierstellen, Resistenz- und Markergene, Selektionsgene, Promotoren wie beispielsweise wie Polyoma-, Adenovirus 2-, Cytomegalovirus- oder Simian Virus 40-Promotoren, Verstärkungssequenzen (enhancer), Sequenzen für *E*. *coli*-ribosomale Bindungsstellen, Polyadenylierungssequenzen, Terminationssequenzen und andere sein. Solche regulatorischen Sequenzen können sowohl die globale Expression einer Nukleinsäure im Gesamtorganismus als auch eine zell- oder gewebespezifische (wie beispielsweise durch gewebespezifische Promotoren wie den leberspezifischen Albuminpromotor oder einen pankreas-spezifischen Promotor [32]) oder eine induzierbare Expression einer Nukleinsäure beeinflussen.

### Antisense-Nukleinsäuren

Weiterhin betrifft die Erfindung Nukleinsäuren, welche zu Syncollin-kodierenden Nukleinsäuren komplementär sind, sogenannte Antisense-Nukleinsäuren. Insbesondere umfasst die Erfindung zu SEQ ID 5 bis SEQ ID 12 komplementäre Antisense-Nukleinsäuren. Antisense-Nukleinsäuren sind bevorzugt komplementär zum kodierenden Strang einer cDNA oder einer mRNA. Die Antisense-Nukleinsäuren können sowohl zum gesamten kodierenden Bereich als auch nur zu Teilbereichen von diesem komplementär sein. Weiterhin können die Antisense-Nukleinsäuren auch zu nicht-kodierenden Bereichen, beispielsweise 5'- oder 3'- untranslatierten Bereichen, komplementär sein, insbesondere zu diesen Bereichen, die in SEQ ID 8 bis SEQ 8 vorliegen. Antisense-Nukleinsäuren können bevorzugt als sogenannte Oligonukleotide vorliegen, welche als kurzkettige Nukleinsäuren mit 7 bis mehr als 100 Nukleotiden Länge zu bestimmten Bereichen der Syncollin-kodierenden Nukleinsäuren komplementär sind und vorzugsweise als Primer, Hybridisierungsreagentien oder zur gezielten Mutagenese oder Expressionsregulation geeignet sind. Hierzu können die Antisense-Nukleinsäuren mit einer Markierung, vorzugsweise einer chemilumineszenden, fluoreszierenden, radioaktiven oder colormetrischen Markierung, versehen vorliegen. Weiterhin können Antisense-Nukleinsäuren chemisch modifiziert vorliegen, insbesondere als alpha-anomerische Nukleinsäuren [33], als chimärische RNA-DNA-Analoge [34] als 2'-o-Methylribonukleotide enthaltende Nukleinsäuren [35] oder als peptide nucleic acids (PNAs) [36]. Weiterhin umfassen Antisense-Nukleinsäuren Ribozyme [37].

### Vektoren und Wirtszellen

Weiterhin betrifft die Erfindung Vektoren und damit transfizierte Wirtszellen, welche Syncollin-kodierende Nukleinsäuren enthalten. Wirtszellen können sowohl prokaryontische als auch eukaryontische Zellen, insbesondere auch Stammzellen wie beispielsweise hämatopoetische Stammzellen, sein. Die Transfektion beziehungsweise Transformation erfolgt vorzugsweise mittels Kalziumphosphat- oder Kalziumchlorid-Kopräzipitation, Diethylaminoethyl-Dextran-(DEAE-Dextran-)vermittelter Transfektion, Lipofektion oder Elektroporation. Insbesondere sind Vektoren und transfizierte Wirtszellen eingeschlossen, welche zur Herstellung und Gewinnung von Syncollinpeptiden und Syncollin-kodierenden Nukleinsäuren geeignet sind. Insbesondere kann in diesen Zellen die Expression der Syncollin-kodierenden Nukleinsäuren moduliert sein, das heißt, es kann eine Überexpression oder Unterexpression der entsprechenden Nukleinsäuresequenzen vorliegen. Hierbei ist auch eine vollständige Nichtexpression der entsprechenden Nukleinsäuresequenzen eingeschlossen, wie sie beispielsweise bei Knockout-Tiermodellen vorliegt. Die Modulation muss nicht unbedingt durch die normalerweise in der Zelle vorliegenden Kontroll- und Regulationssequenzen erfolgen, sondern es können zu diesem Zweck auch heterologe, dass heißt, natürlicherweise nicht der Expressionsmodulation von Syncollin-kodierenden Nukleinsäuren dienende, Kontroll- und Regulationssequenzen eingesetzt werden. Die Vektoren können zur Expression der Nukleinsäuren sowohl in prokaryontischen Zellen wie *E. coli* und in eukaryontischen Zellen wie Insektenzellen, Hefezellen oder Säugerzellen als auch in in vitro-Systemen wie dem T7-Promotor/T7-Polymerase-System [38] eingesetzt werden. Die erfindungsgemäßen Syncollin-kodierenden Nukleinsäuren können sowohl in Sense- als auch in Antisense-Richtung im Vektor vorliegen, insbesondere im Zusammenhang mit einem Einsatz dieser Nukleinsäuren zur Regulation der Expression der Syncollin-kodierenden Nukleinsäuren.

### Verfahren zum Einsatz von Syncollinpeptiden, Syncollin-Antikörpern, Syncollin-kodierenden Nukleinsäuren, dazu korrespondierenden Antisense-Nukleinsäuren oder Agonisten oder Antagonisten dieser Stoffe

Die zuvor beschriebenen Syncollinpeptide, Syncollin-kodierenden Nukleinsäuren und Antisense-Nukleinsäuren, gegen Syncollinpeptide gerichteten Antikörper oder Agonisten beziehungsweise Antagonisten dieser Stoffe können zumindest in einem der folgenden Verfahren eingesetzt werden, insbesondere im Zusammenhang mit dem Auftreten von Stoffwechselerkrankungen:
- Prädiktive medizinische Verfahren (diagnostische Verfahren, prognostische Verfahren, Monitoring von Klinischen Studien, Pharmacogenomics/Pharmacoproteomics)
- Screening-Verfahren
- Therapeutische medizinische Verfahren (prophylaktische Verfahren, therapeutische Verfahren)

### Diagnostische und prädiktive Verfahren

Die vorliegende Erfindung betrifft insbesondere Verfahren der prädiktiven Medizin, wie diagnostische Verfahren, prognostische Verfahren und Begleitung und Kontrolle klinischer Studien. Generell werden hierbei diese erfindungsgemäßen Verfahren zur Bestimmung des Vorliegens einer Stoffwechselerkrankung, insbesondere des Metabolischen Syndroms, oder des Risikos, an einer solchen Stoffwechselerkrankung zu erkranken, eingesetzt. Dies gilt insbesondere für Stoffwechselerkrankungen, welche auf eine Fehlexpression der Syncollinpeptide oder Syncollin-kodierender Nukleinsäuren zurückzuführen sind. Diese Verfahren schließen zumindest eines der folgenden Verfahren ein:
- Verfahren zur Bestimmung des Vorliegens einer Mutation, welche die Expression der Syncollin-kodierenden Nukleinsäuren beeinflusst, oder einer Mutation, welche die Kontrolle oder Regulation der Genexpression beeinflusst (beispielsweise eine Mutation in der 5'-Kontollregion des Syncollingens)
- Verfahren zur Bestimmung des Vorliegens einer Mutation, welche die Struktur des jeweiligen Syncollingens oder Syncollinpeptids beeinflusst
- Verfahren zur Bestimmung des Vorliegens einer Fehlregulation der Expression der Syncollinpeptide auf mRNA-Ebene, beispielsweise durch Bestimmung einer veränderten Syncollin-mRNA-Konzentration im Vergleich zu gesunden Organismen
- Verfahren zur Bestimmung des Vorliegens einer Fehlregulation der Expression der Syncollinpeptide auf Proteinebene, beispielsweise durch Bestimmung einer veränderten Syncollinpeptid-Konzentration im Vergleich zu gesunden Organismen

Erstmalig wurde im Rahmen der vorliegenden Erfindung gefunden, dass in biologischen Proben, insbesondere Pankreasproben von ob/ob-Mäusen, die Konzentrationen bestimmter Syncollinpeptide relativ zu ihren Konzentrationen in Kontrollproben gesunder Mäuse stark erhöht ist. Veränderungen der Konzentration dieser Syncollinpeptide zeigen daher das Vorliegen von Stoffwechselerkrankungen spezifisch an und sind daher zur Diagnose und zum Nachweis von Stoffwechselerkrankungen, insbesondere des Metabolischen Syndroms, mit hoher Sensitivität und Spezifität geeignet. Die hervorragende Eignung der Syncollinpeptide als diagnostische Markerpeptide für das Vorliegen von Stoffwechselerkrankungen ist unter anderem in Beispiel 7 und Beispiel 8 dargelegt.

Hierzu sind verschiedene Lösungsansätze in der medizinischen Diagnostik möglich und üblich:

Zum einem kann generell das Vorhandensein eines Markerpeptides untersucht werden. Die Anwesenheit beziehungsweise Abwesenheit dieses Markerpeptids ermöglicht dann eine Diagnose der Erkrankung.

Bei einer anderen Diagnosestrategie werden zunächst die üblicherweise vorhandenen Konzentrationen des Markerpeptids bei Kontrollen und Organismen, welche an der zu diagnostizierenden Krankheit leiden, bestimmt und anhand dieser Konzentrationswerte ein Grenzwert ("Cut-off-Wert") ermittelt, welcher die Gruppe der als gesund betrachteten Organismen von der Gruppe der als erkrankt betrachteten Organismen trennt. Der für jedes Markerpeptid individuell ermittelte Cut-off-Wert ermöglicht eine Unterscheidung von gesunden und erkrankten Organismen. Solche Grenzwerte sind unter anderem Literaturwerte und

Referenzwerte für das jeweilige Markerpeptid. Ist die Konzentration des jeweiligen Markerpeptids bei Vorliegen der Erkrankung vermindert, so werden alle Organismen, deren Messwert für das jeweilige Markerpeptid unter dem Cut-off-Wert liegt, als erkrankt diagnostiziert. Ist die Konzentration des jeweiligen Markerpeptids bei Vorliegen der Erkrankung erhöht, so werden alle Organismen, deren Messwert für das jeweilige Markerpeptid über dem Cut-off-Wert liegt, als erkrankt diagnostiziert.

Eine weitere Diagnosestrategie ermittelt eine für das jeweilige Markerpeptid spezifische Konzentrationscrhöhung oder Konzentrationsverminderung in der untersuchten Probe, relativ zu der Konzentration des Markerpeptids in einer Kontrollprobe oder relativ zu Konzentrationen anderer Substanzen, sogenannter Referenzsubstanzen, in der untersuchten Probe. Solche Referenzsubstanzen können insbesondere andere Proteine und Peptide sein, aber auch Nukleinsäuren, Lipide, Metaboliten sowie andere organische und anorganische Substanzen, die in den vorliegenden Proben bestimmt werden können. Eine signifikante Konzentrationsänderung des Markerpeptids in der untersuchten Probe relativ zur Kontrollprobe oder zu den Referenzsubstanzen in der vorgenannten Weise wird als positives Nachweisergebnis für ein Vorliegen einer Erkrankung gewertet. Dies wird beispielsweise häufig bei sogenannten relativen Bestimmungen eingesetzt, bei welchen nicht die Absolutwerte der Messwerte als Diagnosekriterien genutzt werden, sondern die relativen Beziehungen der Messwerte zueinander. Analog gilt dies nicht nur für einzelne Markerpeptide, sondern auch für Kombinationen mehrerer Markerpeptide, sogenannte Peptidmuster.

Unter Kontrolle oder Kontrollprobe versteht man allgemein biologische Proben von Organismen, welche die betrachtete Erkrankung nicht aufweisen oder welche eine andere Erkrankung aufweisen, das heißt, die Kontrolle entspricht den Zustand einer Probe aus dem Kollektiv der "gesunden" Organismen. Die Abgrenzung gegenüber der Gruppe der "erkrankten" Organismen erfolgt mittels diagnostischer Verfahren und Marker, mit deren Hilfe die betrachteten Organismen den Kollektiven "gesund" und "erkrankt" zugeteilt werden. Insbesondere geschieht dies mit Hilfe diagnostischer Grenzwerte oder Cut-off-Werte, deren Unter- oder Überschreiten zu einer Einteilung in das jeweilige Kollektiv führt. Diese Grenzwerte können insbesondere Anwesenheit oder Abwesenheit eines diagnostischen Markers, Konzentration eines diagnostischen Markers in einer Probe, Konzentration eines diagnostischen Markers in einer Probe im Vergleich zu Konzentrationen anderer Stoffen in der gleichen Probe oder zur Konzentration des gleichen Stoffes in einer anderen Probe (relative Grenzwerte), Literaturwerte, Referenzwerte, Erfahrungswerte usw. sein. Im Sinne dieser Erfindung sind als Kontrollproben neben den Proben der Kontrollgruppe selbst auch alle oben genannten diagnostischen Grenzwerte oder Cut-off-Werte anzusehen.

### Markersubstanzen

Die Erfindung umfasst somit Verfahren zum Nachweis einer Stoffwechselerkrankung durch Bestimmung der Konzentration wenigstens eines Markerpeptids in einer biologischen Probe eines Organismus, verglichen mit der Konzentration des Markerpeptids in einer Kontrollprobe oder der Konzentration von Referenzsubstanzen, bei denen folgende Punkte erfüllt sein müssen: Als Markerpeptid wird wenigstens ein Syncollinpeptid verwendet. Es tritt eine für das jeweilige Markerpeptid spezifische Konzentrationserhöhung oder Konzentrationsverminderung des Markerpeptids in der Probe des Patienten relativ zu der Konzentration des Markerpeptids in der Kontrollprobe oder zu den Konzentrationen von Referenzsubstanzen auf. Eingeschlossen in diese spezifischen Konzentrationsänderungen sind auch das vollständige Fehlen oder das erstmalige Auftreten des jeweiligen Markerpeptids in einer Probe. Eine signifikante Konzentrationsänderung des Markerpeptids im Vergleich zur Kontrollprobe in der vorgenannten Weise wird als positives Nachweisergebnis für eine Stoffwechselerkrankung gewertet. Dabei kann für ein bestimmtes Syncollinpeptid grundsätzlich nur ein Anstieg der Peptidkonzentration bei Organismen, welche an Stoffwechselerkrankungen leiden, auftreten oder es kann für dieses Syncollinpeptid grundsätzlich nur eine Verminderung der Peptidkonzentration bei Organismen, welche an Stoffwechselerkrankungen leiden, auftreten. Wie bei nahezu allen medizinischen Diagnosen von Erkrankungen sind falsch-positive oder falsch-negative Ergebnisse grundsätzlich möglich, das heißt in wenigen Einzelfällen erfolgt eine falsche Diagnose, da sich die Konzentration der Syncollinpeptide bei Organismen, welche an Stoffwechselerkrankungen leiden, nicht mit hundcrtprozentiger Sicherheit von der Konzentration der Syncollinpeptide bei gesunden Organismen unterscheidet. Dieses Problem kann jedoch meist durch Mehrfachkontrollen behoben werden. Bei einer eventuell verwendeten Kontrollprobe kann es sich um eine Poolprobe aus verschiedenen Kontrollen handeln. Auch die zu untersuchende Probe kann eine Poolprobe sein, wobei bei positivem Diagnoseergebnis Einzeluntersuchungen angestellt werden.

Im Rahmen der Erfindung können verschiedene Methoden zum Nachweis der Syncollinpeptide verwendet werden. Dazu sind alle Methoden geeignet, die es ermöglichen, Syncollinpeptide spezifisch in einer Probe eines Organismus nachzuweisen. Geeignete Methoden sind unter anderem physikalische Methoden wie zum Beispiel Massenspektrometrie oder Flüssigkeits-Chromatographie, biologische Methoden wie zum Beispiel Bioassays oder Stoffwechseltests, molekularbiologische Methoden wie zum Beispiel Reverse-Transkriptase-Polymerase-Kettenreaktion (RT-PCR) oder immunologische Nachweistechniken, wie zum Beispiel "Enzyme Linked Immuno Sorbent Assays" (ELISA).

Eine Ausführungsform der Erfindung ist die Verwendung physikalischer Methoden, welche die erfindungsgemäßen Peptide qualitativ und/oder quantitativ anzeigen können. Zu diesen Methoden gehören unter anderem Massenspektrometrie, Flüssigkeitschromatographie, Dünnschichtchromatographie, weitere chromatographische Methoden, NMR- (Nuclear-Magnetic-Resonance-) Spektroskopie, elektrophoretische Methoden usw. Dabei werden quantitative Messergebnisse aus einer untersuchten Probe mit den Messwerten, die bei einem Kollektiv an einer Stoffwechselerkrankung leidender Organismen und einem Kontrollkollektiv gesunder Organismen gewonnen wurden, verglichen. Aus diesen Ergebnissen kann das Vorliegen und/oder der Schweregrad einer Stoffwechselerkrankung abgeleitet werden.

Eine weitere Ausführungsform dieser Erfindung ist die Durchführung von Fällungsreaktionen zur Fraktionierung der Probe unter Verwendung von Fällungsmitteln wie zum Beispiel Ammoniumsulfat, Polyethylenglykol, Trichloressigsäure, Aceton, Ethanol, Methanol usw. Die so gewonnenen einzelnen Fraktionen werden dann separat dem jeweiligen Nachweisverfahren, zum Beispiel einer massenspektrometrischen Untersuchung, unterzogen.

Eine weitere Ausführungsform der Erfindung ist die Verwendung von Flüssigphasenextraktion. Dazu wird die Probe zum Beispiel mit einem Gemisch aus einem organischen Lösungsmittel, wie etwa Polyethylenglykol (PEG), und einer wässrigen Salzlösung gemischt. Aufgrund ihrer physikochemischen Eigenschaften reichern sich dann bestimmte Inhaltsstoffe der Probe in der organischen und andere in der wässrigen Phase an und können dadurch weiter fraktioniert und anschließend analysiert werden.

Gemäß bevorzugter Ausführungsform dieser Erfindung werden die Peptide in der Probe vor der Identifizierung chromatographisch getrennt, und zwar vorzugsweise mit Reverse-Phase-Chromatographie, insbesondere mit hochauflösender Reverse-Phase High-Performance-Flüssigchromatographie (RP-HPLC). Eine besonders bevorzugte Ausführungsform dieser Erfindung umfasst die Verwendung einer C18 Reverse-Phase-Chromatographiesäule unter Verwendung von Laufmitteln aus Trifluoressigsäure und Acetonitril zur Trennung von Peptiden in humanen Liquor cerebrospinalis. Es werden beispielsweise Fraktionen gesammelt, die je 1/100 des verwendeten Laufmittel-Volumens beinhalten. Die so gewonnenen Fraktionen werden vorzugsweise gefriergetrocknet und anschließend für eine massenspektrometrische Analyse weiterbehandelt (siehe Beispiel 2).

Gemäß einer bevorzugten Ausführungsform der Erfindung wird die Identifizierung mindestens eines Syncollinpeptids mit Hilfe einer massenspektrometrischen Bestimmung, vorzugsweise MALDI- (Matrix-Assisted-Laser-Desorption-and-Ionisation-) Massenspektrometrie, durchgeführt. Zur Präparation der Proben werden sie zunächst mit einer Matrixsubstanz vermischt, die typischerweise eine organische Säure enthält. Typische Matrix-Substanzen, die sich für Peptide eignen, sind die 3,5-Dimethoxy-4-hydroxyzimtsäure, die Alpha-cyano-4-hydroxyzimtsäure und die 2,5-Dihydroxybenzoesäure. Vorzugsweise werden die Fraktionen in einer Matrixlösung, bestehend aus zum Beispiel aus L-Fucose und Alpha-cyano-4-hydroxyzimtsäure in einem Gemisch aus Acetonitril, Wasser, Trifluoressigsäure und Aceton, gelöst und nach Auftragen auf das MALDI-Target getrocknet. Anschließend wird mittels MALDI-Massenspektrometrie das Vorliegen bestimmter Massen festgestellt und die Signalintensität quantifiziert. Dabei umfasst die massenspektrometrische Bestimmung vorzugsweise eines der folgenden Massensignale, jeweils berechnet anhand der theoretischen, monoisotopischen Masse des entsprechenden Peptids: 12538,0 (SYN-1), 12552,0 (SYN-2), 12613,0 (SYN-3) oder 12350,9 (SYN-4). Dabei können bei der experimentellen Bestimmung leichte Abweichungen von der theoretischen, monoisotopischen Masse aufgrund experimenteller Unschärfen und der natürlichen Isotopenverteilung auftreten. Außerdem wird bei MALDI-Massenbestimmungen aufgrund der Messmethodik den Peptiden ein Proton oder auch mehrere Protonen hinzugefügt, wodurch sich die Masse um ca. 1 Da pro Proton erhöht. Die Berechnung der theoretischen, monoisotopischen Massen erfolgt mittels spezieller Software, hier GPMAW 5.02b (Lighthouse Data, Odense, Dänemark). Diese theoretischen, monoisotopischen Massen können einzeln oder in Kombinationen in einer Probe auftreten (siehe Beispiel 3).

In einer weiteren praktischen Ausführungsform ist eine weitere Absicherung des Nachweisergebnisses dadurch möglich und empfehlenswert, dass die Identität der den Massen entsprechenden Peptide ermittelt wird, wobei ausschließlich Peptidsignale berücksichtigt werden, die von einem Syncollinpeptid abgeleitet werden können. Diese Absicherung erfolgt über eine Identifizierung der Peptidsignale vorzugsweise mit massenspektrometrischen Verfahren, zum Beispiel einer MS/MS-Analyse zur Bestimmung der Aminosäuresequenz des jeweiligen Peptids (siehe Beispiel 5).

Ein weiteres Verfahren zur Identifikation der erfindungsgemäßen Peptide ist eine Sequenzanalyse mittels Edman-Abbau in einer dem Fachmann bekannten Weise [39] (siehe Beispiel 6).

Die Bestimmung eines Syncollinpeptids kann allgemein mit Hilfe eines gegen das Syncollinpeptid gerichteten Antikörpers oder Antikörperfragmentes erfolgen. Gemäß einer weiteren bevorzugten Ausführungsform der Erfindung kann die Identifizierung der Syncollinpeptide oder ihrer Vorläufer unter Verwendung eines immunologischen Nachweissystems, vorzugsweise eines ELISAs ("Enzyme Linked Immuno Sorbent Assay"), durchgeführt werden. Dabei erfasst dieser immunologische Nachweis mindestens ein Syncollinpeptid, dessen Fragmente oder dessen Vorläuferprotein. Zur Erhöhung der Spezifität kann weiterhin bevorzugt ein sogenannter "Sandwich-ELISA" verwendet werden, bei dem der Nachweis der Syncollinpeptide von der Spezifität von zwei Antikörpern, die unterschiedliche Epitope innerhalb des gleichen Moleküls erkennen, abhängig ist. Zum Nachweis von Syncollinpeptiden können jedoch auch andere ELISA-Systeme, zum Beispiel direkte oder kompetitive ELISAs, Verwendung finden. Auch weitere immunologische Nachweistechniken, wie zum Beispiel EMIT ("Enzyme Multiplied Immunoassay Technique") RIA ("Radio Immuno Assay"), EIA ("Enzym Immuno Assay"), ELI-Spot usw., sind als Nachweissysteme geeignet. Als Standard für die Quantifizierung können insbesondere aus biologischen Proben isolierte, rekombinant hergestellte oder chemisch synthetisierte Syncollinpeptide verwendet werden. Weitere für solche Nachweise geeignete Methoden sind unter anderem Westemblotting, Immunpräzipitation, Immunofluoreszenz, Dot-Blots, Plasmonresonanzspektrometrie (BIACORE®-Technologie, Biacore International AB, Uppsala, Schweden), Phagenpartikel, Affinitätsmatrizen (zum Beispiel ABICAP-Technologie, ABION Gesellschaft für Biowissenschaften und Technik mbH, Jülich, Deutschland) und andere. Generell sind alle Substanzen als Nachweisagenzien geeignet, welche spezifisch mit Syncollinpeptiden, deren Fragmenten oder Vorläuferproteinen interagieren, vorzugsweise an diese binden, und es dadurch erlauben, ein spezifisches Nachweissystem für diese Substanzen aufzubauen.

Weiterhin umfasst die Erfindung die Verwendung von Syncollin-kodierenden Nukleinsäuren, deren Fragmenten und dazu korrespondierenden Antisense-Nukleinsäuren zur Feststellung von Stoffwechselerkrankungen oder zur indirekten Bestimmung und Quantifizierung der zugehörigen Syncollinpeptide. Darin eingeschlossen sind auch Nukleinsäuren, welche nicht kodierende Sequenzen, wie etwa 5'- oder 3'-untranslatierte Bereiche der mRNA oder noch nicht prozessierte, primäre mRNA-Transkripte, darstellen und Nukleinsäuren, insbesondere Oligonukleotide, die eine für spezifische Hybridisierungsexperimente ausreichende Sequenzübereinstimmung mit Syncollin-kodierenden Nukleinsäuren aufweisen und die daher zum indirekten Nachweis der davon abgeleiteten Syncollinpeptide geeignet sind.

Weiterhin sind Verfahren eingeschlossen, mit deren Hilfe eine genetische Veränderung in einer Syncollin-kodierenden Nukleinsäure, insbesondere einer der Nukleotidsequenzen SEQ ID 5 bis SEQ ID 12, oder deren Expression festgestellt werden kann und welche mit dem Auftreten oder einem veränderten Risiko des Auftretens einer Stoffwechselerkrankung in Zusammenhang stehen. Insbesondere schließen genetische Veränderungen mindestens eine der folgende Veränderungen ein: Deletion einer oder mehrerer Nukleotide des jeweiligen Syncollingens, Addition einer oder mehrere Nukleotide des jeweiligen Syncollingens, Substitution einer oder mehrerer Nukleotide des jeweiligen Syncollingens, Umverteilung des genetischen Materials des jeweiligen Syncollingens (chromosomal rearragement), abweichende Modifizierungen des jeweiligen Syncollingens (beispielsweise des jeweiligen Methylierungsmusters), Verlust eines Alles des jeweiligen Syncollingens (allelic loss), Veränderung der Konzentration oder Aktivität des mRNA-Transkripts des jeweiligen Syncollingens, abweichendes Spleißen des mRNA-Transkripts des jeweiligen Syncollingens, Veränderung der Konzentration oder Aktivität des jeweiligen Syncollinpeptids, abweichende posttranslationale Modifizierungen des jeweiligen Syncollinpeptids.

Hierzu können insbesondere amplifizierende molekularbiologische Methoden wie Polymerase-Kettenreaktion (insbesondere Anchor-PCR und RACE-PCR), Ligase-Kettenreaktion, self sustained sequence amplification-Methoden, transkriptionale Amplifikationssysteme, Q-beta-Replikase-Systeme und weitere amplifizierende Methoden eingesetzt werden, bei welchen eine Bestimmung der Sequenz der Nukleotide der vervielfältigten Nukleinsäuremoleküle in einer dem Fachmann bekannten Weise folgt. Weiterhin können genetische Veränderungen durch Veränderungen der Muster eines Restriktionsenzymverdaus oder eines Ribozymverdaus, durch Hybridisierungsexperimente wie beispielsweise DNA- oder RNA-Chip-Arrays [40], durch Sequenzierung der Nukleotidsequenzen, durch mismatch cleavage-Verfahren [41], durch elektrophoretische Verfahren [42] oder selektive Oligonukleotidhybridisierungs-, Amplifizierungs- oder Primerverlängerungs-Verfahren in einer dem Fachmann bekannten Weise angewendet werden.

Zur Quantifizierung können Methoden wie zum Beispiel Reverse-Transkriptase―(RT-PCR), quantitative real-time-PCR, Ligase-Kettenreaktion, self sustained sequence amplification-Methoden, transkriptionale Amplifikationssysteme, Q-beta-Replikase-Systeme, rolling circle-Replikationssysteme, RNA- oder DNA-Chips, in situ-Hybridisierungen, Southernblots oder Northernblots in einer dem Fachmann bekannten Weise angewendet werden.

### Testkits

Weiterhin umfasst die Erfindung die Verwendung wenigstens eines Syncollinpeptids oder einer Syncollin-kodierenden Nukleinsäure zur Herstellung von Nachweissystemen zur Feststellung einer Stoffwechselerkrankung, insbesondere des Metabolischen Syndroms sowie insbesondere zur Herstellung von syncollinspezifischen Antikörpern oder Antisense-Nukleinsäuren. Die Erfindung umfasst auch die Verwendung von Syncollinpeptiden zur Gewinnung von Phagenpartikeln, die spezifisch diese Peptide binden, oder die umgekehrt Syncollinpeptide auf ihre Oberfläche präsentieren und so die Identifizierung von Bindungspartnern wie beispielsweise Rezeptoren ermöglichen.

Weiterhin sind auf Syncollinpeptiden oder Syncollin-kodierenden Nukleinsäuren basierende Nachweissysteme und Testkits eingeschlossen. Kits zur Bestimmung der Syncollinpeptide enthalten bevorzugt einen ersten Antikörper, welcher bevorzugt immobilisiert vorliegt und an ein erfindungsgemäßes Syncollinpeptid bindet und optional einen zweiten Antikörper, welcher an das Syncollin oder den ersten Antikörper bindet und mit einem Nachweisagenz gekoppelt ist. Kits zur Bestimmung der Syncollin-kodierenden Nukleinsäuren enthalten bevorzugt Oligonukleotide, welche mit einer Syncollin-kodierenden Nukleinsäure hybridisieren können und mit einem Nachweisagenz gekoppelt sind oder Nukleotidsequenzen, die als Primer einer Syncollin-kodierenden Nukleinsäuresequenz eingesetzt werden können. Die Testkits können weitere Reagenzien wie Pufferlösungen, Konservierungs- und Stabilisierungssubstanzen, Nachweisreagenzien wie Enzyme oder Substrate oder Kontroll- und Referenzproben enthalten.

Die vorgenannten Verfahren und der Einsatz der vorgenannten Substanzen können zur Identifizierung von Organismen eingesetzt werden, welche an Stoffwechselerkrankungen, insbesondere Stoffwechselerkrankungen, welche durch eine veränderte oder fehlregulierte Syncollinpeptidexpression oder ―aktivität gekennzeichnet sind, leiden oder ein verändertes Risiko besitzen, solche Erkrankungen zu entwickeln.

Insbesondere können die dargestellten diagnostischen Methoden und die ihnen zugrundeliegenden Substanzen für prädiktive Anwendungen eingesetzt werden, inbesondere zur Feststellung von Organismen, bei welchen Agenzien, wie beispielsweise Agonisten, Antagonisten, Proteine, Peptide, Protein- und Peptidmimetika, Nukleinsäuren, small molecule drugs oder andere biologische oder pharmakologische Wirkstoffe, zur Behandlung von Stoffwechselerkrankungen eingesetzt werden können. Dies ist vor allem in dem Bereichen Pharmacogenomics und Pharmacoproteomics sowie bei der Durchführung klinischer Studien von großer Bedeutung. Unter Pharmacogenomics beziehungsweise Pharmacoproteomics versteht man hierbei die Anwendung genomischer Methoden, wie beispielsweise Gensequenzierung, statistischer Genetik oder Genexpressionsanalysen, beziehungsweise proteomischer Methoden wie Proteinchips oder Protein- oder Peptid-Expressionsmusteranalysen, zur Feststellung oder Vorhersage der Reaktion eines bestimmten Patienten auf eine bestimmte Medikation oder Therapieform. Dies geschieht insbesondere im Rahmen klinischer Studien oder auch zur Überprüfung der Eignung bestehender Medikamente und Therapien, vorzugsweise durch Feststellung des Genotyps beziehungsweise Phänotyps eines Patienten. Insbesondere können mit den dargestellten Methoden Organismen bestimmt werden, welche auf eine Behandlung mit den oben genannten Stoffen wahrscheinlich positiv, insbesondere mit einer Besserung der Erkrankung, reagieren oder Organismen, welche auf eine Behandlung mit den oben genannten Stoffen wahrscheinlich negativ, insbesondere mit keiner Besserung beziehungsweise einer Verschlechterung der Erkrankung oder dem Auftreten von Nebenwirkungen, reagieren. Dadurch ist es möglich, eine möglichst effiziente und nebenwirkungsfreie Therapie beziehungsweise Prophylaxe gezielt für bestimmte Organismen zu entwickeln, die auf die oben genannten Stoffe positiv reagieren und sogenannte Responder darstellen, während für Organismen, die auf die oben genannten Stoffe negativ reagieren und sogenannte Non-Responder darstellen, andere Therapie- und Prophylaxeverfahren eingesetzt werden müssen. Der erfindungsgemäße Einsatz von Syncollinpeptiden und Syncollin-kodierenden Nukleinsäuren ermöglicht es aufgrund seiner hohen diagnostischen Spezifität und Sensitivität weiterhin, im Rahmen von klinischen Studien zur Entwicklung neuer Therapien zur Behandlung von Stoffwechselerkrankungen mit hoher Zuverlässigkeit nur solche Patienten auszuwählen, welche auch wirklich an Stoffwechselerkrankungcn leiden. Insbesondere ermöglichen diese neuartigen Verfahren die Gewinnung aussagekräftiger Studienergebnisse, da zum Beispiel fälschlicherweise als an einer Stoffwechselerkrankung leidend diagnostizierte Patienten die Qualität der Ergebnisse einer solchen klinischen Studie negativ beeinflussen würden. Außerdem ermöglicht der erfindungsgemäße Einsatz von Syncollinpeptiden und Syncollin-kodierenden Nukleinsäuren die Stratifizierung von Patienten oder Probanden, wodurch Subgruppen von Personen mit Stoffwechselerkrankungen gezielt ausgewählt werden können, die für bestimmte Therapiestrategien oder klinische Studien besonders geeignet sind. Unter Stratifizierung versteht man hierbei die Zuordnung eines Patienten oder Probanden zur einer Risikogruppe nach im Studienprotokoll festgelegten Kriterien, beispielsweise im Rahmen einer klinischen Studie.

Weiterhin können Syncollinpeptide und Syncollin-kodierende Nukleinsäuren als Marker für Erkrankungen, insbesondere Stoffwechselerkrankungen, als Marker für eine Prädisposition für eine solche Erkrankung, als Marker für die Wirksamkeit einer Substanz oder als Marker für das pharmakogenomische oder pharmakoproteomische Profil eines Organismus eingesetzt werden. Insbesondere können Syncollinpeptide und Syncollin-kodierende Nukleinsäuren als Surrogatmarker eingesetzt werden, vorzugsweise bei der Durchführung klinischer Studien. Unter Surrogatmarker versteht man allgemein einen objektiven biochemischen Marker, der mit dem Auftreten oder Fortschreiten einer Krankheit korreliert und bereits Vorhersagen über die Wirksamkeit einer Therapie ermöglicht, ohne dass der klinische Endpunkt der Erkrankung erreicht ist. So kann es beispielsweise möglich sein, die Wirksamkeit einer Therapie einer Stoffwechselerkrankung bereits frühzeitig anhand einer Veränderung der Konzentration der Syncollinpeptide oder Syncollin-kodierenden Nukleinsäuren in einer biologischen Probe zu erfassen, ohne die vollständige Heilung des Patienten abwarten zu müssen. Weiterhin können Syncollinpeptide und Syncollin-kodierende Nukleinsäuren als pharmakodynamische Marker eingesetzt werden. Unter pharmakodynamischen Marker versteht man allgemein einen objektiven biochemischen Marker, der spezifisch mit dem Wirkungen eines biologischen oder pharmakologischen Wirkstoffes korreliert, wobei keine Korrelation mit dem Vorliegen einer Erkrankung vorliegen muss, zu deren Therapie der Wirkstoff eingesetzt wird. Die Anwesenheit oder Aktivität eines solchen Markers wird vor allem zur Bestimmung der Anwesenheit oder Aktivität eines Wirkstoffes eingesetzt. Solche Marker werden insbesondere zur Erhöhung der Sensitivität des Nachweises von Effekten eines Wirkstoffes eingesetzt, vor allem wenn der Wirkstoff in sehr geringen Dosen eingesetzt wird. So können beispielsweise die Konzentrationsänderungen von Syncollinpeptiden oder Syncollin-kodierenden mRNAs als pharmakodynamische Marker für Wirkstoffe eingesetzt werden, welche die Transkription oder Expression der Syncollin-kodierenden Nukleinsäuren beeinflussen, insbesondere dann, wenn die Effekte des Wirkstoffes durch multiple Transkriptions- oder Expressionsrunden verstärkt werden. Weiterhin können Syncollinpeptide und Syncollin-kodierende Nukleinsäuren als pharmakogenomische oder pharmakoproteomische Marker eingesetzt werden. Unter einem pharmakogenomischen oder pharmakoproteomischen Marker versteht man allgemein einen objektiven biochemischen Marker, welcher mit einer spezifischen Reaktion oder Empfindlichkeit eines Organismus auf einen bestimmten biologischen oder pharmakologischen Wirkstoff oder eine bestimmtes therapeutisches oder prophylaktisches Verfahren korreliert. Solche Marker werden insbesondere im Vorfeld einer Therapie eingesetzt, um die Auswahl einer auf den jeweiligen Zustand eines Organismus optimierten, effizienten und nebenwirkungsfreien Therapieform zu ermöglichen. So kann beispielsweise der Nachweis bestimmter Mutationen in den Sequenzen von Syncollin-kodierenden Nukleinsäuren dazu genutzt werden, spezielle therapeutische Verfahren, beispielsweise die Applikation von Syncollinpeptiden oder Antisense-Nukleinsäuren, auszuwählen, welche spezifisch auf die durch diese Mutationen hervorgerufenen Wirkungen abzielen.

### Geeignete biologische Proben

Die Bezeichnung "biologische Probe" oder "Probe" umfasst intakte oder homogenisierte Organe, Gewebe, Zellen und biologische Flüssigkeiten, wie beispielsweise Serum, Plasma, Vollblut, Chymus, Urin, Stuhl, Nasensekret, Tränenflüssigkeit, Sputum, Liquor cerebrospinalis oder Synovialflüssigkeit, eines Organismus. Erfindungsgemäß wird als biologische Probe vorzugsweise Pankreasextrakt eingesetzt, aber es können auch weitere biologische Proben wie Chymus, Urin, Homogenate und Extrakte des Gastrointestinaltraktes, Serum oder Plasma geeignet sein. Dies hängt unter anderem von der Empfindlichkeit des gewählten Nachweisverfahrens (Massenspektrometrie, ELISA oder andere) ab. Insbesondere Serum, Plasma und Urin sind von medizinischem Interesse, da dieses Probenmaterial bei Standarduntersuchungen häufig und ohne großen Aufwand gewonnen wird.

Die erfindungsgemäß beschriebenen Syncollinpeptide, Syncollin-kodierenden Nukleinsäuren, gegen Syncollinpeptide gerichtete Antikörper und Antisense-Nukleinsäuren sowie Agonisten und Antagonisten beziehungsweise erfindungsgemäße Verfahren zum Einsatz dieser Stoffe beziehen sich vorzugsweise auf Syncollinpeptide, Syncollin-kodierenden Nukleinsäuren, gegen Syncollinpeptide gerichtete Antikörper und Antisense-Nukleinsäuren sowie Agonisten und Antagonisten humaner Herkunft beziehungsweise den diagnostischen, therapeutischen und prophylaktischen Einsatz dieser Stoffe am Menschen, können aber auch allgemein auf Wirbeltiere, bevorzugt Säugetiere, angewendet werden.

### Screening-Verfahren

Die vorliegende Erfindung betrifft weiterhin Verfahren zur Identifizierung von Modulatoren, welche an Syncollinpeptide binden oder einen stimulierenden oder inhibitorischen Effekt auf die Expression und/oder Aktivität von Syncollinpeptiden oder Syncollin-kodierenden Nukleinsäuren zeigen. Solche Verfahren werden als Screening-Verfahren bezeichnet. Als Modulatoren gelten insbesondere Agonisten und Antagonisten der Syncollinpeptide und Syncollin-kodierenden Nukleinsäuren. Modulatoren können insbesondere Proteine, Peptide, Peptidomimetika, Peptoide, Nukleinsäuren, small molecule-drugs oder andere biologische oder pharmazeutische Wirkstoffe sein. Unter Peptidomimetika versteht man Moleküle, die selbst keine Peptidstruktur haben und deshalb oft stabiler sind, aber wie Peptide wirken. Ein Beispiel hierfür ist das Alkaloid Morphin, welches als Peptidomimetikum das körpereigene Endorphin nachahmt. Unter Peptoiden versteht man peptidähnliche Oligomere mit peptidartig verknüpften N-substituierten Glycinresten. Derartig identifizierte Substanzen werden nachfolgend vorzugsweise zur Modulation der Expression oder Aktivität eines Syncollinpeptids oder einer Syncollin-kodierenden Nukleinsäure, insbesondere in Zusammenhang mit therapeutischen Methoden, oder zur Aufklärung der biologischen Funktion der Syncollinpeptide eingesetzt.

Potentielle Modulatoren können vorzugsweise aus Stoffbanken (libraries) wie biologischen Stoffbanken, insbesondere Peptid- und Proteinbanken, durch kombinatorische Verfahren hergestellten Stoffbanken oder synthetisch hergestellten Stoffbanken stammen. Solche Stoffbanken können in gelöster Form oder an Oberflächen, insbesondere Beads, Chips, Bakterien, Sporen, Plasmiden oder Phagen, fixiert vorliegen.

Screening-Verfahren können insbesondere auf Syncollinpeptid-exprimierenden Zellen basieren, welche mit den zu testenden Substanzen in Kontakt gebracht werden und die Fähigkeit der Testsubstanz, die Expression oder Aktivität der Syncollinpeptide zu veränderten, festgestellt wird. Weiterhin können hierzu auch Zellfraktionen und Zellhomogenate eingesetzt werden. Weiterhin können Screening-Verfahren eingesetzt werden, welche die Fähigkeit einer Testsubstanz überprüfen, an Syncollinpeptide zu binden oder die Bindung von Syncollinpeptiden an andere Substanzen, beispielsweise Rezeptoren, zu modulieren. In einer bevorzugten Ausführungsform können hierbei die Syncollinpeptide, die Testsubstanzen oder andere, mit Syncollinpeptiden wechselwirkenden Substanzen mit einer Markierung versehen vorliegen, welche dann zur Detektion herangezogen werden können. Solche Markierungen sind insbesondere Enzyme, Coenzyme beziehungsweise prosthetische Gruppen, Fluoreszenzmarker, Luminenszenzmarker oder radioaktive Markersubstanzen oder Kontrastmittel.

Screening-Verfahren können weiterhin auf zellfreien Systemen basieren, bei welchen die zu testenden Substanzen mit Syncollinpeptiden oder deren Fragmenten in Kontakt gebracht werden und die Fähigkeit der Testsubstanz, an die Syncollinpeptide oder deren Fragmente zu binden, festgestellt wird. Solche Screening-Verfahren beruhen auf einer spezifischen Interaktion der zu testenden Substanz und des Syncollinpeptids, welche beispielsweise mittels Fluoreszenzenergietransfer (FET)-Methoden oder real-time Biomolecular Interaction Analysis (BIA)-Methoden nachgewiesen werden können. Die Syncollinpeptide, die Testsubstanzen oder andere, mit Syncollinpeptiden wechselwirkende Substanzen können hierbei sowohl frei in einer Lösung oder an Oberflächen gebunden vorliegen oder mit Markierungen versehen vorliegen, welche dann zur Detektion herangezogen werden können.

Screening-Verfahren betreffen weiterhin Verfahren, mit deren Hilfe die Fähigkeit von Testsubstanzen bestimmt werden kann, die Bindung von Syncollinpeptiden an andere Substanzen, sogenannte Bindepartner, zu modulieren. Solche Bindepartner können insbesondere zelluläre und extrazelluläre Proteine wie beispielsweise Rezeptoren, sein. Derartige Verfahren können beispielsweise auf Two-Hybrid- oder Three-Hybrid-Systemen zur Bestimmung der Interaktion zwischen Bindepartnern beruhen [43].

Screening-Verfahren betreffen weiterhin Verfahren, mit deren Hilfe die Fähigkeit von Testsubstanzen bestimmt werden kann, die Expression von Syncollinpeptiden zu modulieren. Insbesondere können mit solchen Verfahren Stimulatoren oder Inhibitoren der Syncollin-mRNA-Expression oder der Syncollinpeptid-Expression ermittelt werden, beispielsweise durch Bestimmung der mRNA- oder Proteinkonzentrationen in Anwesenheit beziehungsweise Abwesenheit der zu testenden Substanzen.

Insbesondere schließt die Erfindung Substanzen ein, welche durch die oben beschriebenen Methoden identifiziert wurden und als Modulatoren, insbesondere Agonisten beziehungsweise Stimulatoren oder Antagonisten beziehungsweise Inhibitoren, der Aktivität oder Expression von Syncollinpeptiden oder Syncollin-kodierenden Nukleinsäuren herangezogen werden können. Solche erfindungsgemäßen Modulatoren können insbesondere Syncollinpeptid-modulierende Substanzen, Antisense-Nukleinsäuren zu Syncollin-kodierenden Nukleinsäuren oder deren Fragmenten, gegen Syncollinpeptide oder deren Fragmente gerichtete Antikörper oder an Syncollinpeptid bindende Substanzen, beispielsweise Rezeptoren, sein. Solche Modulatoren können zur Bestimmung ihrer Wirksamkeit, Toxizität, Nebenwirkungen oder Wirkmechanismen insbesondere in Tiermodellen untersucht werden.

Weiterhin können die mit den oben beschriebenen Methoden identifizierten Modulatoren (Agonisten und Antagonisten) der Aktivität oder Expression von Syncollinpeptiden oder Syncollin-kodierenden Nukleinsäuren zur therapeutischen und prophylaktischen Behandlung von Stoffwechselerkrankungen, insbesondere dem Metabolischen Syndrom, eingesetzt werden. Auch können sie zur Behandlung von Erkrankungen, welche durch eine veränderte Aktivität oder Expression von Syncollinpeptiden oder Syncollin-kodierenden Nukleinsäuren gekennzeichnet sind, eingesetzt werden.

### Therapeutische Verfahren

Die vorliegende Erfindung betrifft insbesondere Verfahren der therapeutischen Medizin, wie therapeutische Verfahren oder prophylaktische Verfahren. Generell werden hierbei diese erfindungsgemäßen Verfahren zur Behandlung von Organismen eingesetzt, welche an einer Stoffwechselerkrankung, insbesondere dem Metabolischen Syndrom, leiden oder ein verändertes Risiko, an einer solchen Stoffwechselerkrankung zu erkranken, besitzen. Dies gilt insbesondere für Stoffwechselerkrankungen, welche durch eine veränderte Aktivität oder Expression von Syncollinpeptiden oder Syncollin-kodierenden Nukleinsäuren gekennzeichnet sind.

Solche Verfahren können beispielsweise auf einer Modulation der Synthese und/oder Sekretion von Enzymen oder Hormonen (insbesondere Verdauungsenzymen wie Trypsin, Lipasen, Colipasen.... und Hormonen wie Insulin, Glucagon, Leptin...), des Kohlenhydratstoffwechsels (insbesondere Glykolyse, Gluconeogenese) oder des Lipidstoffwechsels (insbesondere Lipogenese, Lipolyse) basieren. Insbesondere können auf Syncollinpeptiden oder Syncollin-kodierenden Nukleinsäuren oder deren Modulatoren basierende therapeutische und prophylaktische Anwendungen auf einem der folgenden Wirkmechanismen beruhen: Regulation der Synthese oder Sekretion von Verdauungsenzymen aus dem Pankreas, Regulation der Synthese oder Sekretion von Hormonen oder Transmittern aus dem Pankreas, Regulation der Sekretion oder Synthese von Verdauungsenzymen, Hormonen oder Transmittern aus anderen Organen des Gastrointestinaltraktes, Beeinflussung neurogener und Hormon- oder Transmittervermittelter Regulationen des Stoffwechsels, insbesondere auch des Nahrungsaufnahmeverhaltens und der Nahrungsverwertung und andere.

Insbesondere betrifft die Erfindung Verfahren zur Prävention einer Stoffwechselerkrankung, insbesondere des Metabolischen Syndroms, oder von Erkrankungen, welche durch eine veränderte Expression oder Aktivität von Syncollinpeptiden oder Syncollin-kodierenden Nukleinsäuren gekennzeichnet sind, dadurch gekennzeichnet, dass hierzu einem Organismus Syncollinpeptide, Syncollin-kodierende Nukleinsäuren, Modulatoren dieser Stoffe, wie Antikörper, Antisense-Nukleinsäuren, Agonisten oder Antagonisten, oder Stoffe, welche eine syncollinanaloge Aktivität besitzen, verabreicht werden. Die Verabreichung kann hierbei prophylaktisch schon vor dem Auftreten der ersten krankheitsspezifischen Symptome erfolgen, so dass die Erkrankung sich gar nicht manifestiert oder in ihrem Fortschreiten verzögert wird. Organismen, welche ein erhöhtes Risiko einer solchen Erkrankung besitzen, können insbesondere durch die zuvor beschriebenen diagnostischen und prognostischen Verfahren ermittelt werden. Geeignete Substanzen zur Prävention können insbesondere durch die zuvor beschriebenen Screening-Verfahren aufgefunden werden.

Falls Stoffwechselerkrankungen zumindest teilweise mit einer erhöhten Expression oder Aktivität von Peptiden oder Proteinen, welche sich von Syncollin-kodierenden Nukleinsäuren ableiten, zusammenhängen, können zur Behandlung dieser Stoffwechselerkrankungen insbesondere Verfahren angewendet werden, welche den Konzentration oder die Aktivität dieser Stoffe verringern. Dies ist beispielsweise für SYN-1 in ob/ob-Mäusen der Fall. Insbesondere können zur Verringerung der Aktivität der Syncollinpeptide kleine organische oder anorganische Moleküle oder Antikörper, insbesondere polyklonale, monoklonale, humanisierte, antiidiotypische, chimärische oder single chain-Antikörper oder Fragmente dieser Antikörper, wie

FAb-, F(ab')2- und FAb-Expressionsbanken-Fragmente, scFV-Moleküle oder Epitop-bindende Fragmente, eingesetzt werden. Weiterhin können zur Verringerung der Expression der Syncollinpeptide insbesondere Antisense-Nukleinsäuren, Triple Helix-bildende Moleküle oder Ribozyme eingesetzt werden, wodurch dann ebenfalls die Aktivität der Syncollinpeptide reduziert wird. Weiterhin können vorzugsweise Syncollin-kodierende Nukleinsäuren, insbesondere mittels gentherapeutischer Methoden, zur Erhöhung der Expression und Aktivität der Syncollinpeptide therapeutisch eingesetzt werden. Weiterhin können Nukleinsäuren therapeutisch oder prophylaktisch in Form von Aptameren eingesetzt werden. Unter Aptameren versteht man Nukleinsäuremoleküle, welche eine dreidimensionale Struktur besitzen, die es ihnen ermöglicht, spezifisch an Proteinliganden, insbesondere an Syncollinpeptide zu binden.

Aptamere können bevorzugt zur Reduktion der Aktivität der Syncollinpeptide eingesetzt werden, da sie oft effizienter in Zellen eindringen können als peptidische Antagonisten [44].

Weiterhin umfasst die Erfindung Verfahren zur Modulation der Expression oder Aktivität von Syncollinpeptiden zur therapeutischen Zwecken. Hierzu können beispielsweise Zellen oder Zellverbände mit Syncollinpeptiden oder Modulatoren der Aktivität von Syncollinpeptiden, wie Proteine, Peptide, Nukleinsäuren (beispielsweise Aptamere), natürlicherweise mit Syncollinpeptiden wechselwirkende Moleküle (beispielsweise natürlich vorkommende Rezeptoren oder Substrate), Syncollin-Antikörper, Syncollinpeptid-Agonisten oder - Antagonisten, Peptidomimetika oder small molecule-Wirkstoffe. In einer bevorzugten Ausführungsform stimuliert die Substanz die Aktivität der Syncollinpeptide. Solche Stimulatoren können aktive Syncollinpeptide oder Syncollin-kodierende Nukleinsäuren sein. In einer weiteren bevorzugten Ausführungsform inhibiert die Substanz die Aktivität der Syncollinpeptide. Solche Inhibitoren können Antisense-Syncollin-Nukleinsäuren, Syncollin-Antikörper oder Syncollin-Inhibitoren sein. Eine Stimulation der Aktivität von Syncollinpeptiden wird vorzugsweise bei Erkrankungen eines Organismus eingesetzt, bei welchen diese Aktivität herunterreguliert ist oder bei welchen eine Steigerung dieser Aktivität vorteilhafte Effekte hervorruft. Analog hierzu wird eine Inhibierung der Aktivität von Syncollinpeptiden vorzugsweise bei Erkrankungen eines Organismus eingesetzt, bei welchen diese Aktivität hochreguliert ist oder bei welchen eine Senkung dieser Aktivität vorteilhafte Effekte hervorruft.

Weiterhin betrifft die Erfindung Verfahren zur Überprüfung der therapeutischen Wirksamkeit von Syncollinpeptiden, Syncollin-kodierenden Nukleinsäuren oder Modulatoren dieser Stoffe. Ein Ausführungsbeispiel hierfür umfasst die Kultivierung von Zelllinien und ihre Behandlung mit Syncollinpeptiden oder deren biologisch aktiver Fragmente oder mit Substanzen, welche die Expression oder Aktivität dieser Stoffe modulieren. Dadurch können die biologischen und pharmakologischen Eigenschaften von Syncollinpeptiden bestimmt werden. Auch Fusionsproteine und Fusionspeptide können zur Behandlung der Zelllinien verwendet werden, zum Beispiel Fusionsproteine mit Peptidsequenzen wie HIV-TAT-Sequenzen oder Antennapedia-Sequenzen, die einen Transport des Fusionsproteins ins Zellinnere fördern. Ebenso können Zelllinien mit Expressionsvektoren transfiziert werden, welche Syncollin-kodierende Nukleinsäuren enthalten. Alternativ können geeignete Zelllinien mit Antikörpern gegen Syncollinpeptide oder mit Nukleinsäuren, welche gegen Syncollin-kodierende Nukleinsäuren gerichtet sind, wie zum Beispiel Antisense-Nukleinsäuren, Triplex-Nukleinsäuren oder Ribozyme, behandelt werden. Insbesondere Zelllinien oder Zellsysteme, welche aus mehreren Zelllinien bestehen und welche als Modellsysteme im Zusammenhang mit einer Stoffwechselerkrankung, insbesondere des Metabolischen Syndroms, geeignet sind, können zu solchen Untersuchungen herangezogen werden. Als read-out Systeme für diese Untersuchungen können unter anderem Tests verwendet werden, welche die Proliferationsrate der behandelten Zellen, ihre Stoffwechselaktivität, die Apoptoserate der Zellen, Änderungen der Zellmorphologie, der Expression von zelleigenen Proteinen oder Reportergenen oder die Freisetzung von zytosolischen Zellbestandteilen ins Medium ermitteln. Weiterhin können auch geeignete Gewebe- und Organsysteme als Modellsysteme eingesetzt werden. Als weitere Testsysteme können geeignete Stämme von Versuchtieren, zum Beispiel von Mäusen oder Ratten, die als Modell für Stoffwechselerkrankungen dienen, verwendet werden. Diese Tiermodelle können insbesondere zur Untersuchung der Wirksamkeit von Therapiestrategien bei Stoffwechselerkrankungen herangezogen werden. Read-out Parameter bei Versuchen mit Tiermodellen können unter anderem die Überlebensdauer der Tiere, ihr Verhalten oder ihr Allgemeinzustand sein. Als weitere Parameter kann die Bestimmung von Körperfunktionen, wie zum Beispiel Körpertemperatur, Blutparameter, Stoffwechseltests, die Konzentration von Syncollinpeptiden und anderen, mit der Erkrankung im Zusammenhang stehenden Proteinen und Peptiden sowie morphologische und histologische Untersuchungen an Geweben herangezogen werden.

Diese therapeutischen und prophylaktischen Verfahren können, insbesondere in Hinblick auf aus pharmakogenomischen und pharmakoproteomischen Verfahren gewonnenen Erkenntnisse, spezifisch angepasst oder modifiziert werden. Insbesondere können diese therapeutischen und prophylaktischen Verfahren im Rahmen einen individuellen Therapie oder Prophylaxe derartig modifiziert werden, dass hierzu Syncollinpeptide, Syncollin-kodierende Nukleinsäuren oder Modulatoren dieser Stoffe, wie Antikörper, Antisense-Nukleinsäuren, Agonisten oder Antagonisten, in einer auf den jeweiligen pharmakogenomischen Genotyp oder pharmalcoproteomischen Phänotyp angepassten Weise verabreicht werden, so dass die Wirkungen der Verfahren möglichst optimiert beziehungsweise unerwünschte, insbesondere toxische, Nebenwirkungen vermieden werden. So können individuelle Unterschiede im Metabolismus derartiger therapeutischer Substanzen zu schwerwiegenden Toxizitätserscheinungen oder therapeutischen Misserfolgen führen, beispielsweise durch eine individuelle Änderung der realen vorliegenden Blutkonzentration einer solchen Wirksubstanz im Vergleich zur verabreichten Dosis. Pharmakogenomische oder -proteomische Studien und Untersuchungen ermöglichen in diesem Zusammenhang eine individuelle Entscheidung, ob Syncollinpeptide, Syncollin-kodierende Nukleinsäuren oder Modulatoren dieser Stoffe zur Behandlung eines individuellen Organismus prinzipiell geeignet sind oder ermöglichen es, die Dosierung und Verabreichung einer solchen Substanz an einen individuellen Organismus anzupassen. Individuelle phannakogenomische oder ―proteomische Unterschiede können insbesondere durch einzelne seltene Veränderungen wie beispielsweise Mutationen oder durch natürlicherweise vorkommende Polymorphismen hervorgerufen sein. Insbesondere können solche genetisch bedingten Unterschiede durch hochaufgelöste Kartierung der Gene eines Individuums, beispielsweise im Rahmen einer "genome-wide association"-Analyse oder einer Einzelnukleotidpolymorphismus (single nucleotide polymorphism, SNP)-Analyse, aufgefunden werden [45]. Weiterhin können Verfahren wie "candidate gene approach", "gene expression profiling" oder pharmakoproteomische Studien eingesetzt werden, um Gene oder deren Expressionsprodukte aufzufinden, welche dazu beitragen können, die Reaktion eines Organismus auf einen Wirkstoff hervorzusagen [46]. Insbesondere können solche Verfahren in klinischen Studien eingesetzt werden, beispielsweise zur Stratifizierung von Patienten.

### Pharmazeutische Zusammensetzungen

Die erfindungsgemäß beschriebenen Syncollinpeptide, Syncollin-kodierenden Nukleinsäuren, gegen Syncollinpeptide gerichtete Antikörper und Antisense-Nukleinsäuren sowie Agonisten und Antagonisten können Bestandteile pharmazeutischer Zusammensetzungen sein. Solche Zusammensetzungen enthalten mindestens einen der oben genannte Stoffe und mindestens eine pharmazeutisch verträgliche Trägersubstanz. Als solche Substanzen können insbesondere Lösungsmittel, Verdünnungsmittel, Puffersubstanzen, Dispersionsmedien, Füll- und Bindestoffe, Beschichtungssubstanzen, Gleitmittel, Treibmittel, Antioxidantien, Detergentien, Chelatoren, antibakterielle und antifungale Substanzen, isotonische und elektrolytische Zusätze, oberflächenaktive Substanzen oder Süß-, Geschmacks-, Geruchs- und Aromastoffe eingesetzt werden. Weiterhin können Substanzen eingesetzt werden, welche den Transport, die Lokalisation und die Freisetzung der Wirkstoffe beeinflussen können, wie (abbaubare) Polymere, Proteine, Lipide und Liposomen, drug carrier-Substanzen, drug-targeting-Substanzen und Mikrokapseln. Die erfindungsgemäß beschriebenen Syncollinpeptide, Syncollin-kodierenden Nukleinsäuren, gegen Syncollinpeptide gerichtete Antikörper und Antisense-Nukleinsäuren sowie Agonisten und Antagonisten können in verschiedensten, den jeweiligen Erfordernissen angepassten, galenischen Aufbereitungsformen wie beispielsweise Aerosole oder Sprays, Brausetabletten, Cremes, Dragees, Emulsionen, Gele, Granulate, Injektionslösungen, Kapseln, Kautabletten, Lack- oder Filmtabletten, Lösungen, Lutschtabletten, Pasten, Retardtabletten, Säfte, Salben, Suspensionen, Tabletten, transdermale therapeutische Systeme wie beispielsweise Wirkstoffpflaster, Tropfen oder Zäpfchen (Suppositorien) enthalten sein. Als galenische Aufbereitungsmethode können weiterhin liposomen-verpackte Proteine und Peptide und kovalent mit Transportsubstanzen (zum Beispiel der HIV-TAT-Aminosäuresequenz) fusionierte oder nicht kovalent mit Transportsubstanzen assoziierte Proteine und Peptide verwendet werden. Außerdem können Peptide und Proteine chemisch derart modifiziert werden, dass sie lipophilere Eigenschaften erhalten und daher leichter in Zellen eindringen können. Peptide, die in wässrigen Lösungen nur schwer löslich sind, können umgekehrt chemisch derart modifiziert werden, das sie hydrophiler werden und dann als zum Beispiel intravenös injizierbares Therapeutikum verwendet werden können. Weiterhin können säureresistente Kapseln verwendet werden, um empfindliche Substanzen, die oral verabreicht werden sollen, vor einer vorzeitigen Zersetzung im Magen zu schützen. Die erfindungsgemäßen Nukleinsäuren können insbesondere in Vektoren eingefügt vorliegen, insbesondere beim Einsatz im Rahmen in gentherapeutischer Verfahren. Diese Vektoren können dann in einer dem Fachmann bekannten Weise dem Organismus zugeführt werden. Weiterhin können in bestimmten Fällen auch Zellen, welche einen solchen Vektor beziehungsweise die darauf enthaltenen Syncollinsequenzen tragen, in intakter, abgeschwächter oder toter Form direkt dem Organismus zugeführt werden, wie beispielsweise Zellen, die zur rekombinanten Herstellung der Vektoren eingesetzt werden, aber auch für spezielle Zellsysteme, beispielsweise analog dem Einsatz transgener dendritischer Zellen zur Tumorvakzinierung [47].

Falls in dieser Patenschrift ein Begriff nicht eindeutig definiert ist, oder dem Fachmann auf dem jeweiligem Fachgebiet nicht bekannt sein sollte, oder ein Begriff aus dem Textzusammenhang nicht eindeutig definiert werden kann, so gilt die jeweils in den nachfolgenden Standardwerken genannte Definition des jeweiligen Begriffs. Falls ein Begriff in mehreren der nachfolgend zitierten Werke mit verschiedenen Definitionen aufgeführt wird, so gilt jeweils die Definition, die in dem zuerst in der nachfolgenden Liste aufgeführten Werk genannt wird. Folgende Publikationen werden zu diesem Zweck zitiert:
- The Merek Manual of Diagnosis and Therapy (Seventeenth Edition, 1999, Merck Publications, Merck & Co., Inc.).
- Roche Lexikon Medizin (4. Auflage, 1998, Urban & Fischer Verlag, München)

Die Überschriften in diesem Dokument sind lediglich zur Strukturierung des Textes bestimmt. Sie sind nicht dazu bestimmt, die beschriebenen Sachverhalte zu limitieren oder einzuschränken. Alle Beispiele und Aufzählungen in der vorliegenden Patentanmeldung sind grundsätzlich zur Verdeutlichung des Sachverhalts, nicht aber zur Einschränkung der Ansprüche gedacht. Sie sollen den Erfindungsgedanken näher charakterisieren, sollen jedoch den Äquivalenzbereich der Erfindung nicht eingrenzen.

### Abbildungen

Die Erfindung wird im folgenden anhand von Beispielen näher illustriert. Dabei wird auch Bezug auf die Abbildungen genommen.
- Abbildung 1:: schematische Übersicht zum Metabolischen Syndrom
- Abbildung 2:: Alignment von SYN-1 bis SYN-4
- Abbildung 3:: Massenspektrometrische Messung (MALDI-MS) am Beispiel von SYN-1
- Abbildung 4:: MALDI-MS als quantifizierende massenspektrometrische Methode
- Abbildung 5:: Chromatogramme einer Aminosäuresequenzbestimmung nach Edman am Beispiel von SYN-1
- Abbildung 6:: Signalintensitäten von SYN-1 in Pankreasextrakten von Wildtypmäusen (C57 BL/6J) und ob/ob-Mäusen (B6.V-Lep(ob)) und zugehörige Box-Whisker-Plots
- Abbildung 7:: ROC-Kurve zum Nachweis der Eignung von Syncollinpeptiden als Diagnostika für Stoffwechselerkrankungen am Beispiel von SYN-1

Abbildung 1 zeigt eine schematische Übersicht über verursachende Faktoren, Krankheitsbilder und Folgeerscheinungen des Metabolischen Syndroms.

Abbildung 2 zeigt ein Alignment der Aminosäuresequenzen der Syncollinpeptide SYN-1 bis SYN-4. Dargestellt sind die sowohl die Aminosäuresequenzen des primären Translationsprodukts der für die jeweiligen Syncollinpeptide kodierende Nukleinsäuren (jeweils Sequenzbereich 1 bis 134) als auch die Aminosäuresequenzen des durch posttranslationale Abspaltung eines Signalpeptids (jeweils Sequenzbereich 1 bis 21) entstandenen biologisch aktiven Syncollinpeptids (fettgedruckt; jeweils Sequenzbereich 22 bis 134). SYN-1 (21-134) stellt das hier erstmalig als Protein nachgewiesene Syncollinpeptid der Maus dar, dessen Konzentration im Pankreas mit dem Auftreten von Stoffwechselerkrankungen korreliert. Durch einen Vergleich mit cDNA-Datenbanken wurde ausgehend von der experimentell bestimmten Sequenz (21-134) SEQ ID 1 ermittelt, welche das komplette primäre Translationsprodukt von SYN-1 darstellt (SWISS- Prot Q9DC81). SEQ ID 2 stellt das primäre Translationsprodukt einer natürlich vorkommenden Variante der murinen cDNA des Syncollins dar, deren Translation zu einem Syncollinpeptid SYN-2 führen würde, bei welchem an Position 107 Serin gegen Threonin ausgetauscht ist (SWISS-Prot Q8VCK7). SEQ ID 3 stellt die Aminosäuresequenz eines Syncollinpeptids der Ratte dar, welche durch Kombination von fragmentarischer Aminosäuresequenzierung und Datenbankabgleich ermittelt wurde [15](SWISS-Prot O35775). Zu beachten ist hierbei, dass dieser Swiss-Prot-Orginaleintrag fälschlicherweise N-terminal um 11 Aminosäuren verlängert ist (eingeklammerte Aminosäuren). SEQ ID 4 ist eine Aminosäuresequenz, welche durch Translation einer humanen cDNA (NCBI XP-092067) entsteht, die zur murinen DNA homolog ist und somit das humane Gegenstück des von uns identifizierten murinen Syncollinpeptids SYN-1. Zu beachten ist, dass dieses hypothetische Translationsprodukt der humanen cDNA C-terminal um 16 Aminosäuren gegenüber SYN-1 bis SYN-3 verlängert sein kann (eingeklammerte Aminosäuren).

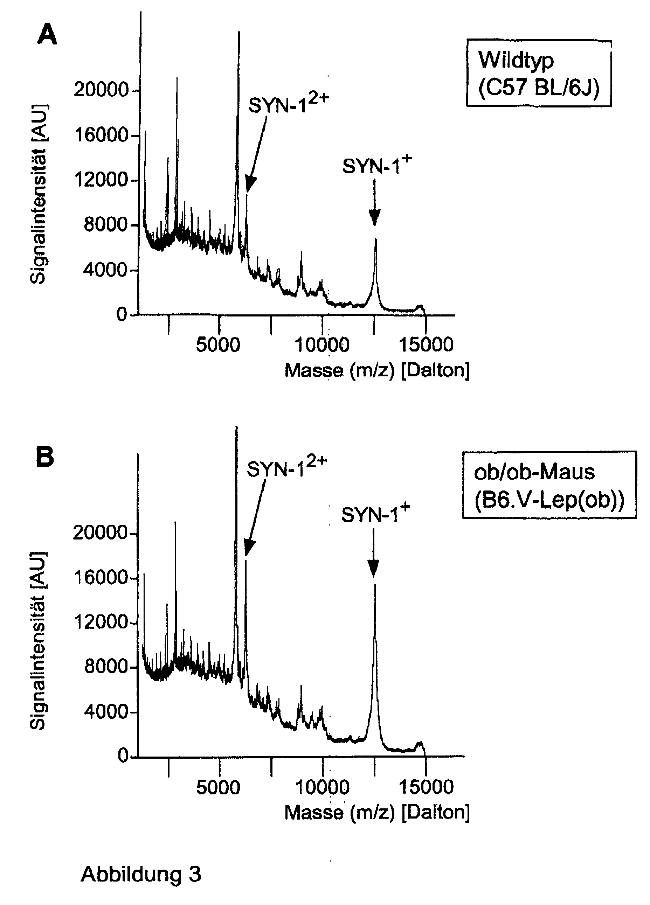

Abbildung 3 bis Abbildung 7 werden im Rahmen der nachfolgenden Ausführungsbeispiele detailliert erläutert.

### Beispiele

Die nachfolgenden Beispiele sind zur exemplarischen Illustration der Erfindung gedacht, sollen aber keinesfalls den Schutzbereich der Erfindung eingrenzen.

### Beispiel 1: Peptidextraktion aus Pankreasgewebe

Pankreata von weiblichen Wildtypmäusen (Stamm C57 BL/6J) und Mäusen eines adipösen Diabetes-Mausmodellsystems (ob/ob-Mäuse; Stamm B6.V-Lep(ob)), welches dem Metabolischen Syndrom des Menschen sehr nahe kommt, wurden von Jackson Laboratory, Bar Harbor, ME, USA bezogen.

Die bei -80°C tiefgekühlten Pankreasproben werden zunächst mit einer kalten 0,2 M wässrigen Essigsäurelösung (10 µl pro mg Pankreasgewebe) versetzt und anschließend auf Eis homogenisiert. Vorzugsweise geschieht dies durch mechanische Homogenisierung, vorzugsweise mittels eines elektrischen Homogenisators, wie beispielsweise Polytron 2100 (Kinematica AG, Luzern, Schweiz), durch mehrmaliges kurzzeitiges Homogenisieren auf Eis, um eine Erwärmung der Probe zu vermeiden. Eine Probe dieses Homogenats wird mit der vierfachen Menge einer 0,575 M wässrigen Essigsäurelösung versetzt und für 10-20 h bei 4°C unter ständigem Schütteln durchmischt. Diese entspricht Probe A. Eine weitere Probe des Homogenats wird mit der vierfachen Menge einer 0,2 M wässrigen Essigsäurelösung versetzt und für 10 min bei 99°C unter ständigem Schütteln durchmischt. Diese entspricht Probe B. Anschließend werden beide Proben für 15 min bei 4°C und 18000 g zentrifugiert. Die Überstände von Probe A und Probe B werden vereinigt und mit der vierzehnfachen Menge einer 0,1%igen wässrigen Trifluoressigsäurelösung versetzt. Die Proben werden bei -80°C gelagert.

### Beispiel 2: Trennung von Peptiden in Pankreashomogenat zur massenspektrometrischen Messung von Syncollinpeptiden

Zum massenspektrometrischen Nachweis von Syncollinpeptiden in Pankreashomogenaten wird in diesem Beispiel eine vorhergehende Trennung der peptidischen Inhaltsstoffe durchgeführt. Diese Probenvorbehandlung dient der Anreicherung der erfindungsgemäßen Peptide und zur Abtrennung vor allem hochmolekularer Komponenten, welche die Messung negativ beeinflussen können. Als Trennverfahren wird eine Reverse-Phase-Chromatographie durchgeführt. Hierbei eignen sich verschiedene RP-Chromatographie-Harze und Elutionsmittel gleichermaßen. Im Folgenden ist beispielhaft die Auftrennung von Peptiden aus Liquor cerebrospinalis unter Verwendung einer C18 Reverse-Phase-Chromatographiesäule mit der Größe 4 mm x 250 mm der Firma Grace Vydac (Hesperia, CA, USA) dargestellt. Es wurden wässrige Laufmittel folgender Zusammensetzung verwendet: Laufmittel A: 0,06 % (v/v) Trifluoressigsäure; Laufmittel B: 0,05 % (v/v) Trifluoressigsäure, 80 % (v/v) Acetonitril. Die Chromatographie erfolgte bei 33 °C unter Verwendung eines Agilent 1100-Chromatographie-Systems (Agilent Technologies, Böblingen, Deutschland). Als Probe wurde nach Beispiel 1 gewonnener Pankreasextrakt verwendet. 1650 µl des Pankreasextraktes wurden für 15 min bei 4°C und 18000 g zentrifugiert und schließlich 1500 µl des Überstandes auf die Chromatographiesäule aufgetragen. Die Chromatographiebedingungen waren wie folgt: 5 % Laufmittel B zum Zeitpunkt 0 min, vom Zeitpunkt 1 bis 45 min kontinuierliche Steigerung der Laufmittel B Konzentration auf 50 %, von Zeitpunkt 45 bis 49 min kontinuierliche Steigerung der Laufmittel B Konzentration auf 100 % und anschließend bis zum Zeitpunkt 53 min konstant 100 % Puffer B. Die Flussgeschwindigkeit betrug 0,5 ml/min. 10 Minuten nach Beginn der Chromatographie wird mit dem Sammeln von 96 Fraktionen zu je 0,25 ml begonnen. Der Verlauf der Chromatographie wurde anhand einer kontinuierlichen Messung der Absorption des Eluats bei 214 nm in Form eines Chromatogramms überwacht.

### Beispiel 3: Ermittlung der Massen von Peptiden mit Hilfe von MALDI-Massenspektrometrie

Zur Massenanalyse werden typische Positiv-Ionen-Spektren von Peptiden in einem MALDI-TOF- (Matrix-Assisted Laser Desorption/Ionisation - Time Of Flight-) Massenspektrometer erstellt. Geeignete MALDI-TOF-Massenspektrometer werden von PerSeptive Biosystems, Foster City, CA, USA (Voyager-DE, Voyager-DE PRO oder Voyager-DE STR) oder von Bruker Daltonik, Bremen (BIFLEX) hergestellt. Zur Messung der erfindungsgemäßen Syncollinpeptide wird ein nach Reverse-Phase-Chromatographie gewonnenes und lyophylisiertes Äquivalent entsprechend 1 mg Pankreasgewebe verwendet. Die chromatographierte und lyophilisierte Probe wird in 15 µl einer Matrix-Lösung gelöst. Diese Matrix-Lösung enthält vorzugsweise 10 g/l Alpha-cyano-4-hydroxyzimtsäure und 10 g/l L(-)Fucose, gelöst in einem Lösungsmittelgemisch bestehend aus Acetonitril, Wasser, Trifluoressigsäure und Aceton im Volumenverhältnis 49:49:1:1. Von dieser Lösung werden 0,3 µl auf eine MALDI-Trägerplatte transferiert und die getrocknete Probe im MALDI-Massenspektrometer Voyager-DE STR von PerSeptive Biosystems analysiert. Die Messung erfolgt im "Linear Mode" mit "Delayed Extraction" ™. Ein Beispiel für eine Messung eines Syncollinpeptids zeigt Abbildung 3 am Beispiel von SYN-1. Zu beachten ist, dass mit dieser sensitiven Methode sowohl das einfach positiv geladene SYN-1⁺ als auch das zweifach positiv geladene SYN-1²⁺ nachgewiesen und quantifiziert werden kann.

Abbildung 3A zeigt eine MALDI-MS-Messung eines Pankreasextrakts aus Wildtypmäusen, Abbildung 3B eine MALDI-MS-Messung eines Pankreasextrakts aus ob/ob-Mäusen. Deutlich ist eine Erhöhung, der Konzentration des hier exemplarisch betrachteten Syncollinpeptids SYN-1 im Pankreas der an Stoffwechselerkrankungen leidenden ob/ob-Mäuse (Abbildung 3B) im Vergleich zu Wildtypmäusen (Abbildung 3A) zu erkennen, sowohl bei Betrachtung des einfach positiv geladenen SYN-1⁺ als auch des zweifach positiv geladenen SYN-1²⁺. Diese Konzentrationsänderung des Syncollinpeptids ist die Grundlage der erfindungsgemäßen Verwendung der Syncollinpeptide als spezifische diagnostische Marker einer Stoffwechselerkrankung und Ansatzpunkt zur Entwicklung neuartiger therapeutischer und prophylaktischer Verfahren zur Bekämpfung und Vermeidung von Stoffwechselerkrankungen, insbesondere im Umfeld des Metabolischen Syndroms.

### Beispiel 4: Quantifizierung der Peptide mit Hilfe von MALDI-Massenspektrometrie

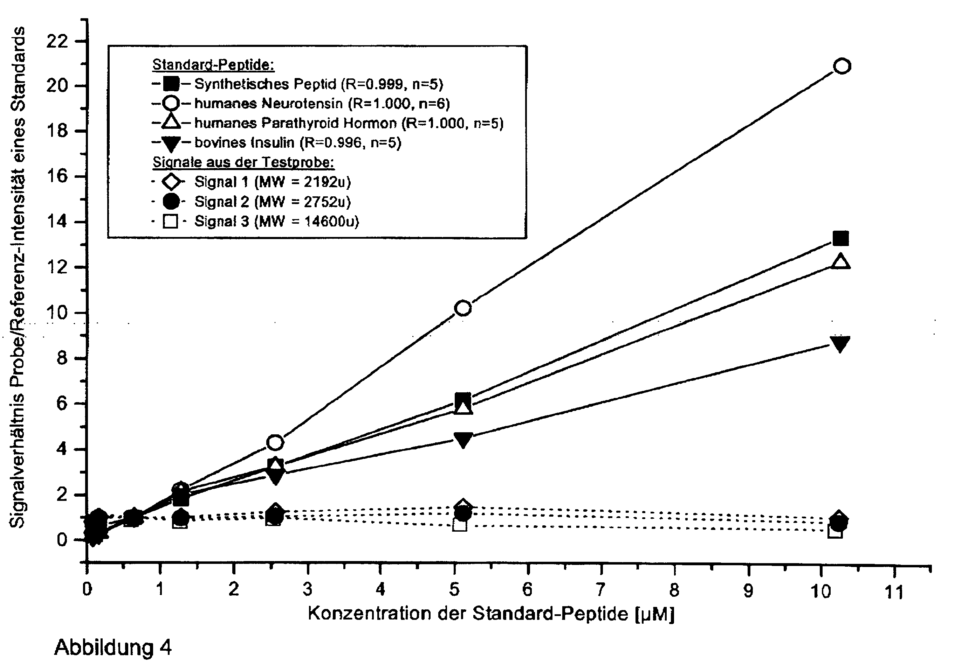

Die MALDI-TOF-Masscnspektrometrie kann zur Quantifizierung von Peptiden, beispielsweise den erfindungsgemäßen Syncollinpeptiden, eingesetzt werden, wenn diese Peptide in einer Konzentration vorliegen, die sich im dynamischen Messbereich des Massenspektrometers befindet, wodurch Detektorsättigung vermieden wird. Für jedes Peptid gibt es ein spezifisches Verhältnis zwischen Messsignal und Konzentration, was bedeutet, dass die MALDI-Massenspektrometrie vorzugsweise zur relativen Quantifizierung von Peptiden herangezogen werden kann. Beispielhaft zeigt Abbildung 4 die Anwendung von MALDI-MS-Messungen als relativ quantifizierende MS-Methode. Hierzu wird eine Probe mit unterschiedlichen Mengen verschiedener Standard-Peptide (im dargestellten Beispiel: bovines Insulin, humanes Neurotensin, humanes Parathyroidhormon, synthetisches Peptid) versetzt und zunächst deren Intensität gemäß Beispiel 3 ermittelt. Alle Signal-Intensitäten der unterschiedlichen Konzentrationen der Standard-Peptide werden auf ihre Signalintensität (Referenzintensität) bei einer Konzentration von 0,64 µM (= 1) normiert und in Form des Signalverhältnisses Probe/Referenz-Intensität, das heißt (Intensität des jeweiligen Standardpeptids bei einer bestimmten Konzentration) / (Referenzintensität des jeweiligen Standardpeptids bei 0,64 µM), gegen die Konzentration des Standard-Peptids aufgetragen. Jedes Standard-Peptid zeigt ein individuelles, typisches Verhältnis von Signalverhältnis zu Konzentration, was anhand des Verlaufs der Kurve, insbesondere bei linearen Verlauf der Kurve anhand der Steigung der Kurve, ablesbar ist. Eine solche Kurve stellt eine Eichkurve für das jeweilige Peptid dar, anhand der eine Signalintensität in eine Peptidkonzentration umgerechnet werden kann. Weiterhin zeigt Abbildung 4 eine interne Kontrolle in Form einer Zugabe einer komplexen Testprobe, welche Peptide in einer für jedes Peptid konstanten Konzentration enthält. Zufällig ausgewählte Peptide (im dargestellten Beispiel: Signal 1, Signal 2, Signal 3) dieser komplexen Testprobe zeigen ein konstantes Signalverhältnis Probe-/Referenz-Intensität, während die zuvor beschriebenen Standard-Peptide eine lineare Änderung dieses Verhältnisses mit steigender Konzentration zeigen, das heißt das massenspektroskopische Verfahren ermöglicht eine exakte Bestimmung der Konzentration von Peptiden unabhängig von parallel in der Probe vorliegenden Begleitpeptiden.

Somit kann aus der experimentell bestimmten Signalintensität eines bestimmten Peptids anhand seiner individuellen Eichkurven seine Konzentration in der Probe ermittelt werden. Daher können solche massenspektrometrische Bestimmungen äquivalent zu anderen üblichen Bestimmungen, wie zum Beispiel ELISA, zur Konzentrationsbestimmung von Peptiden eingesetzt werden.

### Beispiel 5: Massenspektrometrische Identifizierung von Syncollinpeptiden

Zur Quantifizierung der erfindungsgemäßen Syncollinpeptide muss deren Identität sichergestellt werden, das heißt es muss gewährleistet sein, dass es sich bei den zu analysierenden Massensignalen von Peptiden in den Fraktionen, gewonnen durch Reverse-Phase-Chromatographie von Pankreasextrakten gemäß Beispiel 2, tatsächlich um die erfindungsgemäßen Syncollinpeptide handelt.

Die Identifikation der erfindungsgemäßen Peptide in diesen Fraktionen erfolgt zum Beispiel mit Nanospray-MS/MS. Dabei wird zunächst ein Syncollinpeptid-Ion massenspektrometrisch anhand seines spezifischen m/z- (Masse/Ladung-) Wertes in einer dem Fachmann bekannten Art und Weise selektioniert. Dieses selektierte Ion wird anschließend durch Zuführung von Kollisionsenergie mit einem Stoßgas, zum Beispiel Helium oder Stickstoff, fragmentiert und die resultierenden Syncollinpeptid-Bruchstücke im Massenspektrometer in einer integrierten Analyseneinheit detektiert und korrespondierende m/z-Werte bestimmt (Prinzip der Tandem-Massenspektrometrie) [48]. Das Fragmentierungsverhalten von Peptiden ermöglicht bei einer Massengenauigkeit von zum Beispiel 50ppm eine eindeutige Identifizierung der erfindungsgemäßen Syncollinpeptide unter Verwendung von computergestützten Such- und Analyseverfahren in Sequenzdatenbanken, in welche die Sequenz eines Syncollinpeptids, eines zu Syncollin homologen Peptids oder eines deren Vorläufermoleküle eingetragen wurde [49]. In diesem speziellen Fall erfolgte die massenspektrometrische Analyse mit einem Quadrupol-TOF-Massenspektrometer, Modell "QStar-Pulsar" der Firma Applied Biosystems - MDS Sciex, USA.

### Beispiel 6: Identifizierung von Syncollinpeptiden mittels Edman-Abbau

Ein weiteres Verfahren zur Identifikation der erfindungsgemäßen Peptide ist der Edman-Abbau [39]. Beim Edman-Abbau addiert sich die endständige Aminogruppe an Phenylthioisocyanat, wobei ein Thioharnstoffderivat entsteht. Beim Behandeln mit einer schwachen Säure unter Bedingungen, unter denen die Peptidbindungen nicht hydrolysiert werden, spaltet das Molekül die markierte Aminosäure als Phenylthiohydantoin ab, der Rest des Polypeptids bleibt unverändert. Die neue Kette, die eine neue endständige Aminogruppe hat, kann nun einem weiteren Edman-Abbau unterworfen werden, um den nächsten endständigen Aminosäure-Rest zu identifizieren.

Die Bestimmung der Aminosäuresequenz wurde auf einem Sequenzer (Modell 494-(Procise); Applied Biosystems, Weiterstadt) im fast cycle-Protokoll nach den Angaben des Herstellers durchgeführt. Ein Äquivalent aus 12 mg kaltextrahiertem Essigsäure-Gewebeextrakt (Pankreas ob/ob-Maus) wurde nach chromatographischer Aufreinigung auf eine mit PolyBrene Plus (Applied Biosystems) vorbehandelte Glasfasermembran aufgetragen. Die Analyse der abgespaltenen Phenylthiohydantoin-Aminosäuren erfolgte bei einer Wellenlänge von 269 nm über RP-Chromatographie, die an den Sequenzer standardmäßig gekoppelt ist. Die Datenaufnahme und Auswertung wurde mit der Software Model 610A Data Analysis (Applied Biosystems) durchgeführt.

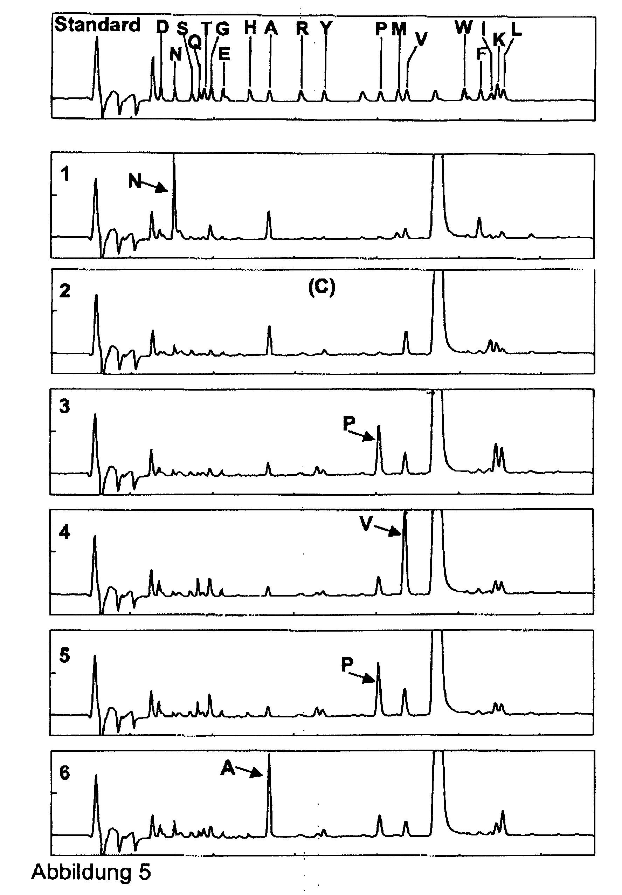

Abbildung 5 zeigt exemplarisch die Chromatogramme eines Aminosäuren-Standards (Standard; oben) und die ersten sechs Abbauschritte des Peptidverdaus nach Edman (1 bis 6; unten). Die Aminosäuren sind im Ein-Buchstaben-Kode angegeben, wobei Cystein aufgrund seiner chemischen Struktur nicht direkt nachgewiesen werden kann, sondern anhand des Fehlens eines sonstigen Aminosäurepeaks im Chromatogramm identifiziert wird (siehe auch Schritt 2). Die Abszisse stellt die relative Zeitachse, die Ordinate die relative Signalintensität des jeweiligen Chromatographiclaufs dar. Die in den jeweiligen Abbauschritten identifizierten Aminosäuren sind in den betreffenden Chromatogrammen gekennzeichnet und beschriftet. Die ersten sechs Aminosäuren der Sequenz des isolierten SYN-1 lauten damit: NCPVPA. Bei der durchgeführten Edman-Sequenzierung wurden insgesamt 18 Aminosäurereste des Syncollinpeptids chemisch bestimmt, wodurch sich unter Zuhilfenahme der experimentell bestimmten Masse des Gesamtpeptids eindeutig die exakte Sequenz von SYN-1 bestimmen ließ. Abbildung 5 zeigt exemplarisch lediglich die ersten sechs Abbauschritte der Edmansequenzierung.

### Beispiel 7: Validierung der Eignung von Syncollinpeptiden zum Nachweis von Stoffwechselerkrankungen

Der Nachweis der Eignung der erfindungsgemäßen Syncollinpeptide zur Diagnostik von Stoffwechselerkrankungen erfolgt insbesondere durch einen Vergleich der Konzentrationen von mindestens einem erfindungsgemäßen Syncollinpeptid in gesunden Organismen und Organismen, welche an Stoffwechselerkrankungen leiden. In einer bevorzugten Ausführungsform werden hierbei die Konzentrationen mindestens eines Syncollinpeptids in mindestens einem Organ, welches an der Regulation von Stoffwechselvorgängen besonders beteiligt ist, in Wildtyptieren und Tieren eines Modellsystems für eine Stoffwechselerkrankung verglichen. Solche bevorzugten Organe sind beispielsweise Pankreas, Leber, Hypophyse, Hypothalamus, Fettgewebe, Magen, Darm, Niere und weitere. In einer besonders bevorzugten Ausführungsform wurden hierbei zunächst Pankreasproben von je 8 weiblichen Wildtyp-Mäusen (Stamm C57 BL/6J) und Mäusen eines adipösen Diabetes-Mausmodellsystems (ob/ob-Mäuse; Stamm B6.V-Lep(ob)), welches dem Metabolischen Syndrom des Menschen sehr nahe kommt, entsprechend Beispiel 1 und Beispiel 2 vorbereitet. Anschließend wurden die Konzentrationen des Syncollinpeptids SYN-1 in den Proben entsprechend Beispiel 3 bestimmt, wobei als Maß für die Konzentration des Syncollinpeptids SYN-1 dessen MALDI-massenspektrometrische Signalintensität nach Beispiel 4 verwendet wird. Zu Minimierung methodischer und experimenteller Messfehlcr erfolgte jede Konzentrationsbestimmung als Doppelmessung. Schließlich wurde die Identität des Syncollinpeptids SYN-1 entsprechend Beispiel 5 und Beispiel 6 nachgewiesen.

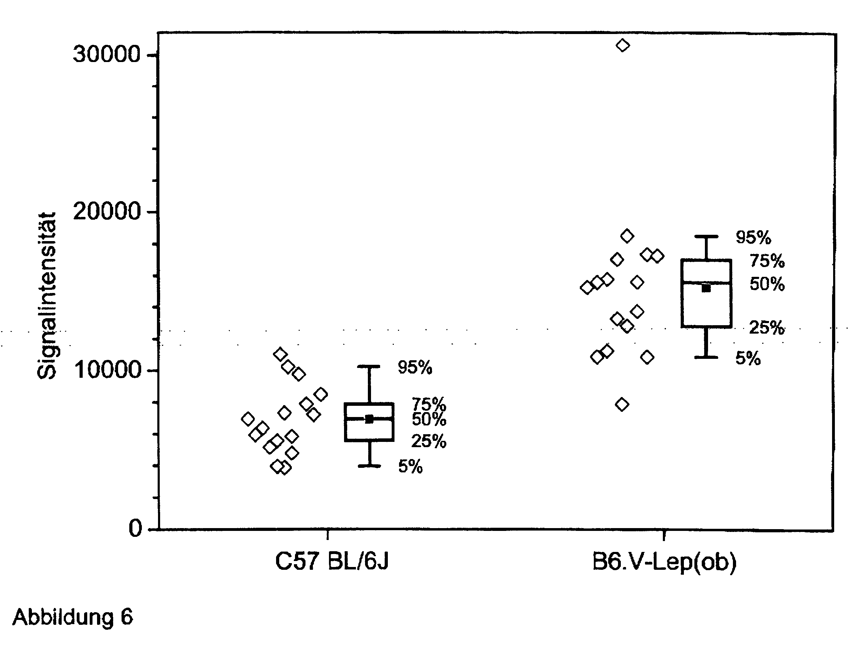

Die so bestimmten Konzentrationen des Syncollinpeptids SYN-1 sind in Abbildung 6 jeweils links in Form der Rohdaten (Rauten) dargestellt. Eine statistische Auswertung dieser Daten in Form von Box-Whisker-Plots ist jeweils auf der rechten Seite dargestellt. Hierbei stellen die Boxen die obere beziehungsweise untere Quartile dar, die Balken die 5% beziehungsweise 95% Percentile, d.h. zwischen diesen Balken liegen 90% der Messwerte. Die waagrechte Linie innerhalb der Boxen stellt den Medianwert, das Rechteck den arithmetischen Mittelwert der gemessen Konzentrationen dar. Die dargestellten Box-Whisker-Plots sind in der medizinischen Biometrie häufig eingesetzte Darstellungsmöglichkeiten der Verteilung von Messwerten und ermöglichen den statistischen Vergleich MALDI-massenspektrometrischer Signalintensitäten und damit der Konzentrationen der Syncollinpeptide in Wildtyp- und ob/ob-Mäusen. Deutlich zeigt sich im dargestellten Beispiel eine mehr als 2,2fache Erhöhung des SYN-1-Konzentration im Pankreas von ob/ob-Mäusen im Vergleich zu Wildtypmäusen, was eine entscheidende Bedeutung dieses Peptids beim Auftreten von Stoffwechselerkrankungen anzeigt.

### Beispiel 8: ROC-Analyse des Syncollinpeptids SYN-1 zur Validierung der Eignung von Syncollinpeptiden zum Diagnose von Stoffwechselerkrankungen

Zur weiteren Validierung des Eignung von Syncollinpeptiden zum Feststellung von Stoffwechselerkrankungen können weitere statistische Auswertungen vorgenommen werden. Insbesondere können statistische Signifikanztests durchgeführt werden, mit deren Hilfe ermittelt werden kann, ob sich die experimentell bestimmten Konzentrationen der Syncollinpeptide signifikant unterscheiden und damit als diagnostische Marker zur Feststellung einer Stoffwechselerkrankung geeignet sind. Im vorliegenden Fall wurde zur weiteren statistischen Auswertung der parametrische t-Test für unverbundene Proben gewählt, da die gemessenen Konzentrationen der beiden Kollektive Wildtyp-Mäuse und ob/ob-Mäuse eine annähernde Normalverteilung zeigen. Die Durchführung eines solchen Testes für die in Abbildung 6 dargestellten Konzentrationen von SYN-1 ergab einen t-Wert (mittlere Differenz dividiert durch Standardfehler des Mittels) von -6,08 und einen p-Wert (Irrtumswahrscheinlichkeit) vom 0,0000011, das heißt die Konzentration des Syncollinpeptids SEQ ID 1 ist im Pankreasgewebe von ob/ob-Mäusen hochsignifikant gegenüber Wildtypmäusen erhöht und korreliert mit dem Auftreten von Stoffwechselerkrankungen. Syncollinpeptide sind somit als diagnostische Marker zum Nachweis von Stoffwechselerkrankungen, insbesondere im Umfeld des Metabolischen Syndroms, hervorragend geeignet.

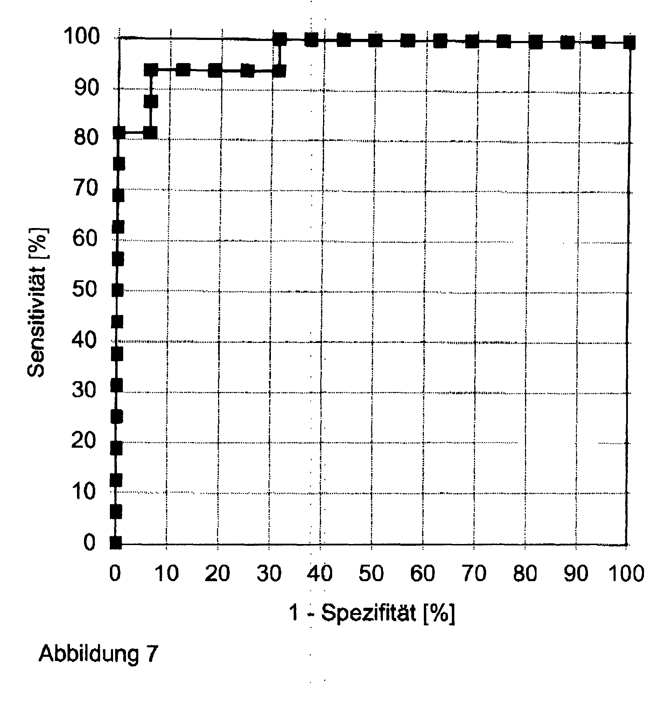

Für die Beurteilung der Qualität diagnostischer Verfahren werden in der medizinischen Biometrie weitere spezielle statische Methoden eingesetzt. Insbesondere dient hierbei die ROC-(Receiver Operating Characteristic-) Analyse zur Validierung der Güte eines Diagnostikums in Hinblick auf Spezifität und Sensitivität. Als Sensitivität wird der Anteil der erkrankten Organismen definiert, die bei einer Diagnose auf die Erkrankung ein positives Diagnoseergebnis erhalten, das heißt die Diagnose zeigt die Erkrankung korrekt an. Als Spezifität wird der Anteil der gesunden Organismen definiert, die bei einer Diagnose auf die Erkrankung ein negatives Diagnoseergebnis erhalten, das heißt die Diagnose zeigt korrekt an, dass keine Erkrankung vorliegt. Ein geeigneter diagnostischer Marker sollte sowohl eine hohe Spezifität als auch eine hohe Sensitivität aufweisen. Prinzipiell wird hierbei für jeden Messwert die jeweilige Sensitivität und Spezifität bestimmt und ein ROC-Diagramm in Form einer Auftragung Sensitivität gegen (1-Spezifität) erstellt [50]. Die Zuordnung von Sensitivität und Spezifität zu den jeweiligen Signalintensitäten des Syncollinpeptids SYN- 1 zeigt Tabelle 1, das zugehörige ROC-Diagramm zeigt Abbildung 7. Deutlich zeigt das ROC-Diagramm, dass Syncollinpeptid SYN-1 in besonderem Maße als diagnostisches Unterscheidungskriterium zwischen gesunden Organismen und Organismen mit Stoffwechselerkrankungen geeignet ist. Dieses Peptid weist sowohl eine sehr hohe Sensitivität als auch eine sehr hohe Spezifität in Hinblick auf eine Unterscheidung gesunder und erkrankter Organismen auf, was die starke Annäherung der ROC-Kurve zu hohen Sensitivitäts- und Spezifitätswerten (linke obere Ecke) zeigt.

Insbesondere das ROC-Integral (Fläche unter der ROC-Kurve) wird als Qualitätsmaß für die Verwendung eines Peptids als Biomarker in der Diagnostik eingesetzt. Je größer dieser Wert ist, desto spezifischer und sensitiver unterscheidet das jeweilige Peptid kranke und gesunde Organismen, wobei eine ideale diagnostische Unterscheidungskraft einem ROC-Integral von 1,0 und eine vollständige fehlende Unterscheidungskraft einem ROC-Integral von 0,5 entspricht. Das ROC-Integral von Syncollinpeptid SYN- 1 besitzt einen sehr hohen Wert von 0,973. Dieses Peptid eignet sich folglich sehr gut als Marker zum Nachweis von Stoffwechselerkrankungen, insbesondere in Zusammenhang mit dem Metabolischen Syndrom.

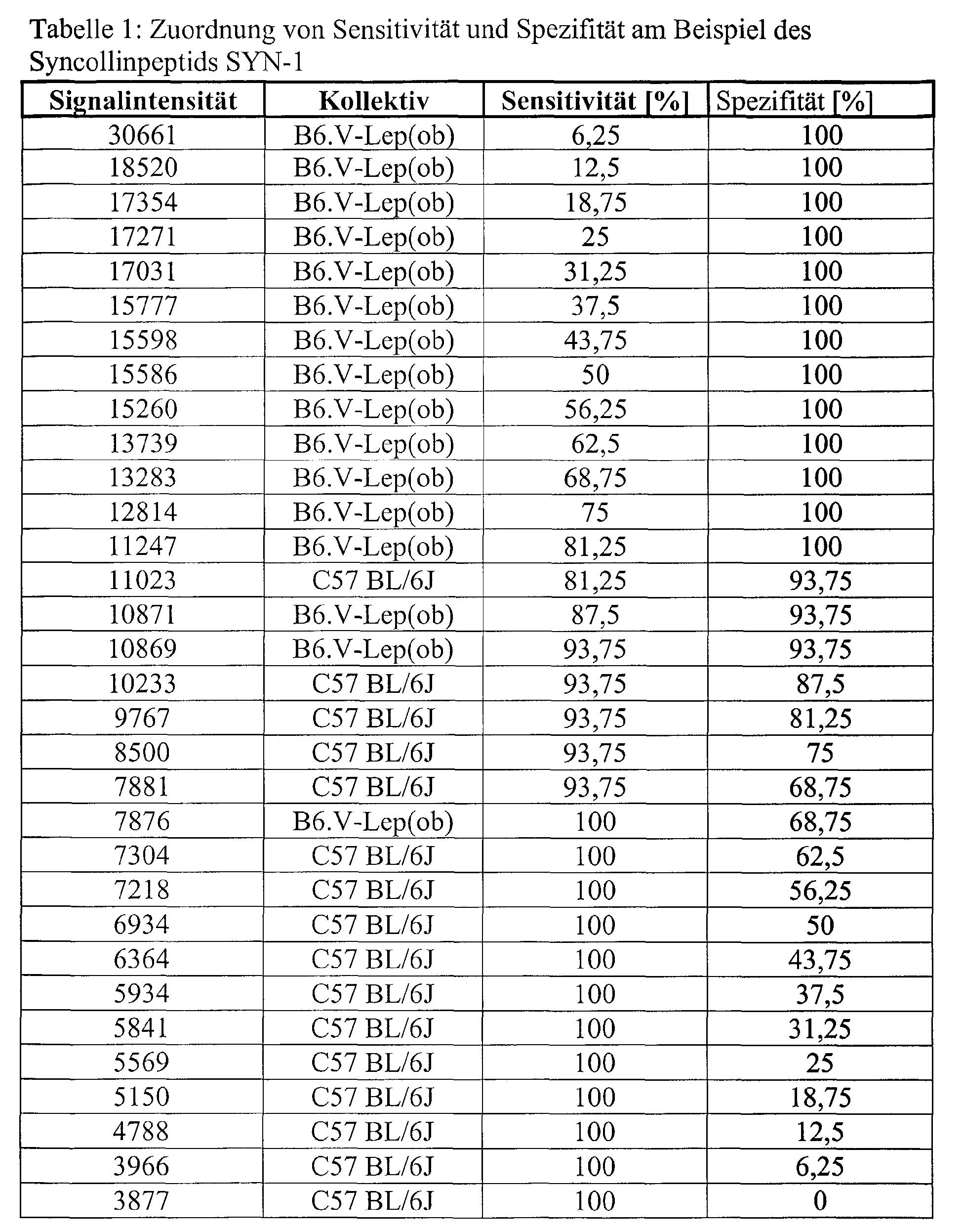

Somit konnte nachgewiesen werden, dass sich Syncollinpeptide, insbesondere das Syncollinpeptid SYN-1, hervorragend als diagnostischer Marker zur Feststellung des Vorhandenseins einer Stoffwechselerkrankung, insbesondere im Umfeld des Metabolischen Syndroms einschließlich seiner Krankheitsbilder Adipositas, Insulinresistenz, Glukoseintoleranz, Typ 2-Diabetes, Dyslipidämie, Hypertonie, Veränderungen des Gefäßsystems und Hyperkoagulabilität, aber auch anderer Stoffwechselerkrankungen wie beispielsweise Typ 1-Diabetes, Anorexie, Bulimie oder Kachexie, eignet. Weiterhin dienen die hier erstmals ermittelten krankheitsspezifischen Konzentrationsänderungen von Syncollinpeptiden als Grundlage für die erfindungsgemäße Verwendung der Syncollinpeptide als spezifische diagnostische Marker einer Stoffwechselerkrankung und als spezifische Ansatzpunkte zur Entwicklung neuartiger therapeutischer und prophylaktischer Verfahren zur Bekämpfung und Vermeidung von Stoffwechselerkrankungen, insbesondere im Umfeld des Metabolischen Syndroms, auf Basis von Syncollinpeptiden oder Syncollin-kodierenden Nukleinsäuren.

Falls in dieser Patenschrift ein Begriff nicht eindeutig definiert ist, oder dem Fachmann auf dem jeweiligem Fachgebiet nicht bekannt sein sollte, oder ein Begriff aus dem Textzusammenhang nicht eindeutig definiert werden kann, so gilt die jeweils in den nachfolgenden Standardwerken genannte Definition des jeweiligen Begriffs. Falls ein Begriff in mehreren der nachfolgend zitierten Werke mit verschiedenen Definitionen aufgeführt wird, so gilt jeweils die Definition, die in dem zuerst in der nachfolgenden Liste aufgeführten Werk genannt wird. Folgende Publikationen werden zu diesem Zweck zitiert:
- The Merck Manual of Diagnosis and Therapy (Seventeenth Edition, 1999, Merck Publications, Merck & Co., Inc.).
- Roche Lexikon Medizin (4. Auflage, 1998, Urban & Fischer Verlag, München)

Die Überschriften in diesem Dokument sind lediglich zur Strukturierung des Textes bestimmt. Sie sind nicht dazu bestimmt, die beschriebenen Sachverhalte zu limitieren oder einzuschränken. Alle Beispiele und Aufzählungen in der vorliegenden Patentanmeldung sind grundsätzlich zur Verdeutlichung des Sachverhalts, nicht aber zur Einschränkung der Ansprüche gedacht. Sie sollen den Erfindungsgedanken näher charakterisieren, sollen jedoch den Äquivalenzbereich der Erfindung nicht eingrenzen.

Sequenzprotokoll

<110>  BioVisioN AG

<120>  Syncollin

<130>  Syncollin

<160>  12

<170>  PatentIn version 3.1

<210>  1
<211>  134
<212>  PRT
<213>  Mus musculus

<400>  1

Met Ser Leu Leu Cys Pro Leu Leu Leu Ala Leu Ala Leu Val Ala Val
1               5                   10                  15

Pro Gly Ala His Gly Asn Cys Pro Val Pro Ala Asp Leu Lys Lys Ser
            20                  25                  30

Asp Gly Thr Arg Thr Cys Ala Arg Leu Tyr Glu Lys Ser Asp Pro Tyr
        35                  40                  45

Tyr Asp Asn Cys Cys Gln Gly Pro Val Leu Ser Val Glu Pro Gly Thr
    50                  55                  60

Asp Leu Pro Tyr Leu Pro Ser Gly Trp Ser Asn Thr Ala Ser Ser Leu
65                  70                  75                  80

Val Val Gly Gln Arg Cys Glu Ile Thr Val Trp Ser Leu Pro Gly Lys
                85                  90                  95

His Gly Lys Thr Arg Lys Phe Thr Ala Gly Ser Tyr Pro Arg Leu Glu
            100                 105                 110

Glu Tyr Arg Lys Gly Ile Phe Gly Asp Trp Ser Asp Ser Ile Ser Ala
        115                 120                 125

Leu Tyr Cys Lys Cys Tyr
    130


<210>  2
<211>  134
<212>  PRT
<213>  Mus musculus

<400>  2

Met Ser Leu Leu Cys Pro Leu Leu Leu Ala Leu Ala Leu Val Ala Val
1               5                   10                  15

Pro Gly Ala His Gly Asn Cys Pro Val Pro Ala Asp Leu Lys Lys Ser
            20                  25                  30

Asp Gly Thr Arg Thr Cys Ala Arg Leu Tyr Glu Lys Ser Asp Pro Tyr
        35                  40                  45

Tyr Asp Asn Cys Cys Gln Gly Pro Val Leu Ser Val Glu Pro Gly Thr
    50                  55                  60

Asp Leu Pro Tyr Leu Pro Ser Gly Trp Ser Asn Thr Ala Ser Ser Leu
65                  70                  75                  80

Val Val Gly Gln Arg Cys Glu Ile Thr Val Trp Ser Leu Pro Gly Lys
                85                  90                  95

His Gly Lys Thr Arg Lys Phe Thr Ala Gly Thr Tyr Pro Arg Leu Glu
            100                 105                 110

Glu Tyr Arg Lys Gly Ile Phe Gly Asp Trp Ser Asp Ser Ile Ser Ala
        115                 120                 125

Leu Tyr Cys Lys Cys Tyr
    130


<210>  3
<211>  145
<212>  PRT
<213>  Rattus norvegicus

<400>  3

Met Ala Tyr His Glu Lys Trp Ser Ile Val Thr Met Ser Pro Leu Cys
1               5                   10                  15

Leu Leu Leu Leu Ala Leu Ala Leu Val Ala Val Pro Gly Ala Arg Gly
            20                  25                  30

Ala Cys Pro Val Pro Ala Asp Leu Lys Lys Ser Asp Gly Thr Arg Thr
        35                  40                  45

Cys Ala Arg Leu Tyr Glu Asn Ser Asp Pro Tyr Tyr Asp Asn Cys Cys
    50                  55                  60

Gln Gly Pro Glu Leu Ser Val Asp Pro Gly Thr Asp Leu Pro Tyr Leu
65                  70                  75                  80

Pro Ser Asp Trp Ser Asn Ser Ala Ser Ser Leu Val Val Ala Gln Arg
                85                  90                  95

Cys Glu Leu Thr Val Trp Ser Leu Pro Gly Lys Arg Gly Lys Thr Arg
            100                 105                 110

Lys Phe Ser Thr Gly Ser Tyr Pro Arg Leu Glu Glu Tyr Arg Lys Gly
        115                 120                 125

Ile Phe Gly Thr Trp Ala Lys Ser Ile Ser Gly Leu Tyr Cys Lys Cys
    130                 135                 140

Tyr
145


<210>  4
<211>  150
<212>  PRT

<213>  Homo sapiens

<400>  4

Met Ser Pro Leu Arg Pro Leu Leu Leu Ala Leu Ala Leu Ala Ser Val
1               5                   10                  15

Pro Cys Ala Gln Gly Ala Cys Pro Ala Ser Ala Asp Leu Lys His Ser
            20                  25                  30

Asp Gly Thr Arg Thr Cys Ala Lys Leu Tyr Asp Lys Ser Asp Pro Tyr
        35                  40                  45

Tyr Glu Asn Cys Cys Gly Gly Ala Glu Leu Ser Leu Glu Ser Gly Ala
    50                  55                  60

Asp Leu Pro Tyr Leu Pro Ser Asn Trp Ala Asn Thr Ala Ser Ser Leu
65                  70                  75                  80

Val Val Ala Pro Arg Cys Glu Leu Thr Val Trp Ser Arg Gln Gly Lys
                85                  90                  95

Ala Gly Lys Thr His Lys Phe Ser Ala Gly Thr Tyr Pro Arg Leu Glu
            100                 105                 110

Glu Tyr Arg Arg Gly Ile Leu Gly Asp Trp Ser Asn Ala Ile Ser Ala
        115                 120                 125

Leu Tyr Cys Arg Cys Pro Ala Ala Gln Ser Ser Glu Pro Thr Pro Gly
    130                 135                 140

Pro Gly Pro Ser Ser Ala
145                 150


<210>  5
<211>  560
<212>  DNA
<213>  Mus musculus

<400>  5
ggcacctcag acctctcatg gttcattgtc accatgtccc tgctgtgccc actgctgctg      60

gctttagccc tggtggctgt ccctggtgcc catggcaatt gcccggtgcc cgcagacctg     120

aagaagtcag atgggacgcg tacgtgcgcc agactctatg agaagagtga tccatactat     180

gacaactgct gccaggggcc cgtgttgtct gtggagccag gtactgacct gccctatctg     240

ccctcgggct ggtctaacac tgcatcgtct ctggtagtgg gccagcgctg tgagatcact     300

gtgtggtctc tccccggtaa gcatggcaag acccgcaagt tcaccgcggg ttcttaccct     360

cgcctggaag agtaccgcaa aggcatcttt ggagactggt ccgactccat ctctgccctc     420

tactgcaagt gctattgatg ccttgaggct ctgtctaccc agcctggcct tgggaattgc     480

tgtagctcca agagccagga ggcaagatga ccccacgacc tgctctcata gcttccctgt     540

aatacagccc tttcaaagtt                                                 560


<210>  6

<211>  589
<212>  DNA
<213>  Mus musculus

<400>  6
agacctctcg tggttcattg tcaccatgtc cctgctgtgc ccactgctgc tggctttagc      60

cctggtggct gtccctggtg cccatggcaa ttgcccggtg cccgcagacc tgaagaagtc     120

agatgggacg cgtacgtgcg ccagactcta tgagaagagt gatccatact atgacaactg     180

ctgccagggg cccgtgttgt ctgtggagcc aggtactgac ctgccctatc tgccctcggg     240

ctggtctaac actgcatcgt ctctggtagt gggccagcgc tgtgagatca ctgtgtggtc     300

tctccccggt aagcatggca agacccgcaa gttcaccgcg ggtacttacc ctcgcctgga     360

agagtaccgc aaaggcatct ttggagactg gtccgactcc atctctgccc tctactgcaa     420

gtgctattga tgccttgagg ctctgtctac ccagcctggc cttgggaatt gctgtagctc     480

caagagccag gaggcaagat gaccccacga cctgctctca tagcttccct gtaatacagc     540

cctttcaaag ttaaaaaaaa aaaaaaaaaa aaaaaaaaaa aaaaaaaaa                589


<210>  7
<211>  609
<212>  DNA
<213>  Rattus norvegicus

<400>  7
atggcatatc acgaaaaatg gtccattgtc accatgtccc cgctgtgcct gctgttgctg      60

gctttagccc tggtggctgt ccccggtgcc cgaggcgctt gtccagtgcc cgcagacctg     120

aagaagtcag atgggacgcg cacgtgcgcc aggctctatg agaacagtga cccctactat     180

gacaactgct gccaggggcc tgaactgtct gtggatccag gcaccgacct gccctacctg     240

ccctcggact ggtctaactc ggcatcttct ctggtagtgg cccagcgctg cgagctcacc     300

gtgtggtctc tccctggtaa acgtggcaag acacgcaagt tctctacggg tagctaccct     360

cgactggaag agtaccgcaa aggcatcttt ggaacctggg ccaagtccat ctctggcctc     420

tactgcaagt gctattgatg tcttgagggt ctgtcgtctc tgattctgca cagtgctgag     480

ccttacccag tctgtccttg gggattgctc tgaccccaag agccaggagg caagatggcc     540

ccattacctg ctatcagagc ttccctataa tacagccctg gtgaagctaa aaaaaaaaaa     600

aaaaaaaaa                                                            609


<210>  8
<211>  453
<212>  DNA
<213>  Homo sapiens

<400>  8
atgtccccgc tgcgcccgct gctgctggcc ctggcccttg cctccgtgcc ttgcgcccag      60

ggcgcctgcc ccgcctccgc cgacctcaag cactcggacg ggacgcgcac ttgcgccaag 120

ctctatgaca agagcgaccc ctactatgag aactgctgcg ggggcgccga gctgtcgctg 180

gagtcgggcg cagacctgcc ctacctgccc tccaactggg ccaacaccgc ctcctcactt 240

gtggtggccc cgcgctgcga gctcaccgtg tggtcccggc aaggcaaggc gggcaagacg 300

cacaagttct ctgccggcac ctacccgcgc ctggaggagt accgccgggg catcttagga 360

gactggtcca acgctatctc cgcgctctac tgcaggtgcc cggccgccca aagctccgag 420

cccaccccag gcccaggccc cagctccgca taa 453

<210> 9
<211> 402
<212> DNA
<213> Mus musculus

<400> 9
atgtccctgc tgtgcccact gctgctggct ttagccctgg tggctgtccc tggtgcccat 60

ggcaattgcc cggtgcccgc agacctgaag aagtcagatg ggacgcgtac gtgcgccaga 120

ctctatgaga agagtgatcc atactatgac aactgctgcc aggggcccgt gttgtctgtg 180

gagccaggta ctgacctgcc ctatctgccc tcgggctggt ctaacactgc atcgtctctg 240

gtagtgggcc agcgctgtga gatcactgtg tggtctctcc ccggtaagca tggcaagacc 300

cgcaagttca ccgcgggttc ttaccctcgc ctggaagagt accgcaaagg catctttgga 360

gactggtccg actccatctc tgccctctac tgcaagtgct at 402

<210> 10
<211> 402
<212> DNA
<213> Mus musculus

<400> 10
atgtccctgc tgtgcccact gctgctggct ttagccctgg tggctgtccc tggtgcccat 60

ggcaattgcc cggtgcccgc agacctgaag aagtcagatg ggacgcgtac gtgcgccaga 120

ctctatgaga agagtgatcc atactatgac aactgctgcc aggggcccgt gttgtctgtg 180

gagccaggta ctgacctgcc ctatctgccc tcgggctggt ctaacactgc atcgtctctg 240

gtagtgggcc agcgctgtga gatcactgtg tggtctctcc ccggtaagca tggcaagacc 300

cgcaagttca ccgcgggtac ttaccctcgc ctggaagagt accgcaaagg catctttgga 360

gactggtccg actccatctc tgccctctac tgcaagtgct at 402

<210> 11
<211> 435
<212> DNA
<213> Rattus norvegicus

<400> 11

atggcatatc acgaaaaatg gtccattgtc accatgtccc cgctgtgcct gctgttgctg 60

gctttagccc tggtggctgt ccccggtgcc cgaggcgctt gtccagtgcc cgcagacctg 120

aagaagtcag atgggacgcg cacgtgcgcc aggctctatg agaacagtga cccctactat 180

gacaactgct gccaggggcc tgaactgtct gtggatccag gcaccgacct gccctacctg 240

ccctcggact ggtctaactc ggcatcttct ctggtagtgg cccagcgctg cgagctcacc 300

gtgtggtctc tccctggtaa acgtggcaag acacgcaagt tctctacggg tagctaccct 360

cgactggaag agtaccgcaa aggcatcttt ggaacctggg ccaagtccat ctctggcctc 420

tactgcaagt gctat 435

<210> 12
<211> 450
<212> DNA
<213> Homo sapiens

<400> 12
atgtccccgc tgcgcccgct gctgctggcc ctggcccttg cctccgtgcc ttgcgcccag 60

ggcgcctgcc ccgcctccgc cgacctcaag cactcggacg ggacgcgcac ttgcgccaag 120

ctctatgaca agagcgaccc ctactatgag aactgctgcg gggcgccgga gctgtcgctg 180

gagtcgggcg cagacctgcc ctacctgccc tccaactggg ccaacaccgc ctcctcactt 240

gtggtggccc cgcgctgcga gctcaccgtg tggtcccggc aaggcaaggc gggcaagacg 300

cacaagttct ctgccggcac ctacccgcgc ctggaggagt accgccgggg catcttagga 360

gactggtcca acgctatctc cgcgctctac tgcaggtgcc cggccgccca aagctccgag 420

cccaccccag gcccaggccc cagctccgca 450

Erläuterungen:

<110>: Anmelder
<120>: Bezeichnung der Anmeldung
<130>: Kurzbezeichnung
<160>: Zahl der eingetragenen Sequenzen
<170>: Programmversion

<210>: Sequenz-ID
<211>: Anzahl der Aminosäuren/Nukleotide
<212>: Sequenztyp (PRT = Protein / Peptid; DNA = Nukleinsäure)
<213>: Spezies
<400>: Sequenz

## Patentansprüche

1. Verfahren zum Auffinden von Anzeichen für das Vorliegen einer Stoffwechselerkrankung oder einer erhöhten Disposition für eine solche Erkrankung durch Bestimmung wenigstens einer Markersubstanz in einer biologischen Probe eines Organismus, **dadurch gekennzeichnet, dass** die Markersubstanz
a) ein Syncollinpeptid oder
b) eine Syncollin-kodierende Nukleinsäure ist.

2. Verfahren gemäß Anspruch 1, **dadurch gekennzeichnet, dass**
a) eine für die jeweilige Markersubstanz spezifische Konzentrationsänderung in der Probe festgestellt wird und
b) eine signifikante Konzentrationsänderung der Markersubstanz in der unter a) genannten Weise als positives Testergebnis für das Vorliegen einer Stoffwechselerkrankung gewertet wird.

3. Verfahren gemäß Anspruch 1 oder 2, **dadurch gekennzeichnet, dass**
das Syncollinpeptid
a) ein Syncollinpeptid entsprechend SEQ ID 1 ist, oder
b) ein Syncollinpeptid entsprechend SEQ ID 2 bis SEQ ID 4 ist, oder
c) ein natürlich vorkommender Abkömmling, insbesondere abstammend von einem natürlich vorkommenden Allel der unter a) und b) genannten Peptide ist, oder
d) eine Mutante eines der unter a) bis c) genannten Peptide ist, wobei die Aminosäuresequenz mindestens 70% Homologie zu einer unter a) bis c) genannten Aminosäuresequenz aufweist, oder
e) eine Mutante eines der unter a) bis c) genannten Peptide ist, wobei die Mutante vorzugsweise in maximal 33 Aminosäuren von einer unter a) bis c) genannten Aminosäuresequenz abweicht, oder
f) eine Fragment eines der unter a) bis e) genannten Peptide ist, oder
g) ein natürlich vorkommendes Vorläuferprotein eines der unter a) bis e) genannten Peptide ist, oder
h) ein chemisch, enzymatisch oder posttranslational modifiziertes Peptid eines der unter a) bis g) genannten Peptide ist, oder
die Syncollin-kodierende Nukleinsäure
i) eine für ein Syncollinpeptid gemäß a) bis h) kodierende Nukleinsäure ist, oder
j) eine Nukleinsäure mit einer Sequenz entsprechend SEQ ID 5 bis SEQ ID 12 ist, oder
k) eine Mutante eines der unter i) oder j) genannten Nukleinsäuren ist, wobei die Nukleotidsequenz mindestens 60% Homologie zu einer unter i) bis j) genannten Nukleotidsequenz aufweist, oder
1) ein Fragment einer Nukleinsäure gemäß i) bis k) ist, oder
m) eine natürlich vorkommende Vorläufernukleinsäure einer Nukleinsäure gemäß i) bis k) ist, oder
n) eine chemisch, enzymatisch oder posttranskriptional modifizierte Nukleinsäure einer der unter i) bis m) genannten Nukleinsäuren ist.

4. Verfahren gemäß einem der Ansprüche 1 bis 3, **dadurch gekennzeichnet, dass** es
a) in Kombination mit anderen Diagnoseverfahren zur Erhöhung von deren Sensitivität und/oder Spezifität durchgeführt wird, oder
b) zur Bestimmung des Vorliegens, der Schwere oder von Vorstufen einer Stoffwechselerkrankung herangezogen wird, oder
c) zur Prognose des Verlaufs einer Stoffwechselerkrankung herangezogen wird, oder
d) zur Stratifizierung von Individuen, welche für eine Therapie oder eine klinischen Studie einer Stoffwechselerkrankung geeignet sind, herangezogen wird, oder
e) zur Bestimmung der Wirksamkeit einer Therapie zur Behandlung von Stoffwechselerkrankungen herangezogen wird.

5. Verfahren gemäß einem der Ansprüche 1 bis 4, **dadurch gekennzeichnet, dass** die Stoffwechsclerkrankung
a) das Metabolische Syndrom ist, oder
b) Diabetes, insbesondere Diabetes mellitus, insbesondere nicht-insulinabhängiger Diabetes mellitus (Typ II-Diabetes) ist, oder
c) Adipositas ist, oder
d) durch mindestens eines der Symptome Insulinresistenz, verminderte Glucosetoleranz, Hyperinsulinämie, endotheliale Dysfunktion, arterielle Hypertonie, erhöhte VLDL-Triglyccride oder erniedrigtes HDL-Cholesterin charakterisiert ist, oder
e) Anorexie, Bulimie oder Kachexie ist.

6. Verfahren gemäß einem der Ansprüche 1 bis 5, **dadurch gekennzeichnet, dass** die biologische Probe
a) Serum, Plasma, Stuhl, Urin, Chymus, Darmsaft, Liquor cerebrospinalis, Synovialflüssigkeit, Tränenflüssigkeit, Sputum oder ein Zell-, Gewebe- oder Organextrakt, insbesondere ein Pankreas-, Leber-, Darm-, Fett- oder Muskelextrakt, ist, und/oder
b) vor der Identifizierung oder Quantifizierung chromatographisch fraktioniert wird, vorzugsweise mit Reverse Phase-Chromatographie, und/oder
c) vor der Identifizierung oder Quantifizierung durch Fällungsreaktionen und/oder Phasentrennungen fraktioniert wird.

7. Syncollinpeptid, **gekennzeichnet dadurch, dass** es
a) ein Syncollinpeptid entsprechend SEQ ID 1 ist, oder
b) ein Syncollinpeptid entsprechend SEQ ID 2 bis SEQ ID 4 ist, oder
c) ein natürlich vorkommender Abkömmling, insbesondere abstammend von einem natürlich vorkommenden Allel der unter a) und b) genannten Peptide ist, oder
d) eine Mutante eines der unter a) bis c) genannten Peptide ist, wobei die Aminosäuresequenz mindestens 70% Homologie zu einer unter a) bis c) genannten Aminosäuresequenz aufweist, oder
e) eine Mutante eines der unter a) bis c) genannten Peptide ist, wobei die Mutante vorzugsweise in maximal 33 Aminosäuren von einer unter a) bis c) genannten Aminosäuresequenz abweicht, oder
f) eine Fragment eines der unter a) bis e) genannten Peptide ist, oder
g) ein natürlich vorkommendes Vorläuferprotein eines der unter a) bis e) genannten Peptide ist, oder
h) ein Peptid gemäß a) bis g) ist, welches ein Bestandteil eines Makromoleküls, insbesondere eines Fusionsproteins, oder eines Proteinkomplexes ist, oder
i) ein chemisch, enzymatisch oder posttranslational modifiziertes Peptid eines der unter a) bis h) genannten Peptide ist, oder
j) ein Peptid oder Peptidanalog, insbesondere ein Peptidomimetikum, ist, welches eine biologische Syncollinaktivität besitzt.

8. Syncollin-kodierende Nukleinsäure, **gekennzeichnet dadurch, dass** sie
a) eine für ein Syncollinpeptid gemäß Anspruch 7 kodierende Nukleinsäure ist, oder
b) eine Nukleinsäure mit einer Sequenz entsprechend SEQ ID 5 bis SEQ ID 12 ist, oder
c) eine Mutante eines der unter a) oder b) genannten Nukleinsäuren ist, wobei die Nukleotidsequenz mindestens 60% Homologie zu einer unter a) oder b) genannten Nukleotidsequenz aufweist, oder
d) ein Fragment einer Nukleinsäure gemäß a) bis c) ist, oder
e) eine natürlich vorkommende Vorläufemukleinsäure einer Nukleinsäure gemäß a) bis c) ist, oder
f) eine Nukleinsäure gemäß a) bis e) ist, welche Bestandteil eines Makromoleküls, insbesondere eines Vektors oder Plasmids oder Bestandteil eines Genoms eines Mikroorganismus ist, oder
g) eine chemisch, enzymatisch oder posttranskriptional modifizierte Nukleinsäure einer der unter a) bis f) genannten Nukleinsäuren ist.

9. Antikörper, Antikörperfragment oder Affinitätsmatrize, **gekennzeichnet dadurch, dass** dieser Stoff
a) mit einen Syncollinpeptid gemäß Anspruch 7 spezifisch wechselwirkt, insbesondere bindet, und/oder
b) das Antikörperfragment insbesondere ein FAb-, F(ab')2- und FAb-Expressions-Fragment, ein scFV-Molekül oder ein Epitop-bindendes Fragment ist.

10. Nukleinsäure, welche zu einer Syncollin-kodierenden Nukleinsäure gemäß Anspruch 8 komplementär ist, wobei die Nukleinsäure insbesondere eine Antisense-Nukleinsäure, ein Ribozym, eine dsRNA, eine PNA (Peptide Nucleic Acid), ein TFO (Triplex Forming Oligonucleotid), eine Triplex-Nukleinsäure oder eine RNAi-Nukleinsäure ist.

11. Agonist oder Antagonist eines der gemäß Anspruch 7 bis 10 beanspruchten Stoffe

12. Galenisch aufbereiteter oder chemisch oder biologisch modifizierter Stoff nach einem der Ansprüche 7 bis 11, insbesondere in einer für spezielle Applikationswege geeigneten Form, insbesondere für eine Gabe in den Blutkreislauf, den Gastrointestinaltrakt, den Urogenitaltrakt, das lymphatische System oder den Subarachnoidalraum, für eine topische Anwendung, für eine direkte Injektion oder Infusion in Gewebe, wie beispielsweise Muskelgewebe, Fettgewebe oder Gehirn, oder für eine Inhalation geeigneten Form

13. Verfahren gemäß einem der Ansprüche 1 bis 6, **dadurch gekennzeichnet, dass** die Identifizierung oder Quantifizierung wenigstens eines Stoffes gemäß einem der Ansprüche 7 bis 12 mit Hilfe eines biologischen Aktivitätstests oder eines immunologischen, molekularbiologischen, physikalischen oder chemischen Tests vorgenommen wird, wobei insbesondere
a) zu einer physikalischen Identifizierung oder Quantifizierung eines Stoffes gemäß einem der Ansprüche 7 bis 12, insbesondere zur Identifizierung oder Quantifizierung eines Syncollinpeptids gemäß Anspruch 7, die Masse der Stoffe herangezogen wird, vorzugsweise unter Verwendung massenspektrometrischer Methoden, und/oder
b) eine solche massenspekrometrische Bestimmung eines Syncollinpeptids gemäß Anspruch 7 insbesondere einen der theoretischen, monoisotopischen Massenpeaks von 12538,0 / 12552,0 / 12613,0 oder 12350,9 Dalton umfasst, und/oder
c) eine immunologische Identifizierung oder Quantifizierung eines Stoffes gemäß einem der Ansprüche 7 bis 12, insbesondere eine Identifizierung oder Quantifizierung eines Syncollinpeptids gemäß Anspruch 7, mit Hilfe eines auf das jeweilige Peptid gerichteten Antikörpers, Antikörperfragmentes, Phagenpartikels oder einer Affinitätsmatrize vorgenommen wird, und/oder
d) zu einer immunologischen Bestimmung vorzugsweise ein ELISA (Enzyme Linked Immuno Sorbent Assay), ein Radioimmunoassay, ein Protein-Chip-Assay oder ein Westernblot eingesetzt wird, und/oder
e) eine molekularbiologische Identifizierung oder Quantifizierung eines Stoffes gemäß einem der Ansprüche 7 bis 12, insbesondere eine Identifizierung oder Quantifizierung einer Syncollin-kodierenden Nukleinsäure gemäß Anspruch 8, vorzugsweise mit Hilfe eines Northernblots, eines DNA-Chip-Assays, einer Reverse Transkriptase PCR oder einer quantitativen PCR vorgenommen wird.

14. Testkit zur Durchführung eines Verfahrens entsprechend Anspruch 13, **dadurch gekennzeichnet, dass** mindestens
a) ein Antikörper, ein Antikörperfragment oder eine Affinitätsmatrize gemäß Anspruch 9 eingesetzt wird, und/oder
b) der Antikörper, das Antikörperfragment oder die Affinitätsmatrize in immobilisierter oder markierter Form vorliegt, oder in einer Form, welche eine Immobilisierung oder Markierung ermöglicht, und/oder
c) ein Stoff gemäß einem der Ansprüche 7 bis 12, insbesondere ein Syncollinpeptid gemäß Anspruch 7, als Standard enthalten ist.

15. Arzneimittel zur Therapie, Diagnose oder Prophylaxe, insbesondere von Stoffwechselerkrankungen, **dadurch gekennzeichnet, dass** es
a) mindestens einen Stoff gemäß einem der Ansprüche 7 bis 12 enthält, oder
b) mindestens einen Stoff gemäß einem der Ansprüche 7 bis 12 und zusätzlich mindestens eine weitere pharmakologisch akzeptable Substanz, vorzugsweise ein Konservierungsmittel, einen Füllstoff, ein Lösungsmittel, einen Farbstoff, einen Geschmacksstoff oder einen Geruchsstoff oder eine weitere pharmazeutische Wirksubstanz enthält,
wobei das Arzneimittel insbesondere
c) zur Verminderung der Aktivität und/oder Konzentration mindestens eines Stoffes gemäß einem der Ansprüche 7 bis 12 eingesetzt wird, wobei insbesondere
i) Substanzen, welche gegen Syncollinpeptide oder deren Vorläuferproteine gerichtet sind, insbesondere Antikörper, Affinitätsmatrizen oder Antikörperfragmente, inbesondere Intrabodies, Fab- (fragment, antigen binding-) oder scFv- (single chain Fv fragment)-Fragmente, oder
ii) Antisense-Nukleinsäuren, Ribozyme, dsRNAs, PNAs oder TFOs zur Verminderung der Expression von Syncollinpeptiden oder deren Vorläuferproteinen, oder
iii) Substanzen, welche eine Prozessierung von Syncollin-Vorläuferproteinen zu Syncollinpeptiden hemmen, oder
iv) Antagonisten der Syncollinpeptide eingesetzt werden, oder
d) zur Erhöhung der Aktivität und/oder Konzentration mindestens eines Stoffes gemäß einem der Ansprüche 7 bis 12 eingesetzt wird, wobei insbesondere
v) Syncollinpeptide oder deren Vorläuferproteine, oder
vi) für Syncollinpeptide oder deren Vorläuferproteine kodierende Nukleinsäuren, oder
vii) Substanzen, welche eine Prozessierung von Syncollin-Vorläuferproteinen zu Syncollinpeptiden begünstigen, oder
viii) Agonisten der Syncollinpeptide eingesetzt werden.

16. Verwendung mindestens eines Stoffes gemäß einem der Ansprüche 7 bis 12 zur Herstellung eines Arzneimittels zur Therapie, Diagnose oder Prophylaxe von Stoffwechselerkrankungen.

17. Verwendung eines Stoffes gemäß einem der Ansprüche 7 bis 12, insbesondere eines Syncollinpeptids, zur Gewinnung von Antikörpern oder zur Entwicklung von Diagnosereagenzien zum Nachweis einer Stoffwechselerkrankung.

18. Screeningverfahren zur Identifizierung von
a) Substanzen, welche die Expression mindestens eines Synollinpeptids gemäß Anspruch 7 oder einer Syncollin-kodierenden Nukleinsäure gemäß Anspruch 8 vermindern oder verstärken können, oder
b) Rezeptoren, die mindestens an eine der in Anspruch 7 bis 12 genannten Substanzen binden, oder
c) Agonisten oder Antagonisten mindestens einer der in Anspruch 7 bis 12 genannten Substanzen.
